# EUROPEAN PATENT APPLICATION

(11) **EP 3 396 379 A1**
(43) Date of publication of application: **31.10.2018**
(21) Application number: 16877841.3
(22) Date of filing: 22.12.2016
(51) Int. Cl.: G01N 33/574

(54) **BIOMARKER FOR THE DIAGNOSIS, PROGNOSIS AND MONITORING OF EARLY-ONSET COLORECTAL CANCER**

(30) Priority: 23.12.2015 ES 201531891
(71) Applicant: Fundación para la Investigación Biomédica del Hospital Universitario 12 de Octubre, 28041 Madrid (ES); Fundación Centro Nacional de Investigaciones Oncológicas (CNIO), 28029 Madrid (ES); Universidad De Salamanca, 37008 Salamanca (ES); Fundación Instituto de Estudios de Ciencias de la Salud de Castilla y León, 42002 Soria (ES)
(72) Inventor: PEREA GARCÍA, José, 28041 Madrid (ES); GONZÁLEZ SARMIENTO, Rogelio, 37008 Salamanca (ES); URIOSTE AZCORRA, Miguel, 28029 Madrid (ES); RUEDA FERNÁNDEZ, Daniel, 28041 Madrid (ES); ARRIBA DOMÈNECH, María, 28041 Madrid (ES); GARCÍA HERNÁNDEZ, Juan Luis, 37008 Salamanca (ES); PÉREZ GARCÍA, Jéssica, 37008 Salamanca (ES)
(74) Representative: Pons Ariño, Angel
(86) International application number: PCT/ES2016/070929
(87) International publication number: WO 2017/109263

(57) **Abstract**

The invention relates to a novel biomarker for the diagnosis, prognosis and monitoring of early-onset colorectal cancer, the gene NOMO-1 or any of the expression products thereof. Thus, the invention also relates to an in vitro method of diagnosis, prognosis and monitoring of early-onset colorectal cancer, in which said biomarker must be quantified in a biological sample containing tumour cells.

SEQUENCE LISTING

<110>  FUNDACION PARA LA INVESTIGACIÓN BIOMÉDICA DEL HOSPITAL
       UNIVERSITARIO 12 DE OCTUBRE
       FUNDACIÓN CENTRO NACIONAL DE INVESTIGACIONES ONCOLÓGICAS
       (CNIO)
       UNIVERSIDAD DE SALAMANCA
       FUNDACIÓN INSTITUTO DE ESTUDIOS DE CIENCIAS DE LA SALUD DE
       CASTTLLA Y LEON

<120>  Biomarker for the diagnosis, prognosis and monitoring of
       Early-onset colorectal cancer.

<130>  ES2535.7

<150>  P201531891
<151>  2015-12-23

<160>  9

<170>  PatentIn version 3.5

<210>  1
<211>  64880
<212>  DNA
<213>  Homo sapiens

<400>  1
catttggaaa caaaatacaa aacctatcaa aataaagcac tagctttttt tttttgagac      60

aaaatggaag aaatcctaat attaggtaag aaaagcaatt ttttggtgtt tttttaattt     120

tatttattta tttttttgag acagagtctt gctctgtcgc ccaggctgga gtgcagtggt     180

gcgatctcgg ctaaccgcaa gctccgcctc ccaggttcac gccattctcc tgcctcagcc     240

tcccgagtag ctggtactac aggcgcccgc caccacgccc ggctaagtgt ttatattttt     300

agtagagacg gggtttcact gtgttagcca ggatggtctc gatctcgtgg cctcatgatc     360

tgccagcctt ggcctcccaa agtgctggga ttacaggcgt gagccactgt gcccggcctc     420

tttggtgtgt attttgatgt ggtgactggt agttaaattc agtgtacata tgaattcact     480

cttgggacaa aatatgctgg atttgagctt gtttttaggc aaatcatgtt tcatcttgca     540

gagctaattc aaagtatact gactgcatgc acttcttgct taggggaaga aaactcatca     600

aaatttgttt ctaggcatat gttattagag ctaggattaa aataaatctt acatactttc     660

tttaatatgc taacattaac acgttttgct taaaaaagta tcgatcattt atcatggaaa     720

aatacaccag cttacacaaa ttcttaaagc gtttttcgtg tgtgtttttc gtgtgtgtgt     780

gtgtgtgcgt gtgtgtgcgt gtgcgcgtgt gtgtgtagag atggggtttc gccatattgc     840

ccaggctggt ctcgaactcc tgggctcaag cgatccaccc acctcagcct cccaaagtgc     900

tgggattaca ggcgtgagcc acacacccgg ccctaaagct ttttgtgaga ttatatattt     960

tacctgcgaa atatatgggt cagaaagatt tgagaaagtt gttagataat tccaccaaca    1020

aaatctcccg tcctttccag ctgtgatggt gaattctaaa gtagaattca tctttggagc    1080

ttctccctgt aggcatgcac cttgcgttgc gcctgccaga tgtcgcgcgg agtcctccag 1140

gcctctagaa ggcagagatg gtctcgtttc cttgccgagc atgcgcctta gttctctctt 1200

ctggggctgt gaacgtgggg tcgaagcgcg cgtgcgcggc ggctctggcg gcggcggtgg 1260

ggcggggcct gggctgtcag ccggcctagg aggaggaagg agcctgcggc gtgcagtgtg 1320

aggggcggga cccggctgcc ggcggtgggt ctagctgggg gaggtcgggc catgctggtg 1380

ggccagggcg cggggccgct ggggcccgcg gtggtcaccg ccgcggtggt gctgctgctg 1440

agcggcgtgg ggccggcgca cggctcggag gacatcgtgg tgggctgcgg tggcttcgtc 1500

aagtcggacg tggagatcaa ctactctctc atcgaggtga gcgcccgccc cgccgcccgg 1560

cgccgagtcg ccgggccggt ggttcagcct ctctgggccg cgctggcctc gtctctgaca 1620

ccgggcggtg tggagtcctt ggagccctta cctcttccga ggctatgctg tccgcgggct 1680

ccctgcagcc cctccccagt cgcgctggag gggaggttct tatcatgggg tcgtctaagg 1740

actgaggggt gccagacctc aggttcttca aaaccccgac tccagatccc cggaacacag 1800

accccagatc ctcaaaaccc agaccctaaa ttccctggag cccagactcc atttctttaa 1860

aacttagacc tgagatctcc tcgaatcaga ccccctgaaa ctcagatttc cccaaaccca 1920

cattccagaa cctagagacc acatgtctta tcccccaaac tccacagccc agaacccccc 1980

gaagttctaa tccactgctc ctccaaaccc cgcggcatga ttttaaagag catcaattcc 2040

catcccatag attctgacct agtagatcag tattggcaac cctggggccg gaaagctatt 2100

ttttatttct ccggctcccc tccacttctg aaaatggaga gttgccagct gccctgattg 2160

ccttaatgag tagttaatgg aattacccta gagatttgtg ctgaaggttt tttttttttt 2220

tttttaaatt aagttgtaaa gtcctgttaa atgtttatca gcccggtggg attatgaccc 2280

agaaaggttc ccgtaaagag gaaagggatg gatggaggta ggggcagaat gtcatttgtc 2340

ccggtagatg ggaggggacc agagcaaggc ctgggcctca tttttgggag gggatttatg 2400

gtggggtagg acaaggaaga ggaaaagagc gggtgcctcg gaggtaattt aggagaaaac 2460

gaaggaagag gctgggaaat gccaaagaga gggattgggg aataagaccc caaagatcgt 2520

ctgaaagcac ctttgagctg ttccagggct ggcagtgaag ggtgaagctc tgtgttctgc 2580

aaagcgggct gctgaggatt gggggagggg tagggagtca gttccacacc ccaccagctc 2640

tgcgacctgc ccttgctcta ggtttcagtt tttgtaccta taggatggtg ttgggagaga 2700

aacagtggaa cccagcagat acatttcttt aacaagcgga tccgtgatct tcttcaaggc 2760

ccccttgttt actgcttttt cagactgaag aggtcagagg tcaggcattg aatgaatcta 2820

cccactggca gcggcagggc tcctaggatt tcctgggtac atgtttctgg gtgcccatgc 2880

caccgcctag gtctgcactg gggacaggac gcgttcaggg tggtatggct gtagacggca 2940

cagggcattt cagaagctga aatgcaagag gccgctaagg cctcttgcaa cttgtagttt 3000
ccaggggttc tggaaggaag gaaaagacag gcacccccct ccaccctcac cctcatacta 3060
atagtgacct tgagagcaga gaacaggttt tgtgacccat ttcaaagtgt gttccttaag 3120
cccaagggct gcctgtccag atggtgtggg acactcattc tttcataagt aattcagatt 3180
ctgaaatcag acatttcata ctggccatct tgatgtagat tacattgcca tcctgtgtcc 3240
tgattgtact ttatgaaaga tgtagaaata atcatgatat ttcacggtta tttcttgatt 3300
gtgttttttg tgttcctatg caactgccgc cattcttctc tagctttctc ccatcctgag 3360
tgtttttaca tcgtgttcat tgagtcaaca actatttact gagccctggc acatactgaa 3420
gccttgtccc cagtgtggat atagcagtac agtgaacaga acagacaaaa ctccatactc 3480
tcatgagact gacattttgt tctctaacag ggaccttact gttaaaaaaa aaaaattata 3540
ctggttgcta atactttaac aatccgcagg tttcacatta aaaaccgggt tctttatttc 3600
tctggaaagc cagccgatct ttaggctgcc acacttggct tgggttttgt agtgatctga 3660
atccttttgg atgaatgtcc cttgatgttg cctccctaac cggtctaggc tggtgaattt 3720
ccagtccctg ccttaaatgc atgtatctga atctctttgt cacctgtagc aaattcactg 3780
agtacctaga gtaagctaga cactgtctca ggcactgggg aatgaagttg tcactgacct 3840
cagagaactt tacatacagg cttaacctta tttcacacga gtcctttccc aaatgttcaa 3900
atgttcgttt tatgagttag tcactttgta catcttctta gctggtgaga ttcgactgtt 3960
tcattgcagc tcgtgtcatg gtttgtttca tggtcataga gcattaacat tttggaggtg 4020
aggcttttag gtgcatcaga cccaactgct tccttttaca gacatggaaa ctgagcctca 4080
gagtggttga gctgtgcagg tacatttagg aaattttcct tgagcccagt atgttcccta 4140
cgtattacac tgtgttgcct ttttcttcag agagagatat taaatagtaa tacaaactgc 4200
tttttccatt ttactttttt tttttttttt ttttttgaga cggagtctcg ctctgtcgcc 4260
caggccggac tgcagactgc agtggcacaa tctcggctca ctgcaagctc cgcttcccgg 4320
gttcacgcca ttctcctgcc tcagcctccc gagtagctgg gactacaggc gcccgccacc 4380
gcgcccggct aattttttgt atttttagta gagacggggt ttcaccttgt tagccaggat 4440
ggtctcgatc tcctgacctc atgatccacc cgcctcggcc tcccaaagtg ctgggattac 4500
aggcgtgagc caccgcgccc ggccccattt tacttatttt attttgtttt attttgaggc 4560
agggtctcac actgttgctc aggctggagt atagtggcac catcatagct caccaaagcc 4620
tctgactcct ggcctcaagc agtcctcctg cctcggcctc ctgagtagct gaggctacag 4680
gcacgtgcca tcatgcctag ctaatttttt ttctagtagg gatgaggtgt ggctatgttg 4740
tccaggctgg tctcctgggc tcaagtgatc ctccttcttt ggcctcccaa aatgctggga 4800
ttacaggcat gagccaccat gcctggccgc aaactgattt ttctttgaat ccatattctg 4860

tagcaggtgc ttaatatgca ttgatgtgaa tctgtacaac cacatgaaaa ggagtggctc 4920

tgtgtctaca tctgaagaaa ccgaacacga gcttgcccaa ggccacgcaa cacgtggaag 4980

actcagatgg catttgggca tcttactggg ttaacacttc gtgtctggta atgctcagaa 5040

tgtaagagtt ttagagagtc cttgaagaga ggacagatat taaatgtctt tttagtctct 5100

ttttcttcgg agaatgaaag taatgagaaa attaagtttc tagatgaatg ttgctccaag 5160

tatttgaaat tattcagagt tagtaaggaa gatttctggc cctcattcca gacctttagt 5220

tgagaaatag aggtgggccc caggaatttg caatttattt tactttattt ttttttatta 5280

ttatttttga gacggagtct tgctctgtct cccaggctgg agtgctgtgg catgatcttg 5340

gctcactgca acctccgcct cctgagttca aacaattttc ctgcctcagc ctcccaagta 5400

cctgggacta ccggtgcctg ccaccatgcc tgggtaactt tttttgtatt tttagtaggg 5460

acagggtttc accttgttgg ccaggctggt ctcaaattcc tgaccttaag tgatctgccc 5520

acctcagcct cctgaagtgc tgggagtact ggcatgagcc accgtgccca gccaggaatt 5580

tgcatgttaa taagcacctt actaatggca aagtttgcaa atttgtttta gacccggaaa 5640

cttacacgag caacctagag acagccacta aaataaacag aaaagcattg aaaaataagg 5700

cattgtaata ggctgtagat ggtttctaat ctgaaaaaca ttgagtcaca tgatttggga 5760

gagagtttgt tgatttccct ctgtccccag taatgccccg tgttaaaagc tggcatataa 5820

taggtcctca gtgaatgtat actgactgag tgcattcagg tgtaacatgt gtagttaatt 5880

cccaaaggca atgttatgat ttctgttttc cttccttcct ttccagataa agctgtacac 5940

caagcatggg actttgaaat accagacaga ctgtgcccct aataatggtt actttatgat 6000

ccctttgtat gataaggtaa gaggggactg cttgtcactt atgatgggaa atcactagtg 6060

tgcctcacca ggctgcatta accatgccct ttcctacact agcaaatgct catctgtttt 6120

gtaaattatt agtggaatat gataatccta gccacatttg gagggagaca acttacaaac 6180

tgccttgacg aatacagtgg ctctctctgt gtttagggcc atttggcaat gtctagagac 6240

gtttttgttg tgacatctgt ggctgtgcgc tacagtcacc cagtgagtag tggccaggcg 6300

tggagctaaa tgcgctacag tgctgcctac gacagcccag atgtcagtag tgccagggct 6360

gagaaaccct gatttcatgg aacccgttac ttgcaagcag cttaaagtat tgttaaaaca 6420

attgtacaag taatacatga attcatgaac tttgtgaaaa agaaagtcaa ggtctttaga 6480

agtatgtgaa ataaatttgt acccttttcc ctccaatttt acctccctct gtagtgataa 6540

cctttgataa tctgataaag ggtttttttg ttgttttttt ttttcttctt ttgagatgga 6600

gtctcgctct gtcgcccagg ctggagtgca gtggtgtgat cttgcctcac tccaacctcc 6660

accccccagg ttcaagccat tttccttcct cagcctccca aatagctggg attacaggca 6720

cctgccacta cacccagcta atgttttgta tttttaatag tgacggggtt ttgccatgtt 6780

ggccaggctg gtctcgaact cctgacctca agtgaaccac ctgcctcagc ctcccaaagt 6840

gctgggatta caggtgtgag ccgatgtttc cggccctctt ataaaatttt gatgtgcatc 6900

ctaacagatt gctttctatt tatagttatt cagatagcca cgggtacaca tgcatatgta 6960

tacatggaaa tacacacaag cacacatgca catcgatatg tatccacaaa cacctctata 7020

caatcccatc ctcccacata taaacctttt atttggaaat acagattcat aggaagttgc 7080

aaaaatagtc aatacacata gttttgattt tgatatattt tttaatacaa atgaaatcaa 7140

acaataggat ttattctgca gtttcatttt ttcccctgtg ttaatgcttt caataccaga 7200

gggacatttg gttttttcct cttgcatgct ttcaacatta gcatagatag atctactgac 7260

ttttttttct tttttttttg agacagagtg tcactctgtc agccaggctg gagtgcagtg 7320

gcgcgatctc ggctcactgc aacctctgcc tcctgggttc cagtgattct tctgcctcag 7380

cctcccaagt agctgggatt acaggcaccc accaccatgc ccagctaatt ttttttgtat 7440

ttttagtaga gatggtgttt caccatgttg gccaggctgg ttttgaactc ctgacctcag 7500

gtgatccgcc cacctcagcc tcccagagtg ctgggattac aggcatgagc caccatgctc 7560

agcctactga cttcttttta atcgctgcat agtgtgtcag agtcaaaccc aaactgtaat 7620

ttatttaact acttggacat atagactttc cccaatttta aaataatagt aattttaaca 7680

taacacacca ggaaatattc ttgaacttaa ctcttcatcc agaatgcata ggactgattt 7740

ctagagatgg actagctggg tctgtcctga aatctattta attaattttt aaatagtatc 7800

agtatcagtc tcaagaaacc aggaggctgg acgtggtagc tcatgcctgt aatcctagca 7860

ctttgggagg ttgaggcagg tggatcgcct gagctcagga gtgtgagacc agcctgggca 7920

acatggtgca accccgtctc tactaaaata caaaaaaatt agccgggtgt cacgtggatg 7980

cctgtagtcc cagctacttg ggaggctgag gcaggagaat cgcttgaacc caggagacgg 8040

aggttgcagt gagccaagat cgcaccactt cactccagcc tgggcaacag agcgaggctc 8100

catctcaaaa aaaaaaaact aaaacaaaaa caaagaaacc agggttgata agtaaaatct 8160

agttttattt attgaattaa tgagttaatg tattgtttag agacaggatc tccctccgct 8220

gcccatgctg gagtgcaggg gcacaattat ggttcactgc agcactgacc tcctgggctc 8280

aagtgatctt cctgcctctt cctcctaagt agctaagact gtaggcgcct gccaccacac 8340

ccagctgatt ttttaagaac attttttgta cagatggggt cttgctatgt tgcttaggct 8400

ggtcttgaac tcctggcctc aagtgaccct cctgccttgg cctcccacag tgttgggatt 8460

ataggtgtga ggtgctgtgc ctggccaaat ctagttttag ggggtgcctt tagttcctga 8520

agatacatgc taacttgtgc aactgtaggc atgtgagggc ttaatgggag actcaattaa 8580

tagaattata gtatgtttag tattaaaaga cagtgagtgg ctgaacctag tgtctttaga 8640

actcatgaat gggcttcagc actgtcttct gattgtttag atgaaaactt ctggtgttca 8700

gcttcagctt gtagcttctg aagagaatca gtgctcagta ccatgtagaa gaacaaggct 8760

cactcttaga atttcatttc cggtgtaaaa aaattttaga ttaaatgata acactaaact 8820

aaatttagcc agagtcacaa ttctcatttc ttgaaattac attttctttt tttcctttta 8880

gggggatttc attctgaaga ttgagcctcc cctagggtgg agttttggta agttaactga 8940

atcactagac attctttgta aaagattaga tgatatgccg aatattaatt taggctagca 9000

gagtgcataa actattaaaa tattaaggat catttccagt atggaagagg gtttgtattc 9060

ctcccctcct tgcccccatc actctggttt agttcggatt taccttaatc taccttaaga 9120

ctttctgtca cctttgaaaa atagacatct tttcttctac ctttctggaa actcttccct 9180

tgcagtttca ctcattacgt ggccgtggaa cagtatagaa atcaagagtg gggacttggt 9240

tcccgtccca ggatggcttc ttttttagcc ccctcagttt tttaagtttt tatggaatta 9300

attgaaacat ttaaagattg tgagatttgt cataaaaatc cagatttatg tcccatgaga 9360

tgagaagact gtcagcctta taccttacca gttaaataac tttgggcaag ttacttcccc 9420

cgtgtcagcc tggctcctca ctgtcgagca agggtagtta tagtccctgc cccactgggt 9480

acttggacga ttaataaacc aatactgagt gttcagaggg gctggcacgt aattagcaag 9540

tactcagtat tcagaaacag cccttctgca cacatcccaa acaaaagctc caaactttcc 9600

tcatataact gccttcaagc atagctgggg agtcggggat ggggttgaga tcagaagaga 9660

gggtctgttt tgatgtagtt tacatttttg aagaggagag actcttgatg tttcagtaga 9720

gcaggtgtgg gtgaccccag tccatggctt ggggagagga gtagggatca ccgttgttga 9780

tatcaggctt gctcgtgcat tagacaagga agaatttagc acctgccatc gccaaggcta 9840

aaatgaggct caggcagctg acagctaatt tggctctgtg agagcagctc ttttatcagt 9900

ttatagtcag gggtagggtg gggggtgcct ggctggtacg ttccagaaag ctggagagga 9960

atgcagccct gctcatggcc aaagcagaag tccagattgg tggcctggag gagtgttgtg 10020

tttgccaccc cccaccctac ccccaactgg tgctgttgat aagcttttta attgaattat 10080

actttcagaa aagtgccttg gatgaatttt cactaagtga acacacccat gtatccacca 10140

tccagatgat aagatagcac ttcaccagca ccccagaagt ccccatcctg ggtgctcctt 10200

agccatcccg ccccaccctc caggtgacca ccaccctcac cttctacaaa ttagagatcc 10260

atttggtctg ttttgaactt tatacaaatg ctatcatgca gggaactttt taatatccgg 10320

tttctttcac tcaacattat gaactttatc tgtatcattt gtagctttag ttcattctct 10380

ttgctgttga gaattctgat gcataaatat aaaattattt atattttata aaatatttat 10440

attttatttt atttttcttt tttttgaga ctgagtctcg ctctgttgcc caggctggag 10500

tgtgatctca gttcactgca acttctgccc gggttcaagc aattcttgtg cctcagcctc 10560

ctcagtagat gagattacag gtgtgcacca ccacacccag ctaatttttg tatttttagt 10620

agagatgggg tttcatcatg ttgacccagg ctggtctcga actcctgacc tcaagtgatc 10680

tgcccgcttt ggcctcccaa agtgttggga ttataggcaa gagccaccac gcctggccta 10740

tatttatatt ttatattcat attttgtatc agtgatctct tctactgttg atggccattt 10800

gagttgctta ccgattttgg ccactaagag cagtgctcct gtgaacattc ttgtatgtat 10860

attttgatac atgtgtctgc atttttgctg agtatatcac aggagtaaag tcgctgagcc 10920

atagagctga tacatgctca gctttaggtg tttgcaaaca tccaaagtgg atgcaccagt 10980

tggtgttctc ccagcagtgt acaagggttt ccattgctcc acatcctaac ctacacttgg 11040

tatcgtcagt tttttttaat tttagccctt ctggtggggg tgtggtatta tctcactgtg 11100

gccttgattt acattccact gatggaatgt aaatgaaaat tccacttttt tgtatgttta 11160

tgtttgtttg ccatgggcta gctatttttg taagattcct gttcaagtct tttgcccatt 11220

ttttattgga gtctttgtgt gtgtgtgtgt gtgtgtgtgt gtgcatgtac gcgtgcaagc 11280

acgtgtgtaa ttcatcttaa ctgaaaatct catctcatgg ttttccattt ttttctctac 11340

tcattactcc atattcttcc taacctttca aaaactcaac cttaacccct attatgatga 11400

actcttcagt taaaaaatgg agttaattgc tgatattgaa aaatcaagag atggtatata 11460

aaatccagat ttccagtttt tcggagaagt tggaagctct ggcaaagcat ggtagtggtt 11520

ggcagacttg agtggcggtt gtctcctttc aatttgccac agtccccctg aggatgctcc 11580

gctcacttcg aacccatgag gcccctgtgg ccctggagtt tgtgttcctg gtttaacatg 11640

gctgagctca tgtccttggt cagagaacag atccagagcc agacttttag ggttgaattg 11700

gggctctgtc actcagaagc tctgtgatct tgggagctcc tttaaccttt ctgtgcctta 11760

atttacccct cagtaaattg cagctattac tgcctgtctc ttaagataat tgtgaggatt 11820

aaaggcactc agctcagtgc ctggtcgttg atgagcattc aggaaataaa attatttggt 11880

gctcactgaa ttttcactgg aactctgaga tataacttag ctgaaaggcg ttgtaaaatt 11940

attttatact ctaaacacga attccctctg gggcaggaaa ccaggaggct agggggatag 12000

gggtgagagg gagacttttc ccagaatact tttttgtacc tttggatttg gaaacacacg 12060

agtgtattaa gtgttcaaaa gtaaaatgaa acctgaataa taaaaatacg ttctacttag 12120

gttcactgta ccccaaaact aagcttgcct ttgtgtcttg tgccccggtt tcctcccctg 12180

cttcgttcct cagagccgac gaccgtggag ctccatgtgg atggagtcag tgacatctgc 12240

acaaagggtg gggacatcaa ctttgtcttc actgggttct ctgtgaatgg caaggtttgt 12300

ctttggaact tgattatttt tcctgttcac tctataatgt atactaaatc cttttttaaa 12360

aaaatgcagg atattttaac ctggggactt tgatcccaca agggacccat ggataagtgg 12420

caagtgagcc tgtgaactct gtacttcatc cgtgtagaac gcagcaggtg tatttaggaa 12480

tgagattgag gttgctggtc atcccctctg ctcccagagc ataggctgtt ggtgttttta 12540

tttttttatt ttttattttg agacagggtc ttgctctgtt gcccaggctg gagtgcagtg 12600

gtgcaatctt gactcactgc agcctccacc tcccaggttc aagtgattct cctgcctcac 12660

cctcccgctc cttcaccctc ccgagtagct aggattacaa gtgtgtgcca ccatgcctgg 12720

ctaatttttg tattttcagc agagatgggc ttctgctgtg ttggccaggc tggtctcaaa 12780

ctcctgacct caggtgatcc gcctgcctcc acttcccata gtgctgggat tacaggtgta 12840

cgctgtgcac ccagccgctg ttagcgttta tgtcagggat ggccttttac agggatgccg 12900

aattggaagg acgcactctc taagccttgt ggcatcttgc tgtccctgtg ggtgaattga 12960

agtcagcttc tcccagagag agcatgcatc tgcttctgac acttacctag gaggattcct 13020

attatttaag ttctgagatt tttttggacc gccacggtgg tgtgaattta gagtcctaat 13080

gggtgtgtgc ccagctcccg cgattcagat tctcagggga gagtcatttc tcctccacct 13140

gccctgtgcc tgcggttgag atgtgcttcc ttccttcctg tccttctctt catgatggac 13200

ttctttctcc tctacctttg cggtgacggg gaggcccttc catcccagct gtatgtgtgg 13260

gcttcttgtt agagatgggg atcttgggta gttttttctt tatcgatggt ttgcaaaata 13320

atggtgaatc tttttttatt tttattttta tttatttatt tttttgagac agagtctcgc 13380

tctgtggcct aagctggagt gcagtagcgc agtctctctc accacaacct ccgcctcctg 13440

ggttcgagca attctcctgc ctcagcctcc ccagtagctg ggattacagg cgtgtgccac 13500

ctgacccagc taatttttat atttttattt ttatgtacat ttactttttt tttttttttt 13560

ttttaagatg gagtctccct ctgtcgccca ggctggagtg cagtggcgcg attgcagctt 13620

actgcaacct ccgcctcctg ggttcaagtg attctcctgc ctcagcctca ggagtagctg 13680

ggattacaag catgtgccat gacgcccagc taatttttgt atttttagta gagacagggt 13740

ttcaccatgt tggccaggct ggtctcaaac tcctgacctc aagtggtctg cctgcctcag 13800

cctcccaaag tgctaggatt acaggcgtga accaccacac ccgcccagac caataatggt 13860

gaatctataa ctgatgatgt cttagattca ttgaaataga gtcttatttt actgttactg 13920

ttccctctcc tacctcatcc ccaggaaaca tatccatgat ttatagcatc ttttcaatgg 13980

gattcttgac ccaaagaggg ttcctgggct ccacctggga gttgtaagca ggactgccag 14040

tgctttgaga ggagggtgct ttgctgggtg ggccctaact ttcttctcca tggcaggtcc 14100

tcagcaaagg gcagcccctg ggtcctgcgg gagttcaggt gtctctgaga aacactggga 14160

ccgaagcaaa gatccagtcc acagttacac agcctggcgg aaagtgagta gcgtcctgtc 14220

tcttagtgtt gccttagagc cgggctctga caggggtcat ggagctgggt ttgggagttt 14280

ggattcaggg agttctgggt tcagatcctg attcagcctc tttgggtgag ttcatgcccg 14340

tcagagtgct gagttcccca catgtgaaat ggaatccatt tgacccatct tgcctagcat 14400

tgatgagatg attaaatatg agttcatact ggccaggcat attggcttgc gcctataatg 14460

ccagcacttt gggaggccag gatgggagga tcacttgagt tcaggagttc gagaccagcc 14520

tggacaactt agggagactc aatctccaca aataatttaa aaaattatat caagtagagt 14580

cccacggggg aagaaaatta gctgggtgtg gtggcatgct tctgtggtcg cagctactcg 14640

ggaggctgag gcgggaggat tgcttgagcc tggaaggttg aggctgcggt gagccgtgat 14700

catgtgccac tgtgctccag cctcggcagc agagcaagac cctgtctgaa acaaacaaaa 14760

aaagagttaa tacccataaa gcacctgctt caaggcctgg ggcttagtgg gtcttcattt 14820

agcagctcag cctggctctg gagcctgcca gcccgggctt gaatcccagc tcccccattt 14880

gctggctggt tgacctaggg caagttcctt aacccctctg tgcctcagtt tcctcttttg 14940

taaatgaaga taattgtgga acctatctcc taaggtactg gaaagatgaa attaatatta 15000

ttacttcaag ctgcctggca tattagctca aataggttag ctcagttatg attagaaaaa 15060

atcattatca ttattatatc aaaactgctt tgtagttgga acagatgcct agctgacctt 15120

gctgagtcag atttaaagca tttatttaat ggatgtatat agttgagttg taaatgttaa 15180

cagatatgcc aagattaata ccagaatagt tcaaactgat aattccgaaa gcgttcaaat 15240

cattactaac tccgttaata tacaaactaa gtaaattgca aaatagaagt ttataatgag 15300

tctgtcagta agttattcaa ttttaaagtt attattgttg aaactagttt tcatttttga 15360

aataacattt acttctgctt gactggtgcc tctccaggcc tggtgtgtag caggaatatg 15420

aagctgtaat tttttttctg ggctagtaat ttggtgttga ccttactaga aaaaaatagt 15480

gtcttgagcc ttctgaatac ctttcatctg cactaactga aacaatgaaa catgtagcac 15540

tgggctatta tgaaaatgaa atagaagctc aatcttacca atacttgtag taatgtgatt 15600

tccaagccct atccagttat tgcaggcaag atttgacaca ataaaatacc acagaagaag 15660

aaatcaaatg cgttttctag ttcatttcac cggacttagc cttctcattt gtcagttgcc 15720

ttacaaattt gaagggataa acatgttagt atactggcag cagcatttcc agctttagct 15780

cggctcactc tggtgttagt ctgtctatgg ctagtagagt aattttcatc agctgctggt 15840

ttcttatctc agttgctttg tgcaaaaacc cagaatgact tatctgtgtt tctctaaagc 15900

cttttttctg ggattcaaag cttatcatag tttaccctgc cctgtgtcat ttgcatcatc 15960

ttgggtcagc gtccccctca gcctgtgcat ctgttctgtt ctttttcacc ccacttctcc 16020

cttcccatgt gtttccttgg acacttctgt ccccttctcc ttgtcagttc atgcctgtca 16080

cctctgtcaa acccagtcga atgttctgtg actggtccct gctgtgctgc aaggattggt 16140

tttatgccag aaatccttga gattaaggga gacgtaagca ctcctctttc cgatcagcac 16200

atggagcacc cctcttggca cacagtaagt gctcaataag cgctctttta tgcagatttg 16260

tacagtgaag gattgaattt cattcatgct catttaacaa gctaatggaa gtttgcgata 16320

attccaacat tgttttcttg tgctgccaac attagtcata gaattagaaa tacttgggtt 16380

gtttggagaa agttaaatgg ttatttttta tgtcttaggt ttgcattttt taaagttctg 16440

cctggagatt atgaaatcct cgcaactcat ccaacctggg cgttgaaaga ggtgagtgac 16500

agctacgtct atagccatgc caataatatg ctggtcttgg gacaaatgta gggcttttca 16560

atttaggata tgaaaagaat ttatggttcc tccaggacta gatgaagaac caatggtgct 16620

gttgtttagt ttggtagtta tttataaggt gtaactcctc actgtagcag atgttgcttg 16680

tgttagtttc agttttaggt gacagtgttg tcttcctgct gtgaaataga tctgttttat 16740

taactatagc tattttaaac taaatcaaca acagaaggcc ctgacgagat ttatttgttg 16800

ttcttttttc agcctgaaac agaaatctgt aaatatacta aacattctag aaactgtggt 16860

acgcgtgacc aaggagaatg tatgagaatg tttattgcag ctttgtttgt aatagcaaaa 16920

tattgtaaac aacctaaatg ccttttaaga gaagaatgga ttggggttcc atcatacaat 16980

aggctgctgt atgtcagcag taatgaatga accagagcta catatatcaa tgtaactaca 17040

tttaaaaaca gagttgaagg aaatatgatt ttaaatactt gtgataatca tttatggata 17100

cagtcttttt tttttttttt ttttaaagac ggagtctcgc tctgttgcca agactggagt 17160

gcagtggcat gatcttggct tattgcaacc tcggccttct gtgtccaagc gagtctccgg 17220

cttcagtctc ctgagcagct gaattacagg gatgtgccac cacgcccagc taatttttgt 17280

atttttagta tatatgggat tttgccatgt tggccaggct ggtctcgaac tcctagcctc 17340

aaatgatcca ccagcctcgg gctctcaaag tggtgggatt acaggtataa accagtgcac 17400

tcggccagat acagtctttt tttttttttt tttttttgca acagggtctt gctctgttgc 17460

ccaggctaga gtacagtgac atattcatgg ctcattgcag cctctggttc ctggccttaa 17520

gcgatccttc accttggctt cccaaggatc tggaactgca ggcatgtgcc actgccccta 17580

gctaaggata cagtcttttt tttttttgag atgaagtctt gctcttgttg cccaggctgg 17640

agtgcaatgg ggcgatctcg gctcattgca acctccgcct cttgggttca agcgattctc 17700

ctgcctcagc ctcctgagta gctgggttta caggtgcctg ccaccatgcc cagttaattt 17760

ttgtattttt agtagggaca gggaggatac agtcttatgt agaaaaatta tgaagacatg 17820

tatgggaata gcagacgctg ttcaaaatga tactgacttc tagagaaaga gggaggaggg 17880

ggacttcggg gtatgccagc atcttgaatt gtattttttt aaatttcttt agctggttag 17940

aaggtatgta agtgttcttt agcatatctt tttttgtttg actgaaatag tttatagtca 18000

aagatcaaaa aaggaactcc acctagtagg tgaggttctg taatagccgt gaaagaccaa 18060

attgaaataa gacttaaggg acatcttgac atgttagctt ctgtgttagt attgaagctg 18120
tatctcattt ttgctaaaga agcattggaa tcagtatttc agcttctact ctatctgttt 18180
tattattatt tttactctgt aggttttacg cacttataat tgatgatagt ttcacaccta 18240
gattctcttc ttactgttta tgacttttgg ttttgggggt ttttctcttc tgcagcaaca 18300
tttaaaaata ttttattcaa ggctgggcgc agttgctcat gctgtaattc cagcattttg 18360
ggaggctgag gtgggtggat cacttgaggt caggagtttg agaccaacct ggccaacatg 18420
gtgaaaccgc atctctacta aaaatagaaa aaaattagcc aggaatggtg gcatacacct 18480
gtaatcccag ctactcggga ggctgaggca ggagaatcgc ttgaacctgg gaggtggagg 18540
tagcagtgag ccaagattgc accactgcac ttcagcttgg gtgacagagt gagactccat 18600
ctcaaaaaaa aaaaaaaaaa atttattcga aatgaaagag cttacacatt tataaataga 18660
agagatttta aggtttacat tgtatgttaa ttgtctgcat agaagaatct gagtttatta 18720
atatgagatt gggattttca agtaaagcat gttgttagaa gtcaggataa tgtttatcct 18780
cctggggtgt tagtgaccgg aagcaagtgt gagggcactt ctggcttgct ggtgctattc 18840
tgttgggtaa ccagtaccca atttgtgaca agccctgtgc tgtgtgctta gaatctgtac 18900
acttttctct gtgtttattg taccttaaca aaaatgttta aaaatataca catattgtga 18960
aaagaacaac ttaaggcaaa ttaaacagag tttaattgag catagaacaa tttgtcaatt 19020
gggcagctcc ctgagccaga ataggtttac agcgactgtg gcactgttgc gtgattggaa 19080
agggtttatg gacagaaaaa gaaagtgacg gacagaaagt ggaagtgagg tacagaaaca 19140
gttggattgg ttacagctgg acgtttgtct tatttgcaca tggtttgaac agttggctgc 19200
ctgtgagtag ttgaagggtg gctgcttgaa ttgggtgaga cttggcaaca cgtacagata 19260
aacctttagg ccaaacttaa aatatgtaag gaggcagctt taggctaatc ttcagttaac 19320
actatatatc attaaaaata gaaaaaaagg caaaatatgc tcattaaaaa aattggaaca 19380
tatagaggaa tataaagcag tggaaacaaa ttttggaagc tggaaaagta tacaagtaaa 19440
atagcaccat atagacaaaa tcattattgt ttacattttg ccacatttcc tttcacttca 19500
ttttgggcat taaaaaataa tgataagcca ggcacggtgg ctcacacctg taatcccagc 19560
actttgggag gctgaggcgg gcggatcacc tgaggtcagg agttcgagac cagcctggcc 19620
aacacggtga aaccctgtct ctactaaaaa tacaaaaatt agccgggcgt ggtggcgagc 19680
acttgtaatc cccgctactt gggaggctga ggcaggagaa tctcttgaac ccaggaggca 19740
gaggttgcag tgagccgagg ttgcaccatt gcactcaagc ctgtgtgaca tactgagact 19800
ccgtctcaag aaaagaagtg atcatactga ataagcaatt ttatagatta cgtttcccag 19860
ttaatatttt agcttcagca tgtcctcatg ttgttaggca ctttgcaagt gtttattttt 19920
ttacaggtta aaatcttttt ctgttacagg caagcaccac agtgcgtgta accaactcca 19980

atgccaatgc ggccagtccc ctcatagttg ctggctacaa tgtgtctggc tctgtccgaa 20040

gtgatgggga gcccatgaaa ggcgtgaagt ttcttctctt ttcttcttta gtaactaaag 20100

aggtaagcaa agaaaagaac aaaagagatg gtgtgaaggg taggagggtg ggggattata 20160

gaccagaacg tactgtttta aaaaatgcag gtctgagttg atttatcatt ttaacactta 20220

agaatatcgt ctgtctcagg ccgggtgtgg tagcacatgc ctgtaatccc agcactttgg 20280

gaggccaagg tgggtggatc acttgaggtc aggagtttta gaccagcctg gccaacatta 20340

tgaaacccca tctctactaa aaatacaaaa acattagccg ggcatgttgg cacgtgcctg 20400

tagtcccagc tgcgtgggag gctgaggcag tagaatcgct tgaacccagg aggtggaggt 20460

tgcagtgagc caagatcaca ccactgcact ccagcctggg tgacagagtg agactctcaa 20520

aaaaaaaaaa aaaaaattca agaatatcat gtgtctctgt tgactcacaa gacattgcac 20580

atctacactg ggaggcagtg gtatgtgatg gaaagtacac aggctttgaa atcagacctg 20640

ggtttaaaca aatctcaggt ttaaacaaat tcctcttcct agctatgcga ccctgggccg 20700

gtcacattac cttcctgagc ttccgtttcc ttatttggag aatggtgacc catgataagg 20760

aaggacagtc atgtcatctt tcccgggaag actgtgccag tttttaactt tgcaacttga 20820

atgtcaagat ttttgttcct tttatctgta ttaatgtgtc ttagatttca gaaatacata 20880

tattgacatg aaacttaaaa aaattttcct ataatgtggc taattgatgg tattcagaac 20940

atgtaaatgg aaaaggaagt cttgtctttt ttaccccctg gcataggatg tcctgggctg 21000

caatgtctca ccagtgcctg ggttccagcc ccaagacgag agtctggtgt atttgtgcta 21060

cacggtctcc agagaagatg gctcgttctc tttctattcc ttgccaagtg ggggctacac 21120

tgtggtgagt aaagcagatt tccgttctgt ttatgtctga gactctcatg acacagtaaa 21180

agccaatgct gtttgggtgt taaaggaaaa gatggttggc ctgcagttct ctgaacacgc 21240

tgttaagcag taacttactt aagatgagac actcacccct tcgtaatagc tacaaaaagg 21300

gcaaggcttc ctcttttcga atctgcaaca gttatgatga atgtttctct tggtctgtct 21360

gaaccccttg tcatttgtgg gctgctagat cgattattaa tattgctgat tgatcatgtt 21420

agtggttctt agtgattaac agactcttcc ttccagattc cgttctatcg aggggagagg 21480

attacctttg atgtggcgcc ttccagactt gacttcacag tggagcatga cagcttgaaa 21540

atcgaggtaa ggcttttctg tcttctggag ggacaggtgt ttgagttccc atccctaggc 21600

acaggtattg caaacatgtt ttaaaaaaaa atcaattatt ttatctggaa gggctgttgg 21660

aaaaaatgcc atctggtatt atattcttac ccatgatgtc cttatcgtag agagctggat 21720

tcctagttaa atctactcat tcaatgaaat atatttattg attgccagtg acatgcattt 21780

caggcaccat tccaggactg gggatatagc agtgaccaaa agaggcaaaa attcttgtcc 21840

ctgtggacct catttttttt tttttttttt ttaagatagg gtctcactgt gtggctcaag 21900

ctggagtgca gtggtacgat ctcatctcac tgcagccatt gcctccaggg ctcaagtgat 21960

cctcccacct cagcctgttg agtagctggg accacaggca cgtgccacca tgcccagcta 22020

atttttgtat ttttgttaga gatggggttt caccatgttg cccaggctgt tctctaactc 22080

ctgggttcaa gtgatctgcc tgcctcggct tcccaaagtg ttgggattac aggtgtgagc 22140

catcgcacct ggctagacct cattttctaa tgacaggaag ggtcaataca tgtatagatg 22200

tataagttag aaagtacagt agaaggtgat aagtgttctc tagaaaaata aaggaagaaa 22260

ggaggtcgta cagtatcagg gtggagacag gggttgcagt tgtacatagg gtgtttttgg 22320

gaagtcctcc ctgtgaaggt gacatttgag caacaacctg aagggaaaga acaagagaac 22380

catacttcac tcctgcagga agagtgtgta agactgaggg aacggcacgt gcaaaggtcc 22440

tgaggcagga gtgtgctgaa gggtttgggg catcccaagg aggccagggt ggctggagtg 22500

gagggaatga gcaaggaagg aggaggcgga aagagggtca gggaggtgac agggcagatt 22560

gtgcagggct tggtgggcca ctttgcctat aactctgggt gagaattggt gccactggaa 22620

ggttcctagc agaggagtgg cgtgatcctt ctggcagctg cattgaggag agtttgcagg 22680

gtgtcagggt ggaagtggga gaccagttgg gatgccatgg ccgtaaacca ggtgagaact 22740

ggcagagctt ggaccgtggt ggtaggaatg ggcagtgtga tacatggctt gataaactgc 22800

ctagaccctc acataaatgg taaatgtatt tttcatctca cttacgtctc ctttaattac 22860

agaagtagaa aatggttact gtagaaaatt ggctagctag aacaaatccc aaaagaaaat 22920

aacagcctct cctaagtgga ccacccaatg ttagagttat cgaagcaatt atattttaat 22980

cactttcttt taatattaca tttgaaagta ctgtgttaaa atcgcttaaa ctggcagaag 23040

cggactttta ggtcatatgc acagtggggt gcagactctg cattttaaga ttcagtggag 23100

tgtttgaccg ttctgctgta cactggaggg gaaagagaag ggtaagaaga tgaagccttt 23160

actttgcttc tcatagcttg cagagaagga tgggggcagg tgagcagaga cagctattcc 23220

cagattacag ccaaatacat attttgaaaa tgatgaatgg aatttcaggg aatggggccc 23280

tgtaagttcc ctgaggtcag gtaccgtgtc ttattcttta ctgttttcct aacgtgtgat 23340

ttagtgcctg gcacatagta ggtgcccagc aaaaatattt gttgagtaca tatgtgaatt 23400

aataagtagg taatgcatgg aatgaaacat gtctttttgg aaagatcaag aaggactacc 23460

tgtcatgtga gaccatcact aagaaaccat ttgctgaaaa aaaaataaaa aaataaaaaa 23520

gaagggctcc ctgcataaaa aggaagtcat ttaattattt aaagcttcat ctcaaatgct 23580

gttgcacacg tgcgtgcaca cattcattca ttcaggagtc agttattgag tgcctgctgt 23640

gtaccaggtg actaagacag aaaaaatccc tgtctccatg aggtaacatg ctagtgggag 23700

ggagacaact aagaaatcga acgaggacat ttcagatact ggtaagtttg atgaagatac 23760

taaaatgggt tagcagtggt gaccttgggg atacacagat cagggctgga actcagtaac 23820

tgcagagaaa gcaccggtgt gctgctcaca ggcgagtggg gcacagacac agacacagac 23880

acatggagca tgggaccacg tgtgagccag tgatggtggt ggaggagggt ggtctaggtg 23940

gatttctttt tttttctttg agacagagtc ttgctgcgtc acacaggctg gcactgtctt 24000

ggctcactgc aacctccgca tcccgagttg aagcgattct cctgcctcag cctcccgagt 24060

agctgggatt acaggcgcct gccaccacac ctggctaatt tttgtatttt tagtggagac 24120

agggcttcac catgttggct aggctggtct tggactcctg acctcaagtg atctgcctgc 24180

ctcggcctcc caaagtgctg ggattacagg cgtgaaccac tgcgcctggc ctaggtggat 24240

ttcataagga cctgtgttca ggtcgtggtg gaaagacagg agggggcacg tgagaccttg 24300

cagaggtgga atccacagga ctgatcgatg ctggaggagc ggggcggggc atccaaagtg 24360

atgccggggt gtgcatggga gactggagga agacgtggtt caccaaaatg ggggcatctg 24420

gaggggagga gatggcaccg gggtaagggt ggggggcttc tagaagcttg agtcagtgac 24480

cttttaacga gggaagtaag tctctagact tttctgttgt ttgacactca gcagtgtgtt 24540

gggaacacaa cagagaagac agatgtgggt aaagtgagag ggagcctggc tggcacacag 24600

gaccggggca ctgagtgttg ggaggtgggc ggcaggagca gacatggtag gtggtggccg 24660

gcgcagcaga aggagtcttt gtggctctgg cacaggagca ccttcgagca ccttcttctt 24720

gttctttgtt ttctagcccg tgttccacgt catgggattc tccgtcaccg ggagggtctt 24780

gaacggaccc gaaggagatg gtgttccaga agcagtagtc accctgaata accaaatcaa 24840

aggtgggctg acacagcagc cccaggctga tggccagcgc tctttttgga tcacagagag 24900

aaatggagag gaactgatga ctatatgaga acatggcagc ttttggtcca agtattctgg 24960

tgtacatgta aggatacact gttgtttttg gcttatcttc tgtttgtgtg tttttgtttt 25020

acagttaaaa caaaagctga tggctcattc cgccttgaga acataaccac agggacatac 25080

accatccatg ctcagaaaga gcacctctac tttgaaacgg tcaccatcaa aattgcaccg 25140

aacacacctc agctggctga cattattgca acagggtaag cttatcgtgt ggatttggaa 25200

gcgccagtaa atatgctggc agccagtgta gaaccgaatg acctgtgatc tgtgtgtctt 25260

tgccgagctt aagaacttaa gaaagattag tacttttttt tttttttttt tgagagacag 25320

agtcttgccc tgttgcccag gctgtagtgc agtggtgcca tctcgactca ctgcaacctc 25380

tgcctcccag gttcaagtga ttctcctgcc tcagcttccc aagtagctgg gactacaggc 25440

gtgccccacc acacccagct aatttttgta ttttttagta gagacagggt ttcactatat 25500

gttgtccagg ctagtctcaa aactcctaac ctcaggctgt ctgcctgctt tggcctccca 25560

aagtgctggg attataggtg tgagacactg cacccggcca agaaagatta gttctaaatc 25620

ttacaaactg aaggtactga catgtcggat gcttaatgaa aagtcccttg cttcaaatta 25680
aaatgggctt gtgaaattaa aaaatctttc atgtctgact ttcacgatct ttcacaggac 25740
ccctctttgc ttaatctttg aggatttttg gaagatggta ttgttggttg agaaagctta 25800
ttggaagatg cttcaggtca ttggtgtagt tcttcctgct tgtagttact cattatacta 25860
taagtacagg cagggacatc ttgtggttga attcatgtgg ttttaccatc atctgaagca 25920
agattgtgta gtgccaagaa cttgggcttt ggagtcagat accagatcca cctgtatgta 25980
acctgcagaa cagctttaga cataacaggt gctcacaaaa cagtagctta gaaggagact 26040
gcagggtctg cttgggagaa gcgcggcatg agcaaagtca ggcagtctca cagcagcatg 26100
aggtggctgc gggtgtaaag ctgggtgcta ctgaagcaga atatactggc aggtgaaggt 26160
gatgatgagg ccaaccccat agcacctcct cacaccccac cggggcgtag gggctttctc 26220
ccactggggc caacggccca tgagtggttt tgagcattaa tataacaagc tttgatcatt 26280
gttttcaata gggcattctg aaaacaatgg catggagtag agtggctttg aaggggacag 26340
gacttgggca caggagctct gaaagcagag acgacaaggg cttgccttgt agggaagtca 26400
gggacagact ggtgagatat ttagggtgcc cagcttgaga ggtgtgatgg ctggggacag 26460
aaagagaagc cagttaggtg atgagggttc agatttggcc acattggtct gtaggaccac 26520
tgggtggagc tcttcaatag gtacttggat ggatggtaaa tcctgaagat ttggccgcag 26580
gtggcagcca ggacctcaaa tatgtatgtg caagtagaat tagaagaaca gtaggccaaa 26640
aacagagtgc caggtggccc aggggggacc tagggagcat gaggtcacag gagctaagaa 26700
agtaagtttg gggtaggagg tagtgcccaa cagggaggtg cagggagctg ggccctggtg 26760
aacacagtcc cctggcttga tcactgagga ggccacagtg actcttttct gtagacagat 26820
ggaggtcgag gtcaaggagt ccagtggtga atggaagaca gggaagcaaa ttaccctcag 26880
aaagtaattc ttggatttaa gtctttccca ccttccataa acactttgtg caattactct 26940
agatgaggca ctgaaaaatg cttcatacct aataattaat caaactagtg ttccagagat 27000
ggggctttca ccctaccagg gagacagact gaagacaaat acatctgtgg ccaggcttaa 27060
tgcctcacac ctgtaatcca tcccagcact ttgggaggca gaggtgggcg gatcgattga 27120
gcccaggagt ttgagaccag cctgggcaac ttggcaaaac accatctcta ccaaaaatgc 27180
aaaaattagc tagtttcata acctggtcta aaaataaata aataaagaga taaaaattaa 27240
aaaataaaat aacaagattt taaaaaaaga caagacaaat acatttatgc tgctaaatta 27300
tggcaagtgt tgtgcagaga agagatcagg gtgctgcaag aagagctaat ttagagtggg 27360
agctctgcaa ggctctctgg gttgcagaga ccttagtaaa gactaaaagt tagctaaaga 27420
aagatcttcc ctagtagaga agcaacatgc tcagaggcct gcataggata ggaaggacct 27480
gaaaggcagt ctgcgcagct ggaggaggtg ggtgaggtca gggagctctg gggccttgta 27540

gccttgcttt gggattgggc tttgctccct aatgcagtgg aaacccgtta ggagttgtct 27600

gttggttggc cgggcacggt ggctcacgcc tatgatctca gcactttggg aggctgaggt 27660

gggcggatca tgaggtcagg agttcaagcc cagcctggcc gcatggtgaa acccggtctg 27720

tactaaagat acaaaaggtt ggcggggcat ggtgatgcgc gcctataatc ccagcaactc 27780

aggaggctga ggcaggagaa tcgcttgaac ccgggaggca ggggttgcag tgagctgaga 27840

ttgcaccatt gcactccagc ctgggcgaca gggtgagatt ccgtctcaaa aaaaaaaaaa 27900

aaagagttat cagttggcaa gggagtgata gggatcatgg tactttaaaa agtttgtttt 27960

tcttcctggc tgctgtgtgg agaatggatt ggaggaaagc catggtgtga atggaggggc 28020

cagatatgag gcagtggtgg ttgtctggag gagatatggt agtggccggg cccaggcggc 28080

tggcaggcga gggagcgagg agtagatgga tttggtaggt cacatggaga acacacccac 28140

aggcctgtgc tgttgaggtt acaggtttct tatttgagcc actgggtaga tggaggcggc 28200

gctgttcacg gagatggagg aaatatattt attttctcct ttaaagtaga gtatttgggt 28260

caaagaaaga aaaatagcat ttgttgaaca tttttggaaa aatctctgtt ttttaattgg 28320

ccgacgtagt acatttgcct ttttagcaca ctgatacttc ttactatttc ttttcctttt 28380

tatttttttt ttgagacagg gtcttactcc gttgcccagg ctggagtaca gtggcgccat 28440

ctcggctcac tgcagcctcc gcctctcggg ttcaagtgat tctcctgccc cagcctcctg 28500

agtagctggg attacaggct aatttttata ttttttagta gagatgtggt tttgccgtgt 28560

tggctaggct ggtctcgaac tcctgacctc aagtgaccca cccgcctcgg cttcccaaag 28620

tgctgggatt acaggctgag ccatggtgcc tagtcttcct ttaaagtaaa ataaaaaaat 28680

tactcaattt attgtaaaaa caaactcttt ggcttctgtc caaatgactg tgaaaataga 28740

tttctctcaa tatccaccaa tattacctgt ttctgtccat atatgtattt aaaccctgtt 28800

gaaagaattt attgatacaa atcaattttt aagggcttca gtataacgga acctttttct 28860

accacccttt tggtttattt ttccagacta agccagtata gatttgaggt ttagggaaaa 28920

taggcacttc tttttaataa gaatgtgggc taaaacagat cttatggttc aaatatttac 28980

aacttaagga ggcaaagaga tttgatagga aatagacaag gtgatgtgtc ctctgacagg 29040

tacacaaatc aagagaatat gacaggtcct tgggaatgat tgtgtcagtg ttcgcttccc 29100

atccttttat tctgaaagtt cacagtaggg gaatgtgaga accaaaatgc atatgctagg 29160

tcagggactg ggacactttc tctgtggaga gccagagaat aactatttta ggctgcgtga 29220

gccatacggt ctctaggaca acagctgaga tagacaattt gtaaatgaat gggcagagcg 29280

caacacaact acatttttgg acaccacagt ttgcatttca tgtaattttt acatgtgaaa 29340

tattatcttt cttttgattt tttttttttt aactttaacc acttaagagt gtaaaattct 29400

ttttctcggc ttgcagttca tacacaaacg ggaggggggt ccttgggtgg tagtttgctg 29460
acccatgctg gagatagtga gctcatatgg ctgggcagtc ccttcactct gaagaaatgg 29520
ctgtgttttt catgattcct agatttcaca tacacaatct tccaccattt ccttgtgttt 29580
tccactgttt tttttatttg aggcggagtg gtggttccaa catttaatgt acccctaatt 29640
gtcagaatcc aacattcttg attatcttta tgtcagaagt aactaatttt tatctaaaat 29700
gattcttgaa gattctttaa aatgaacagt tgaaaagaca actgtgaccc ccagctagga 29760
tgtactatta cactttaaca caccagctgc ttcagaaatg aagaccagca ctccaaggat 29820
gatggtcgtt gtaggggggc ctctcgtgtg ttattcgagg gatgccttgc aacaccttac 29880
tccttattta gaatctcaaa tctgaagcat taccttaata gggcttttca gttataagtt 29940
acatttgtgg ctcctacctc ccaaggagtt caattttagc tttgcatttt ttaacttaat 30000
taccttttct gttccaatct ccctgtttgc tttcatccat gtattatata taatcacaag 30060
caaggaagac gtctttgata atcctgactt atttgatggc ctggtaatgc cacagtttct 30120
taggcagctt agagctcttg cttttaaggg ctagcccaca ttctcagtcc aaggccgctc 30180
cgctgtcaac gatccaatta cctgaaacgt ttttattatc tgcagatttt cttaaaattg 30240
cctctgcagt gggggattgc cagatgtcac ccggaacaca caaacagccc cactacgggc 30300
ccctgttctg tagaaagatg cccagtcttg cttctgaagg ttgcttttta aaaccagaat 30360
gaaggttgct ttttaaaacc agaatcatct ggactgcatt ctgtctcttc attaagatta 30420
gactattctc aggaacaagg gctgggagag ggctgcatct tttttggtct ataagagcct 30480
ttcctgttat aattgagtcc tcgtgctgga atgaatggtc ttctctttgc aggttcagtg 30540
tctgtggtca gatatcaatc attcgcttcc ccgacaccgt caagcagatg aataaataca 30600
aagttgtcct gtcatctcaa gacaaggaca agtctttggt caccgtggag acagatgctc 30660
atggatcatt ttgttttaaa gcaaaaccag ggacttacaa agtgcaggtg cgatgcattg 30720
ttttaaattt aaaatgtttt gagaaaaagc agtgattttt agaaggttta ttaggtcgaa 30780
tggggttaga gaaggagcat tccagtttcc aacttcttgt ttctaagtct ttacaatcct 30840
gcaccagtag aggagagtca gataggcatc cagctaaaaa ataatgccat ctgcctctct 30900
tttttccagt cttgatgttt attagtatca ggctgccagt cactcctcta ccttcaggct 30960
tctggctgct gtagaatgtc gatgagtcca agaggacacc aaaaatgaac tctactaatg 31020
gccgctcaaa aatggccata gtgcgtggct tctgggaccc caggccaaaa tggagcttcc 31080
catttcctgc tttgccttga cttcaagatg gctaggcagg gaccacgcga cttatccatt 31140
caagcaggcc gagaacgggg agacggtctg gcctgtggtc tcatcaccat ggtgaaggcc 31200
caagccacag ctggcccaag caagcaagag acaggacaga gcagaggtca gcccaccatg 31260
cctttcagtg cagtggccct gcccttgggg agagactgtt tccgtgggct gtcacccatc 31320

aaatcagcag ctgcccttcc tccctgtgga agagcaaata acacaaaggg agagggccga 31380

gaaggttccc tgttagactc tgctgtcctc tagggcaaag tggggtccct ccctctgtgg 31440

gaaacagggt aggagattgc gttctccctg cttccacaga acagcgtggt gtgtgggttg 31500

acagctgcct ttattagatg gtagctggct tcttgcgaag atgtccacat tgacaagtct 31560

ttggaagccc tttcggaagc atgatggact tctccaagga agggcagtgt cgtctttact 31620

agggatgaaa gagttgaccg ctgctacctt cacctgttcc tcttcttagc ccatgaaagg 31680

cacgcagaac aataggagga agaaaatgac ttaattccac caaagtgtca tccacatagt 31740

gttaacaggt tcagagctta tgctacaagt gatccgaagc cgggaaaagt gactgggatg 31800

tagtatggtt tctagtatgt aagtgaggcg tgtccgcatt gcgggcctgt ggggaattag 31860

gccagataga cttttgttca tacacatcgg tctgttgagg ctaatttttc atctgggtag 31920

aactgtgctt acttcacaca agttattcgt ggatttgttg tgattgtaaa atcggcctgg 31980

aaacgaacct ttggccttca aaatctcttt agccttggtc ccagatgaat gttctagcgc 32040

cctgtaggtc agggggaaga tctccccgaa tgcagcctct aacgttccat ccacgtttcc 32100

acaggtgatg gttcctgagg cagaaaccag agcagggctg acgttgaaac cccagacatt 32160

tcctcttact gtgaccaaca ggcccatgat ggatgtggcc tttgtacagt tcttggcatc 32220

agtttctggg aaagtctctt gtttgggtaa gatatcactg gaaagtaaga acacatagtt 32280

tcaaagaagt cagccggtag gagtgggatt tgggaaactt ttccttttgc ctttgttgtt 32340

tgctactgat cacctgcgtg cgagggaggc ttcttggagt caagtgggtg catgttagct 32400

tgaaagaagc actccgtctg cctccggcca ctgcctctta ggagctcagc agtagcaaga 32460

agctattaca tagggttagg attcaaacct gtgctgaagc cttataaggg gtcacatgga 32520

gccctccctt cttttccctg cagacacctg tggtgacttg ctggtgactc tacagtccct 32580

gagccgccag ggtgagaagc ggagcctcca gctctccggc aaggtcaacg ccatgacttt 32640

cacctttgac aacgtgctcc ctggaaaata caaaagtaag aattggaatg caacatcctg 32700

tggccctcac acacttcttg tctttgtaaa ctttctaaaa ctcaggtctc aaattgtatc 32760

acgaccatgt acctttcctt gttttaagtc gtctttgcct cgtgaccttg actgcattat 32820

ttagcgttgg acaagcatgg aggagtccag gccctgagct gaccgccagc ttgtctgcac 32880

agcccactgg cggcttctgt taagggaggg tgccccgacg tatggcattt tatttctaaa 32940

gcccagagaa aggagtgtga aatagttcca ctgatgtgtt ctactaactt agcagtactg 33000

gtgctgagta ctggccatgg caaggtactt gacctccctg aaactcagtt ttcccatctg 33060

taaaatagac gtaatagaac ctttctgcag aatataagac taaaagaagt aaagtttata 33120

aagcccgttg tgcagagagc cagctaagta gctggtgccc acaaatgtta cttccctttt 33180

ccttgccctt cttaagaagc cacttcttgc ctgtatttct gctttttcag aaatcagctt 33240

ggggctgctg gcagtggata gctcataaaa cagactgctt tttctggaat tatattaata 33300

ttttctgtta tggcagggct tggcagacta caaaaactgc cttttataag cgtagtttcc 33360

ttagaataca gccttgccca tttgtttatg gattctctat ggctgctcgt gtgtaacagt 33420

ggccaagtcg agtggttgag gctgacaaag catgtggcct gcaaaactga aaagagttac 33480

agaaaaagtt tgtggatccc tgaatacctt acttgcagcc tgatcttaca ggccccatta 33540

gatttctttc ttgatgtaat ggaaattggt tccagttttt ctttcttttt tttttgaaat 33600

ttagcctcgc tctgtcaccc aggccggagt gtggtggcgc gaccttggct cactgcaacc 33660

tctccgcctt ctgggtgcag gagattctct tgcctcagct tcccgagtag ctgggattac 33720

aggcatgcac caccatgcct ggataacttt tgtattttta gtagagatgg ggttttgcta 33780

tgttggtcag actggtcttg aactcctggc ttcaagccat ccaagtgcgt cggcctccca 33840

gagtgctggg attataggca tgagccacca cgctcgactc caggttttct ttagcagtaa 33900

tttattccaa atactgctag ttataaatat atgtatgtgt atatttctat ctttatgtct 33960

acatacacac ctgcttttta aaattgatta attcatgatc attgtttctg gtggcgtgga 34020

gggaggtggt gtgctccacg cccatgtatc cgtttttggt gtttgctttt gcagtaagca 34080

tcatgcatga ggattggtgc tggaagaaca agagcctgga ggtggaagtg ctggaggatg 34140

acatgtctgc agttgagttc aggcagacgg gctacatgct gagatgttcc ctgtctcacg 34200

ccatcactct ggtatgtacg gcttattgag tctcttattt ggaaaagcgc tcgccttgtg 34260

gatgtcaaga aagactaaca tcccaggaat attgtaaacg taggcaagtt agatttcctt 34320

ttctgcctct ccactcgccc acctgttacg caacgcataa tcaggaagca tttatactct 34380

ctcagtggaa ggacccctgt atttaggagg tttccttgtc ctggccctac actaaatctg 34440

actggtgatt cggggtgacc tttggtacag tgcagagcac actggcttta tattattagt 34500

aataatagct ttgggtaaat tcgtttactg cttctcagcc tcagttttca ccgaaaattg 34560

agatcttaat acctacttct cagggttatg gcagggctta agtgaaccct cttaggtatc 34620

aaagtgtcct tggtgtacac cgctctgtac tgccaagtga gtttctctga tatatatata 34680

tatatatata tatatatttt tttttttttt tttttttttt tttttgagat ggagtcttgc 34740

tctgttgccc aggctggagt gcagtggtgc aatctcggct cactgaaacc tccgcctccc 34800

gagttcaagc gattctcctg cttcagcccc ccgagtagct gggactagag gtgcgtgccg 34860

ccatgcccag ctaatttttt tctttttttt ttgtattttt gttagagaca gagttccacc 34920

atgttggcca gactggtctt aaaactcctg acctcagggg atccacccgc gttggcctcg 34980

cagagtgcta ggattacagg tgtgagccat tgtgccccac cttctctggt gtatttgttc 35040

cagtgcaggg gaagaagctg taaacaggat cttagcttct tgttgactgt ggtctctgtg 35100

gcctgaaagg cagtgtgaaa tgggtttcag gagcacctcc gtagatccct tcgtgaattt 35160

tctataatgt tttgatgtcg agcggcggca gatgtcatcg tcaggtctta gtttctctgt 35220

agagaaacta gaagaattgg aactggtgtt tcatgtgcca acttactccc ctaactgctt 35280

aattacaaaa actcgacagg tcgatttgtt ataggagatc gattaatatc cggttcataa 35340

taagtgatag atatttagga aacctttccc ttccagcagt ggagtggatt tccagctctc 35400

ttgtaataag atcctttttt tttttttttt gacacttcct taaccccttc tttatctcct 35460

cccactgtcc cctcactcgc cgggccccag ccacagtggt cctctgcagt cccacaccaa 35520

gctcactccc tcctcggggc ctttgcacat actctcccct ctgcctggag tgctgtgtct 35580

tctccagaaa cacgtgttgt tggtttttcc tcctcattca ggtgcagccc tattgttcag 35640

gagagatgtg ccctgggacc agcgagctaa agacccctcg gtggggattg ttcttctctt 35700

gctttcctgt ttattttcac agcttcgtta ctgaccagtt gagatcacta atttatttgt 35760

tgatgcgttg attttgtctt cctgtattag aacataagcc ctgtgagagc aggagccatg 35820

gtcatgactc ccaaggccca gccttgtgtc tgtcacatag caggtgctca ataaatatta 35880

gttgattgaa tgaaaaatag cacacttttg ttggaagttt tgggaggttt ttaattttcc 35940

tgtggcaccc aactgctcca atgattaatg ggcaaaacag atgactggtt tggaaagaga 36000

aatattatta atcatcttgg tgtctccatc tctcttagta gtcattgtat tggctttgct 36060

tccttaggaa ttttatcagg atggaaatgg gcgtgagaat gtggggattt ataacctctc 36120

caaaggagtc aaccgattct gcctgtccaa gcctggtaag tttggaagga ttgatgtgcc 36180

atgaattaga aaaatggaaa ggcaccagag gatggttttg aaggcatttt tttctacctt 36240

agttctgttt gcatcaagct ttcaatttct gtgtgttcaa cctgctcttg tttttctgtc 36300

tgtcttgggt ttgagttggg tagacaccac tgggcccttt cacctccaaa aatataccat 36360

tggccaatgg cctctcttta tagagctgtt ttaaaatttg aatcatttcc cactttgctt 36420

aaaatctttc agtggcttct cgctctttct catttttttt tctctttttt ttttttgaga 36480

tggagtcttg ctctgtcacc caggctggag tgtagtggct tgatcttggc tcactgcagc 36540

ctccgcctcc tgggttcaag cgattcccct gcctcagcct ctcgagtagc tgggattaca 36600

ggtgcccacc accacatccg gctaattttt gtatttttag tagggatggg tttcaccaca 36660

ttgtccaggc tggtctcaaa ctcctgacct aaagtgatcc gcccgcctca gcctcccaaa 36720

gtgctgggat tacaggtgtg agctgctgca cccaaccttc tctctctttt ataatggctt 36780

tttttggagg cagaatttgc atatcataaa actcacctat ttaagatgta caataaaatg 36840

atttgtagta aatttatcaa gttgcacaac catggccaga atccagtttt agatttgttc 36900

atcacccaca atgtaagatc ctctgtgccc gttgacagtt aatccctgtt tctagccatg 36960

gccagccact gatctatttt ctgtccccat ggattggctt tttttggacg tttatcatac 37020

gatgctggta ttttgtttgg cttcttttat ttaacatgtt tttgaagttt atctgtgtca 37080

tggcacatat cagtagtttg ttctgttaca ttgctgtgta gtattctggt gtgtggttat 37140

atcatatttt gtgtatccat ttatcagttg gtggatattt gagtggtttc cagtttgacg 37200

ctgttatgac taatgttacg gacattcttg tacaagtctt tgtgtccgta tgttttcatt 37260

tttcttgttt cgatacctgg gaatggaatt gctgcattgt gtgataagtt tatgtttaac 37320

cttttaagaa actgtcaaat tgttttccac agaggctgtg tcgtttgata ttcccaccag 37380

caatgcacga gggttccggt ttcttcacct ccttcgccaa catttgttat tgtctgtttt 37440

attatagtca tcttggtaga tgtgaagtcg tgtctcatgg tggttttgtt ttgcattttc 37500

ctaatgaata attcagtggc tcctctgttt ggttcccaag ataaaataca aactccttaa 37560

cctgatctta gctcctgtgt aacccagccc tgtctgccac tgtctctact ccagccactc 37620

tgtccttctg ggtgtcagtt cagctgcctt tggctacatg taacaaaatc catacagcag 37680

tgccattggc aagtggtagt ttatttggct caataaagaa gtgtggcagt gcgtggctgc 37740

cagcatgggt tcagcagctg gtagatgtta ggggtggtgt ctttgtgatt ctttaggcct 37800

ttcccttcta ttttttgcct tgtattttca agatggctgt tgttagaaaa cttatgttta 37860

aaggagaaag tcaaggagag ggcaggcaaa catttccttg cgccctctcc tccagtaagc 37920

tacttgtctg tattattggc cagaaccgtg ccacatggtt acttctagct gcaaggaggg 37980

ctgggacagt tggatgtgct acccgaggct gagcacgttg ccaccctcaa cataaccagg 38040

gtttgttctc aaggcaggaa ggatgcctgt tgaggaaaca atgagccagc ctgtgaggca 38100

gatgctgttc tctgtggaca tgtcctcctg cccctcctag ctggggcctt cttacccatc 38160

agttcagtat tgcttccctg gggaaggatg gcctccaggg ccacttaggc ctgcctccta 38220

cactcttgct atcagcactc acttctatcc ccttcttgca tgtgccacaa ttgatatgtt 38280

tgtggctgtc aaatgctccc cctctctgtg gatggtaaac tccatgaaga cagggaccac 38340

tgctctgtca ttccccactg tgtaccagta cctagcataa tacctggcca ggaagaggtg 38400

ctctgtcgat atttgttgaa tgaaagacca aacaaaacaa acagagaaac ctgatttttt 38460

tttttttttt gatggtaact actctggggc ttgttgagtg caatgaaaac agcagggtgg 38520

ttttagctaa tttcaggttt ttgtaggaag tgtggccaaa atattggtta atgatgattt 38580

gggggaaata cattgttatt ccttgtatct tcctgaaatt gactgtgttc ctctggcaag 38640

tgatgaaatg tacccctagc cggagtgtcc tcccacacag actcacactt ttctggggct 38700

tcccttactc cttacctcct ttttaggaat atttatagga agaaattgag gctgtggtta 38760

tctcttggat ccttaataga atcttttaag taggaaatta tcctcagtcc aatttgcagt 38820

agaaacgtac tttctgttcc ttctcgtgca tgggtccgtt cacctggtct gtgcagattt 38880

aactactgtg agttgttacc ttatggaaaa tgaaaatcat gcgcctgtga tcgcgccatt 38940

gcatggatcg ggttattggt aagacgcctt cataggcagt gaaaacgatg cctgcccagt 39000

atttctgatc ccattattgc agatctgcga tacgtttgag ttttcacatg caaagagctg 39060

ccactcgatt tcagatgtcg gaattgctcg gtgtaataga tgccattctc tgaaaattac 39120

ttttgatttc ctgtctgtag gtgtgtacaa agtgacccct cgctcctgcc accggtttga 39180

gcaagcgttc tacacctatg acacgtaagc ctgggaattg aatgctttgt ggtgtttgta 39240

tacattccgt ggaggattct tcatttactt cagagaacaa aaggagtttt tctgtttttt 39300

tttctgaaag aggcaaggtt aggccttcaa cagcatttct agataaggat tttaagacac 39360

ttggattagt tactgaggaa ccggagacag gatccctttc ccaagaattc tgtaacccca 39420

ggatagaggg ctgtgttttt gaagtgcctt aaatagaagc tttgtcaaat ctcccagccc 39480

tcaccgatgg gcgtctgtca agttcaagtc tgccattttt gtatttttac agaagtgtgt 39540

gggtttcact gggaattgga agcataaagt tgtggagtgg agaattagtt tactctttaa 39600

aatacttctc tggatctcca gagttatttt accccagact aagagcaatt ttctgtagta 39660

tatttagggt gaagaaggat gcccaaaaag ggtattgctg atgatcttct gagaatgagt 39720

aggttctagc gtttcctctg ctttgatttg taggtcttca cctagtatct tgacattgac 39780

agccattcgc caccatgtcc ttggaactat caccaccgac aaaatgatgg atgtcactgt 39840

gactatcaag taagatgagc gttctgcagt gggccgagag tggcgggaga gggtgtggat 39900

gaggcctggg gagtctctaa taggctctct ggaaattagt tttgatttct tatctctaga 39960

tgtatataca aatggccttc cctctcttat aggacattgc cgtcaattcc cagcagcctt 40020

gatcctttcc ttttctgatt cagaaattac ctgtgggtga ggaagagcgt tgcgacagct 40080

tgtttcagtg tcttcaattt ctgtttgaac tctttgtact tctcatcttt gctaaggaaa 40140

aaaaaaatct ctggggtttg tgcattgtat ttcaagtgtt ttagcaaatt caaattgcct 40200

atttttcttt agacttttgc ttgaaggctt gggaaataga gatggcacct ggtataagag 40260

aagggtcgtg agagaggaag tatttgaaag gcaagtccct caccgaaggt cacgttcatg 40320

tgaatgtcac ctttcaggga atttttgcag tgtggtgagg tacctgtgac gccccaattt 40380

tcttgagaaa tgaacgagtt ttggcaacat gaccgtctct gtcaagtaga atgggcattg 40440

cagtccctgt ctccctcctg cccctggttg aagtcacctt tccagctatg tttccatctt 40500

tctgtccacc atctgtttat ccatccgtca tccctgcaga gtacatgatg actgaaaaac 40560

tgagagtctt cagtgtttag gcactgggct tatgaggcca cagcccctac acttatggga 40620

ttgacagtct catgggggga acagtggtca gatactcaca tgcagaaccg taaatgacaa 40680

ctgttctgag tgctttgagc ttgaccttgg gggcatgcag gacaggtagg ctgcgggggg 40740

ccaggccatg ttaaggtcca tagtctttgt cccatgatgc aattgacatg ttttacatag 40800

aggaatgaca tttttcttcc tgaaaggtcc ccctattggc cgagtggaga acagacagaa 40860

gaggcctgag agtgggaggt ggtggaggca gggggaggca atgggggccc cagactagtg 40920

gctgcggaga tggagagcga cggactggtc ggagagtcgt tgagggactg agttgatgag 40980

acatggcact aaagtgacca ctgggttagg gcgggagaaa tgtcagggct ggccctggtt 41040

tctggtgatg gggagccagg tttttagctg gggtggattt tgccgaaggt ctgttgctgt 41100

cttttactat catttactgt ctccctgctt ggaaagtatg aattactgat aggactagca 41160

atctaccttc ttaccagtag taactgcata ttacctcctt tcagcttgat tacattcatt 41220

tttgtaactt cacaagggtg tttgtgttgc ttgcttgtgc cggaggttgt atggaatgca 41280

tcttccagct agacagagcc actcggattc cccctgggga tcttgtactc agcagaccca 41340

aaacccgact gctgacagtt gccctccatg agcccctctg tggtcttttc tgattcagat 41400

atggcagctc ccgctttctc cttgtttctt gattcttatt tcccttgaac cctaggccca 41460

gccaattccc caacaaagcc ttggctttac aatcaaaata cacccgtcat ctagtccctt 41520

tttaccactt ggccctcagc cttggtctga cccccctctg gggtcactgt agcagcttcc 41580

cagctgggct tcgcttctta ccacgtggcc ctcagccctg gtctgacccc cctctggggt 41640

cactgcagca gcttcccagc tggccttcct gtttccgcca ttgctttgtg tattttccat 41700

gaaggagcga acgatttctt ttatcatacc attcctcgac tgaaaactcc agtggctcca 41760

ggtcggcacc aagcctgtga agcctcagtc tgacatctgc tgccccttgg acctctctgt 41820

tggatatgac tcttccgtgg gcccctgtgc cccaggctgg actggtgttg ccattgcttg 41880

aacatctgag tgagctttct ctccaaggac tttgcacttg gcatcgcttt tcccaagcta 41940

ctgtttaccc agagagctgc gtggcgcatt ctctcccttc cttcagacct ttgctcaacg 42000

tcaccttatc agaaaggcct tgactgtccc tttaatgtaa aacagtgctc cgggctctcc 42060

gtcttcccac actgctttat ttttctttat tgcacttttc ctctggtatt atttttctct 42120

ttattttcag ccccttccca ttgaaacgtg gattccacag gggtaggaag tgtggtctgt 42180

tttgttgatg ctgtatcctt ggtgcctagt tctctatcag agcagttctc agcgaaggta 42240

gaatggagaa cagaggatgg tggtgtggag aaaactccat gtcctacatg gaagctacaa 42300

gcttatatcc agtcgccata aaatcaaccg tatttcattc cagcccagat ctaccagata 42360

ctgccaccat cactgcgaag ctcagttgtt attttttgtg aaactactag tttatttaaa 42420

acagaagact ccgccactgt ggtgtcatga taagatgtcc agtttgtaac tcgcaagtcc 42480

atactcgtag aaaggtcaaa ttcccactga ccacaaggat acgcaactat gtcctagggg 42540

ccgtctttct tctaggtctt ccatcgacag tgaacccgcc ttggtcttag gccctctgaa 42600

gtctgtgcag gagctgcgga gggagcagca gctggctgag atcgaggccc gcaggcagga 42660

gagggagaaa aacggcaatg aggaaggcga agaaagaatg accaagcctc ccgtgcagga 42720

gatggtagat gagttacaag gccccttctc gtatgatttc tcttactggg cgcggtaagc 42780

tctcttgtgc gtttccctac agtgtcctct gttttgtggg gacaggaccc gccaaactga 42840

agtatattaa ttattttagg tctggagaga aaatcactgt tacaccgtca tctaaagagc 42900

tgctctttta tcccccttca atggaagccg ttgtcagtgg aggtaaatgt caactcagcg 42960

agcgaatgtc acacacacct ttgcagttgt tcccttgcct gcatttacaa gtagatttgt 43020

gatgttatag tgaagccatc tttgtaagcc accttacatc ctctctggca cacagatgtt 43080

actgttggtt ggatggatgg atggatggat ggatggttgg gttggatgga tggatggatg 43140

gatggatgga tggatggatg gatgaatgaa tgaatgattt agataaataa aagtaacttt 43200

tgctgttaga attgagttga tctttttgga aaaggacttg gttttctttg acattatcag 43260

gtcgacacca gaaggtagat gaagcacaaa gctgccagtg atgatcttta atgatctttc 43320

tgttgttcgg cagagcatga tgggagggtc tttttttggg gaatggatga ctgaagtgat 43380

cacttgtgga gtatttgtct tttgccactt attatttttt tcccagcact ctggcttcgt 43440

taagttgtgg gtgttatgtg gttcgtgttt ggaagcagag ggtgttgaag agcgtgactt 43500

ctagcaggca ggctgtttgg ggcggtcttt ctcagtgggt cctccagagg ctgagcagca 43560

cggcctcctg tctgctgtaa agtgttccct cctgatctgt ggcctcctga gaacctagga 43620

agactagtag taaaaaccca gccttcagcg ttttttgatt ctcttgctaa agtccttcac 43680

actgagaaag gcctttcagg gatggaaaga ggcttgggct gtatccgatt attgagctcc 43740

ccggggtgtt aaataaccgc atttggaact agcttctaag atctgtttta gccacttttg 43800

tcatgggaaa cccccaacca agaagctcct gtctctgcta ccaagcccca tccttgtgtt 43860

tatttcttcc cctcttagaa agctgcccag ggaagctgat cgagatccat gggaaggcag 43920

gcctgttttt agaaggccag atccaccccg agttggaagg agtcgagatt gtcatcagtg 43980

aaaagggggc aagttcaccg ctgatcacag tctttactga tgacaaaggt gcctacaggt 44040

gagcccggga tagagacaca tgtgcctggg atcagcgtgg gagtcctctg aagaaactgg 44100

ggcccacatt tccttgggct tggtaaggct tcctgcaggg tgtgaacaaa gccgttgcta 44160

gcattctgct ctctcctctt cagtgttggc cccctgcaca gtgacctgga gtacacggtg 44220

acctcacaga aggagggcta tgttctgact gcggtggaag gaaccatcgg agacttcaag 44280

gcctatgccc tggcaggcgt aagctttgag gtaactaaca ctgtattttc aaaaggcagt 44340

tatactgagg tataattaac atataataaa ctgcacaaaa agggtacagt gtggtagtct 44400

tgacctagga atacacccat gaaaacacct ccacaattaa aatagcacat ctgtggccct 44460

caaggtttcc tcttgcccct tggtaatccc tccctgccac ccttccgctg agctgatgat 44520

ctgcttttca tcaccattgg ttagtttgca ttttcgagtt tttataggtt ggtgcaaaag 44580
tgattgtggt ttttgccatt gagaataatg gcagaaaccg caatcacttt tgcaccaacc 44640
tgtatataaa tggaatcaaa cgggatatac tgttgttttt tttttttttt tggtctgact 44700
tctttcacgt agcataattt ggagagctgt tgactttagc atgtttgtgt accttctggg 44760
aagtagttaa agctgtcatc cataactgtt ccattttgaa ggacacagaa tgttcttact 44820
gaggcgtttg ctgcctagct cccttccacc ggtcctttct gggaagaagc gtgctgcgat 44880
tgtacagagt gggtctggag atcgttgaga atgaatattt ataaactata ggccaaagga 44940
aaagagtgac agattacatc gcataaaaat ttaaaattct atattgctga atacatttgg 45000
aaccaaactg aaagatattc aaactcggaa gaaatgtttg tatcataaat aatatcccca 45060
ttttacaagg agctcctaag attggtaaga aaaagacaac ccgctagaaa aaaaatgaac 45120
aaagattatg aaaaggaaat tcatgcaagt aacgcaggtg actaataaac acagaaacac 45180
aaagcctctg tagttactga ggaaatgtgc taaggaagtt acagtcgaaa cactgttttg 45240
cagggattag atttgctgta attaagacaa tctctatttt ttggtaagtg tgggttttcc 45300
caagcataaa taatttatgg gactgtaagt ggttacagcc actttggaat gcaattagat 45360
agtatctttc tgaatttaaa gtgcatgtat ccaacaattc tacttctagg aatggattgt 45420
tcaggaaata aaaattaagt gcctagagat acatatttgg ggatattcca attgtctctt 45480
gtgtaccaca agagtaggtg ctgtggtttg tcaaaccctg ccttagaatg ctatgcagta 45540
gttaagaaac aggcagattt ctattggctg gcagaagaga accaggataa attgttgagg 45600
aagagagcaa gaactgtaca tataacatgc cattttattt tcacctcctc cacccactca 45660
atgaacttgc cagtctgtga acaataatat gtaatgttta taaaatttgt gtgggcatag 45720
aaagagttct gggcggggca cggtggctca tgcctctaat cccagcactt tgggaggccc 45780
aggcatgtgg attgcttgag cccagtagtt tgagatcagc ctgggcaacg tggcaagacc 45840
cgtctctata aaaaatacag aaatgtagtt gggcatggtg gtgtgcgcct atagtcccat 45900
ctcctcagga ggttaaagct gaaggattgc ttgagcttgg gaggcgaagg ttgtagtgag 45960
cccaggttgt gccactgcac tccagcctgg gtgacagagc aagaccctgt ctcaaaaaaa 46020
aaaaaaaaaa aaaaaaaaaa agttctagaa ggctacacac caaacttaca aaacttacaa 46080
cagtagttac ctgtggggag gaagattagg tcccctgtcc tcctttgagt aacaataaaa 46140
gaaaaaaacc ttgaaaaaat gacctcttga cttgtggaac tgttattttc atagttagta 46200
ggagtttaac cttgctctgg tctcaccttc tcatccccag ataaaagctg aggatgacca 46260
gcccctcccg ggagtcctct tatccctgag cggtggcctg tttcgttcca acctcttgac 46320
ccaggacaac ggcattctga cattctcaaa cctggtaacg tgttctgcaa tttaccacct 46380
gcctgtcttc cctgagagag agccaggatg cagcatgcga gacttacgtg ttgcttgaca 46440

acgtgagaag agaaagccaa tgtggagtgg tttcagtttc ttgggggccc acggtcatta 46500

gagtattgct cttactcgaa cttaatgctg ctgattcatg ttcccttcca cacgcgcttc 46560

ttgttttctg atcacccact tgtcactaag acagtgtaat taatttccct ggccacaacg 46620

gctgctctct taggcatctt ctcgttttgc cacggaatca tgcttatgat gctgagtctt 46680

gtttggtggt tagcttgcgt ttcttctgaa aagacattcc acttggtggg aagagagcag 46740

cagtttttct ttcaagatgc agtcggaggc cctgactggt ggggattcag gaagtgtgtg 46800

ttagttggtc atcttgtcag tcatggtgac aaagtggctg tggtggggct actggagacc 46860

gggcagcggg ggcatctgaa gacaccactt tgcagtgatg cagactgctc cttacttgct 46920

gtgtcctatc aggcaagtta ctttgcctct ctgagcctca gtttctttat tcattaaatt 46980

caaatatgag ccaggtatgg tggcccacgc ctgtaatccc accgctttgg gagggtgagg 47040

cagtaggatc gcttgagctt gggagtttga gaccagcaac ataggaagac cccatctcta 47100

caaaaaataa aaaaattagc tgggcatggt ggtgcatgcc tgtggtccca ggtactcagg 47160

aggccgaggt gggagatttg cttgagccca gaaggttgag actacggtgt gctgtgatcg 47220

tgcactccat cctgggggac agagtcaggc cctgtctcag aaaaaaaaaa aaaaaaaaga 47280

aaagaaaaat taaatatgac ttctacctct ctgagtcatt gcaggcaggt gataccatca 47340

gctgccgttg ttagagttgt gattgccact acctgactgt gagtggtgat tatagagaga 47400

gggttactat ttctggctgc cagggaagcc atggcgctcc cctggtgtaa ttctggtcca 47460

tcgtgacatt cgcccctcac ttttaagcca tgcctagatg tggctgctga ggctcagtgt 47520

gattatcttg gtagagccct ggccagtatt acttcaaacc catgatgaag gagttccggt 47580

ttgagccatc ctcacagatg atcgaggtgc aggaaggcca gaacctgaag atcaccatca 47640

cggggtaccg aaccgcttac aggtaagtgc cctggccacc ccactctctt ccagggctgg 47700

gctggtgaat cacattcagg cctctgttgc ctggaaacgc atcccaggct tcacactgat 47760

tttacttggg agggaaggga gatgagatat gagggcaaag gagttttgac tgctgccctt 47820

ctctcctagg agctcaacct ggtggttcag tctcaaattt cctattttgg aattggcttg 47880

agagagccta ttgagttgct aaaagctttt atttattttt tatttttgga gacggggttt 47940

cattcttctt gcccaggctg gagtacaatg gcgcagcctt ggctcactgc aacctctgcc 48000

tcctgggttc cagcaattct cttgtcctgg cctcccaagt agctgggact gccaccatgc 48060

atggctaatt tttctatttt tagtagagac aagatttcac tttgttggcc aggttggtct 48120

cggactcctg atctcagatg atctgcctgc ctcggtctcc caaagtgttg ggattacagg 48180

cgtgagccac cgcgcctggc cacttttatt ttttaaaaga gtttcaaacc tgaattaaat 48240

tattaaaagg agaaaatgtg ctcagttgaa taaaacttca aaatgctgaa aagagtatag 48300

tgtaaaattt cccctctgtc tcgtctccta gccacccaag ttcctcttct gggagacaac 48360

cagtacttcc agtttttctg aatgctccca gctgtagata gatagataca catgagtgtc 48420

tggcaaggca cagtggctca cacctataat cccagcactt ttggaggctg aagtgggaag 48480

ttctcttgag tctggaagtt caagaccagc ctaggcaaca tagcaagacc ccgtctctac 48540

aaaaaaataa aaatacaaaa taaaaaagat acacatctat agatagagag atgcatacat 48600

atatacatgt atacacacag atacacacat atatgtgtat tttttgtaga cttatacaga 48660

atcactttag aggaagagta attggaaaga ttgggtttag aaaaatggac cattttctac 48720

tgaatatatg gagtgaaaga ggaagtgagt tatttccatg ttattgaaag ctccattatt 48780

tcaaataaag cctagaaaga ataaaataaa gctttagcct tcttaaatta caattttcca 48840

gagcatccct caaaatgtat gagaaaagaa attgatggca gcttatccct ccggcaggaa 48900

ggtgtggttt ggaagtgggc cggccacact gagttgcctg ggtcattgag gcacagttat 48960

cccctgaact attttgcccc ataattgttg gtaaacggga aacacttggg tccttcagat 49020

aaccatgaga atatctcatt cgtgcctgtt cttgccatac tagttgctat ggcacagtgt 49080

cttccttaaa cggagagccc gaacaagggg ttgccatgga agcggtgggc cagaacgact 49140

gcagcattta cggagaagac accgtgacag acgaagaggg caagttcaga ttacgtggat 49200

tgctggtgag acttggaatg ggttttcttt ggggactttt ttttcatcct gtgtccagaa 49260

gtatcttgtg gtggccccaa gactgctagg agtgggttgg gcgagagggc tgggggtggg 49320

gctctggagc cctcccagtt cttaccttta attggtagag tttgcctttt ttctagccaa 49380

caggtcagat caaggatctg tggcacaatg gagttggaaa accatgggtt tggtagggct 49440

tttccctagc agtgtgtggc catggcttac gccagggctc aagccaaacg cctatggagc 49500

cagaagagca gtatgaacgg gggagaggag gctgtgcgaa atgataggga gtggtgaggg 49560

ctgtggagtg ggcacggcct gtggaaaggg acagcctgtc acttggttcc agcttattgt 49620

agccatgggg gaagacagag tcagggttgc cagatccttg aaattttaaa aagagaagtc 49680

agaaattccc acttcccaga aataataccc aacacaagca tttgggaatg cttatggtgg 49740

acattccaga tatccatcaa tgtgtatata cgggtagagg ggaggggtgg atggagagga 49800

aaaacatctt agaagagtaa gagcacatag tatgcttgga attttgaaat aagaaaccaa 49860

atttgattta ttttggggta ggagaaaatg aaactgagtt gggatggaag gagtcgcaaa 49920

atttcagatg tttcaacact aggggtgttc ttaaaggaac tctggactct taaggttaac 49980

aggccacaga ttaggaaatg aggggagaga aatgctcctg tcttaatgat ctcgtgcagc 50040

caaaccaggc ttggaccagg cacgatacga caagccccct ttctagagag ctgactcctg 50100

agtttttttg cagccgggat gtgtgtacca cgttcagctc aaggcagaag gcaacgacca 50160

cattgagcgg gcgctccccc accatagggt gattgaggta aggcattcag tgctgccgct 50220

gcacctgggt gtgggtgcct ccctgatcag aagtcctccc gtctcctctg gctgtctgcc 50280

tttcatctgt ggcgggggga actttcatcc taattaaggg tcctcttaga atagtgtcat 50340

cttcacaagc agcatcttat gtgggttttt aatgaacagc gttgctgtca tgcatccaga 50400

aaggaaattg gaactgttgt atcctccccg cgacttgccc tgtaagctct gatcattatt 50460

ctgcaagtta aggaacttaa ggcctaagca gcatttcaaa tcaaggacaa ataatagaac 50520

atgggcttaa tgggtggcag ggccacgcag ttatcttagg ttcagatgac ctttttcctg 50580

ctcccatttt gtgggcagag tcttttgata ctcaagatca tgacctagct aaattaatcc 50640

ctaaagagga catccttcga gtcacagaat gtggcctggg aatcatttga agtaaggctt 50700

ttctcttact gagtaactct cattgggaag actagagggc ttaggtaggg taagaggggc 50760

tgcctcttag tggctttatt taggtggctt cattatgcat gaagattgtg agggaggata 50820

gaacttttga cctaggaagt ggatttcttt ttctttttct tttttttttt ttttgagacg 50880

gagtctcacc ctgtcacgca ggatggagtg cagtgccgtg atctcggctc actgcaacct 50940

cctccttgtg ggttcaagtg attttcccac ctcagcctgt agctgggact acaggcacac 51000

accatcacac ccggctaatt tttgtatttt tagtagagac ggggtttcac catgttggcc 51060

agattggtct tgaactcctg acctcaggtg atctgctcgc ttcggcctcc caaagtgctg 51120

ggattacagg catgagccac cgcactgggc cggaagtgga tttccttgac gtctttacgg 51180

tgttggcatt ttaaatggct gggatgactg catctcacac caaaatgctt tgcatgttct 51240

cggtgtagta acttaggcca ggatcactag cataatgtgg actgtggccg ggcaggtatg 51300

aaagggaagg gggccggccc tctgggatct cgcaaactcc atgtacagac ctcacgcaga 51360

gggggtgctg tgactctgcc cctgctctaa caggcaagag catccttctc cttttttttt 51420

taagagaagc tggaaaatct ggcttcatat atgaaatttc ttaattttta acatgttggc 51480

aagtaattca aacgtagaaa aaaaatactg tgctttcgaa atgaaatgtg tctatgggcc 51540

agggacatgc ccctgctcta acaggcaaga gcatccttct cctttttttt ttaagagaag 51600

ctggaaaatc tggcttcata tatgaaattt cctaattttt aacatgttgg caagtaattc 51660

aaacgtagaa aaaaaatact gtgctttcga aatgaaatgt gtctatgggc cagggatagc 51720

catcttctgt cagtttgtga ccttggacgt agggtactca atgacaaact tcgcttcctt 51780

aatgtaagaa gcaaagtttg ccatgaagca gctctcaggc catcggacag ctggcataga 51840

agttcctcca ccagctttct cattactggt attccctctt tctctctcta ggttgggaat 51900

aatgacatcg atgatgtaaa catcatagtt ttccggcaga ttaatcaatt tgatttaagt 51960

ggaaatgtga tcacttcctc tgaatacctt cctacattat gggtaagtcc agacttttaa 52020

gctccaagta ttgtgttccc tttggctttg agacaagtac gaatgggatg tttttgggtt 52080

tgtgcctgtc tcgtgcctta ggtgtgacag gtggaggtgc tccagagcgc cgcctgctga 52140

gggttgaggt tgagatttaa gaaccttgga ccacaacacg tatttgggag agtgattgcc 52200

agttgtggga gagaatgtgg tccagtttca gataactgtc tgccatcagt taatcttcca 52260

aagaattata gtagaagcct gatcaatgat caatacaata tggaatctaa cgtggaaagc 52320

caattttaaa gaaagcgcct tgtaacttaa aattttctct ggagtggagg tcgtattaaa 52380

tgttcttgca ttggtttaaa gaaatgcctg aggctagata atttataaca aaagaggttt 52440

cattggctcc tcgttctgta ggttgtacag gaagcatagt ggtgtctgct tctggggagg 52500

cctcaggagg cttccaatca tggcagaagg tgaagtggga gcaggcatgt catgtgggag 52560

aagctggagc aaaagagaga gttggggcat ggtgccacac acttttaaat gatcagatct 52620

cacaggactc ataacgaaga cagcaccaag ccacgaggga tccaccccaa agatccgatc 52680

atctcccacc aggccctacc tccggcacgg gggattacaa ttcaacatga gatttgggca 52740

gggacaaata tccaaacagt atcagaggtg gtggacggtg ggctggagag caggtggtct 52800

gggctttctc ttgtctccag tagtgactca ccttgctgcc tgggccagtc acttcatttc 52860

cacatgcctc gacttcgtcc tctgcaaaac ctagattttg gactcagaga cctcttataa 52920

tcttgttcag ctctaacaaa atctccatgt taccattaat gaagaaatgt ttataaaata 52980

ccctgcagac cctctacgga tgttagtgat ttgctctggg ggtacattgt taagatgcac 53040

ttgtaatttt ataagcagtt aagtatttgg catgggtgag aaagtgtgtg tgcgctttag 53100

tccactctgt tcagggcttg caggcgtgtg cttgtaggga ccaggcaggt aatgtcagag 53160

agtgcagagg gttgggtagg gtgggggcca aactggggaa ctcctgcccc atccatgagt 53220

ggcacagggg aggggaagag gcagagagga ggggagggcg ccactctctt gctcctgagt 53280

gttgctgtgc tagaattgag gctcagggtt ggtgtcactt cccagttttc cggagaagct 53340

ggaaattaga attatttgaa atcacctgat ttttaaatat agtcggtgaa ttcagattta 53400

cttaagatgt tatagaagaa aaaagaacac ataagggatc ttcaatgcga cccataacct 53460

tcagtctgtg actttcattt taggaaaatt ccccttgtgt gttatgtaga aaggtgccag 53520

tgtgggccca gggaggtggt cctggcattc tcttgcggca ggtgcactgt ggcatcctca 53580

cagcctttgc catcagccag ggctgcacct taaccctgct catgccccat gccgtgtggg 53640

agtgagaaca ggcaagaacc ccagaaataa agccagaaac aaagtactcc agtgcctcct 53700

gaagcagcgt ggagagccac gtacatgatg gaatgcgttt tgagtttaac agattggtgg 53760

agttaaggtg acatcatatg atgaggcagt gcaaggcggg aaggaccctc aggagtcgtc 53820

agggggttga gtcccaggct ctaccatgtg taagttgtgt gaccttaggt gactttggct 53880

ctctgggtct ccatagggat aataacaata cctgttttgt agtcaggatt ggcgataatg 53940

tagataaaat atctggcgcc tgcctggcat attacagatg ttcaacacgt gttagaagct 54000

atttttaata ttactattat attaacttat attactattt tgagcaagaa catgggtagt 54060
cagaatctga tttgaatctt aatttctgtg catgatacat aactggatgg agtctcagat 54120
gacagaggga tgtgtgatgt ctatggaggg agctttcttt ttcaaatatc catgtttcca 54180
aagacaccca cccagaaagc ggctgtcctg aggatgtctg ctatgaaata ttgattgtgt 54240
ttcagtttca aagcatgtct gactttgttt ctccctccag gtcaagcttt acaaaagcga 54300
aaacctcgac aatccaatcc agacagtttc ccttggccag tccctgttct tccatttccc 54360
cccactgctc agagacggcg aggtaatgcc tgtggccgga ttctaccttc tgcctttgtt 54420
ttaataattc tgctgtttag tttaaggaag cacagttctc tccttttctc cctaaatacc 54480
actctgcaga aatacgctct ctgaatcaaa agaggttggg ttctgtaggg attatttttt 54540
cttcattgtt tttctgttta caaaaagaat acatattcat ggcaggagat tttgggaaaa 54600
agaagacata agcccatcat ccagaaatag ccactgctaa cacggtgatt ggtatccttt 54660
gggtcttttt tcaggcacgt gtgcgcatgc atgtataaat ataaatagac atttatttta 54720
ataattcaaa actgacactg ttttgtacat tgttttgtaa tccgtgcttt tcaatgaaga 54780
atttatcaca aatctgttcc tcatcatcaa atctttcttt tttttttttt tgagacggag 54840
tctcgccttg tcgcccaggc tggagtgcag tggcgcaatc tgggctcact gcaagctccg 54900
cctcctgggt tcacaccatt ctcctgcctc agcctccgaa gtagctggga ctacaggcgt 54960
gtgccaccac acccggctaa ttcttttttt tttttatttt tagtagagac ggggtttcac 55020
catgttagcc aggatggtct tgatctcctg acctcgtgat ccacctgcct cggcctccca 55080
aagtgctgag attacaggcg tgagcccccg tgcctggcca aatctttcat actatgattt 55140
ttatggacag tagtccataa actaggaaac gccatgattt atttaaccaa ggcctactgt 55200
tgggcatgat cgactcttgc actttgaaca tatcacccca tgctttcctg ctaagaagtc 55260
cacttttaaa aatgccttgt aatttttaaa aattcctcta tttaacattc tgctattgag 55320
tttaaggaaa tacagtttcc tcatgatggg ggatcccttg aatgagatga gttgcttttc 55380
tcttgctgct ttgaacgttc tctctgtctt tgataatctg attataacgt gtgtcgtgta 55440
gaacacttgc agctccacct tttcagtgtc ctttgggttc catgaatctg gatgtctccc 55500
tccttggaac tgggaagttt tcagccatta ttctggccgt catttactga ttataaagtg 55560
cttattatca attctatttg gacagagcta acttgtagtt ggtgtgggga gtgcaaactt 55620
tgcaaagaat ttggttcttt tctggtggtc ttagcctgag gatgtcaagt gtgagcctag 55680
agggtgacgt ttcctctcct ggctccttac cacctgccgt gaagatgatc tactctggcc 55740
tttctctgtg gaaaatggct gcaaaataat gaaacaggct gtcacggaat tttctcctcc 55800
tctttctcca ggggtgttga aatagtcact tcctacagcg atgcggaaac atcttgggct 55860

ttggggtcac acttcccctg agttcagagc cttcatagat gtgtggcagc cttcttagct 55920

gagtgacctt gggcaagtta ctcttagtct cttcgtgctt gactttcctc gtctataaga 55980

cggggtgatg atcccgacct tgccagtggt agaaagcaaa gcagccgcgg gcctcatgca 56040

atgtgcatgg tgcctggcag ctggtcggtg ctcagcacac agagctgtga tgggtctcat 56100

gcaatgtgca tggtgcctgg cagctggtcg gtgctcagca cacagagctg tggctgcccc 56160

tggtgccgtt ccagggatgc tgtattttta ggatttgcca gcttacgagc ctctcaagca 56220

tcgtcccttt gaagtcagcc ccgttgtgga tcctcagttg tatcacgtac ctccctcatc 56280

agaattggct cataataatt ttttgtgttt cataaagtca gatcctcaga ggaccgtaat 56340

tgtcaaggtt gggtactcat aaaaaggctg caggctctga cagccttatc agaagccaca 56400

gtctcagaga cactggggac acatgcccgc cactgatgga atagcccgct gaggttgata 56460

ctttgaaggc agcaaccttg gtttggatgt gtagtcttgg ggatttcttt aaaaacataa 56520

agttctttac atcacagcca tacgttaggt tttagttttc atttgctttg ccagagctgt 56580

ccttgtaaaa ataacttctt cccatgtgtg cacagaacta tgttgtgctt ctggactcca 56640

cactccccag atcccagtat gactacatct tgcctcaagt ttctttcacc gcagtgggct 56700

accataaaca catcaccttg atttttaatc ccacggtaag taaaagaggg agttaaaaaa 56760

aaacccatgg gctgggtttg gtggctcacg cctgtaatcc cagcactttg ggaggccaag 56820

gctggtggat ctcttgagcc caggagtttc agaccagcct aggcaatacg gtaaaacccc 56880

aactctacaa aaaatacaaa aattaaccgg gcgtggtgga acgcacctat agtcccggct 56940

actcgggagg ctgaggtggg aggatcactt gagcctggga ggttgaggcc tcgatgagcc 57000

atgatcatgc ccctgcactc cagcttgggt aacagagtga gaccctttct ccagaagaaa 57060

aaaaacaaaa caaaacaaaa ccaaacaatc catggggtgg cgaaagaagg catgtccata 57120

caacctgtgc tccagatcgc aggttgcagg gcagatattt aaagggctcc tggcattggg 57180

tcactttctc attctgcctt ctggattctg ggcttcccca gattgttttt agcttttctg 57240

agcactgatg cctgcgattc cctttgagga ggaaactggt aataagagca gtgtaattgt 57300

ttagatcggt ggctcaggaa tgattttggg ggaggaggga caccactatc gctggtgtgg 57360

cgtgtgcttc tccatgaggg agcacatggt acatggccac gcaaacctct agaaccaact 57420

gctactttac aggaaatagg gaggacccag gagcacgcaa atgactccac agggatgcag 57480

gacaaccacc tggagcctga tttgtttgac aaataaattg caaggaaaaa aaaaccaaca 57540

acagatggac agaatacctg tgggttaaaa gaaacttaac ttatcgctta attcattgca 57600

atatgtggcc cttactattt gcaatatgtg tatctgttgc atacggtctg tcctgtatcc 57660

gataggctgg aacagtggaa caaacaaaac atacacgtat cttttacctt cttgataaaa 57720

ttgtttagtg attcctaaag ctcagatgca gtattgtcac ctgatgatta aacagacaca 57780

tcagcaatag caaaaacaga gtccagggct cagcctcagg actgctgatt ctgaacttga 57840

cggggtgaaa gtgtagcaca ggagtctgca ttaaaaacaa aaatctggcc tcatagctac 57900

tgtgttggtt gttgcttcta ggccttttca gtgggcagaa taagaacttt attgttttca 57960

aaaaatgtaa ctgtaagtgg aacaatatcc tccgatcctc cttttccttt tttgataac 58020

gacacagcat tcctctgtgt ggatataccg tgtgtcttag gccatttggg ctgctatttt 58080

gtatcttaga ctgggtaact tataaacaac aaacatttat ttcttacagt actagaggct 58140

gggaagtcca agatcaaggc attggcagat ttggggtctg gcgagggctt gctgtctgct 58200

tcatagatgg tgccacctag ctgtgtcccc acaaggcgga aaggggcaaa caagttccct 58260

caggccactc ttacggggca ttaatccctg acttaatcac ctcctaaaaa gggtgtcctc 58320

ttgacactat cacattatgg attagggttc aacctatgaa ttacgtaggc acagacacat 58380

tcagatcctg gcactacatg taggatattc agttagcccc tggttgaggg gcgtttgtgt 58440

tgttactggt tttctgctat tacacataac cctttaatga attgccttac gcatgcatgt 58500

ttttgagtgt tcgtcagtct ctctttggaa tagactccca gaagtggaat tgctgagtca 58560

aagggtaaat gcatacggaa ttttgattga tacggccact tcccatccat gggggtttta 58620

ctgttttata ttcccgccag cagtgaatga gtaccctttt ttccccaaca gagtattttg 58680

tcaaattttt caatttttgc aggcttatag atgagaagtg atattatctc agtgtacttc 58740

taattgcatt tctctctttt catgtggtta agagccattt gtattttctg tgaactattt 58800

aacctatttt tctatagaat ttttggtctt tttcaaccat tttcagctct ttgtatacta 58860

ggaatattaa ccctttgtaa tgtttgttgt acatattttt cccaatttgt catttatctt 58920

ttcacttggt ttctgcaaag atttacttta tttctatgtt aaatgtgttg atttttttct 58980

tattgcttct ggattttaga gtcataggaa ggttttcttc attcccagct tttacaggaa 59040

tttattgtgt ttttttcagg cagactttta tagtttctgt ttttacattt aaatctctct 59100

atccatttgg agtttttcct ggtatagatg tgaagtatgg ctccagattg tctttttcca 59160

catgctatcc agttaccatt tcatgctttt caaacgccat cctgcatgga aggtggacag 59220

gaattacctt tcatttagag atgagaagat gggaacctag aaggagatgt gagcagccca 59280

aggtcaccag ctcacgtgga gcagtgctag ggcttgagca gagctctgct gctgattccg 59340

ggcttctgtg aagagtgatt catgctcagt gagcctgtaa atatgatctt acctgaacaa 59400

gagagttttt ctcatgaccc acgaggaaac acttccttgt tactcacagt agagacctgg 59460

ttagaaagtg ccggagaggg ctaggtgtgg tggctcacgc ctgtaatccc agcattttag 59520

gaggctgagg tgggcagatc atttgaggtc aggagttcga gaccagcctg ggcaatgtgg 59580

tgaaagtgaa accccatctc tgctaacaat acaaacatta gccaggcgtg gtagcacgca 59640

cctgtaatcc cagctactcg agaggctgtg gcaggagaat tgtgaacctg ggatgcagag 59700

tttgtagtga gctaagatag ccccagtgca ctccagcctg ggcgacagag caagactgtt 59760

tcaaaaaaaa aaaagaaagt gccagaaaga taagcatata caagaaccac ttacctttgc 59820

ttggggctgc actgtggccc cagttgctgg gagaggcttc tgtgaaggcc gttgatagct 59880

gcaggcagtg acattctcag gggcccctag cagacaactc atccgggctc agggacacac 59940

ctgggagcca gtggcaggaa tgtggagaca ctgcttggtg gtggccaagc agcccagcct 60000

cgtagctgct gcctgtcact tctgagaatg taagaaactg tgccctggtg tgtgatgttc 60060

cccttcctgt atacctatgt aactaacctg cacattgtgc acatatatcc taaaacttaa 60120

agtataatta aaaaaaaaaa aaaaaaaagc tatgcctgct ggggcttgga ttcccgagca 60180

gggccaagtg ttgaatgaag agagcgccca ctctgtgcca ccccgtacag ggccctcaca 60240

ggttgatcct cacaacaagc cttcgagcag gtgtgccatt cctcccattt tcacagaagc 60300

ccagaaagat tgagtcactt actgagtgtt agcagaacca ggactcaagt gaggcggttt 60360

gtctcaagcc acatggtttc cacctcgttc tgccctccgc gtgatgagtg gctcagcttt 60420

gcacgttcac gggggctttg taagcacatt ggtttccttc tttgttgatg tgactgccca 60480

cagcctcgcg actggatttt actcagtatt ccatgccctg ctgagcattt cacatactcc 60540

tttcatcaag tttcacagca tccaccccca gtgggtgttg ttcatatgat ctccatgtca 60600

tagttgagta aactgagtct caacgtggtt gcctgagttt acccagtgag gcctgggacc 60660

tgagcctaga ctgaatcact ctaattcctg ctgaattaaa cttttattta tttattcatt 60720

attatttttt tctgagacag agtcttgctt tgttgcccct gctggagtgc agtggcacga 60780

tcttggccca ctgcaacctc tgcttcccgg gttcaagtga ttctcacctc ctgagtagct 60840

ggaattacag gcatgcacca ccacttctgg ctaattttgt atttttatag agacggggtt 60900

tcaccatgct ggccaggctg ttctcaaact cctgacctca ggtgatctgc ccacctcagc 60960

ctcccaaagt gctgggatta caggcgtgag ccactgcacc tggcctgaat taaactttta 61020

agtaagtgtt tacttcagtg gaaaaatggg ttgggttcta agagatgtcc aaagatacag 61080

gagtaggcag ttgttgatgg aaatctgttc ctgcccttat tctttcattt atgaaagggc 61140

atcccaggta cagcggcgcc cacagtcact ctctagccca gcagttctcg gatgggttgg 61200

ttgcaggacc cctttatact gttaaaaatt attgaggatt ttaaagagtt tgagtttgca 61260

tggattatct ctgtgtatat ttatcatatt agtaattaaa accgagatgt tgaaaacgca 61320

agaactcaca agctacacat tccgttagct gtgggcgtga tgacatcagg gtgcgtagtc 61380

tctggaaaag tccactgtgc gcttgcgaat gaaagagggc ggaaaaggca catcacatct 61440

tactattggc tgaaaatagc tgtggcctcg ggaacctctg cgagaggagt cccggtatcc 61500

tacactgaga acactgctct ggctcattaa cttgttttta tcattattgg gaagttctcc 61560

ttccatttat ttgcttcctt tatgagagta gagtgggcca atgggatata ggttcacttg 61620

atgctttaga acccagcatt tcaggggctt tagcaagatg gaaatgtctg tctcatgtca 61680

agtgtccagg agtgaccagt gcagagatag tctggtagct ccaaggcgtc aggggaccca 61740

ggccccttct gtctcattgc gctgttccag tgcattgctt ttctccaaaa tggggcaatt 61800

ggccaaatgc tgtctgccca gcaaaaggga gggcacacca cttccctagg gtccagccag 61860

ggtgtggcac ttaccgcttc tgttcctgta ccattggcca aaatttagtt ccagggctgc 61920

gcctgctgca ggggaggctg gaaaaggtga tctatattgt cggtggccca gataaaagtt 61980

gggggttcta ttgggagaaa gggagaatgg ccacagggat gctagtagca gtgcctgctt 62040

tgcttcctca ctagaccgta ctggctggga atagctgccc atcactgtgt gccctgggtc 62100

tagaactgta ggttcttgat acatccttgt aggactaagt ccctgcatgt tgctgggact 62160

caacaaactg agacccagtt gcttttcaac caccaagact ctctgatagc tgcatctctt 62220

catttcctca gttgggagga gggtccagag gctgaggaat gccttgtcat tggaatctct 62280

ttgtccctga gtttcagaag caaatagtga agctttgggc agaagaggat tcatgggaaa 62340

atatatccaa atcactttgg ttccttcctt tggatagaag gaatttggat catgagggcc 62400

cctgggtgtc ctcaagtggc cactttggaa ggctgggtgg gtggtggctg tggcattgtg 62460

gatgatggac aatcgtgtgg ccttggtgag gagtgatggg gctctcggtg tttgcagagg 62520

aagctgcctg aacaggacat cgcacaagga tcctacattg ccctgccatt gacgctgctg 62580

gttctgctgg ccggttacaa ccatgacaag gtaggaaatc cagaggcctc aggagatggc 62640

gggcatgtca gggagagcga gtcctcacag ctgcaggagt gatagcagag ggatgtccag 62700

ggtcacagaa atcacatttc cgggtcggga gggcccgtga aggcctcaag tccagactta 62760

ggtctccttt caaggctgta aacttctgta acatctccta atccttaaac ctggaacacc 62820

tctagggaca tattaggccc ggagagaggc cagcccatcc ctggggactc attagtaaga 62880

ggcctgcctc gtttgaactg aaacccaccc attagcacgt ggcagctgct gatcatgtct 62940

gaagaaggtg tgttgagaag tgaggctccg ctgttcatct ggcaaaagcc tcctctttgt 63000

acccctctct ctcacacttt ctctcttttc tagctcattc ctttgctgct gcagttgaca 63060

agccggctac agggagtccg cgcgctcggc caggcagcct ctgacaatag cggcccagaa 63120

gatgcaaaga gacaagccaa gaaacagaag acaaggcgga cttgaggagg aaggggacag 63180

ttgcagtctc acttgggaca ggccacagcc aggggtccgg ccactacccg cccgtgggat 63240

aaaagccaaa agcatgcgtc agctaacttc agcctgtgct gctgggcccg caccccatgt 63300

cccttgtcac tgtggcatcc tgcacccatc ctcacccctc cgtagagccc ctcgtgcaat 63360

gcaatgaatg gaccctcctg tcactctgct gaacagaatt tattttctga gtcaaatata 63420

atttattatt atttttgtca aagaagtatt taagctgtgc tgtggtgtga gaatgtcatt 63480

cttgatcttc agccttcgtt tgcaaggaga gttccagttg acgtggtgtt tggttccatg 63540

gcggggtacc ctagggattc atctgttttc ttcacttccc tttgcatctg agatcctgct 63600

ggaaaccaca gcaacctgta tccactatta ggaggtaaaa atcaataaaa tggcccattc 63660

atttgtgttg tagctcatca tagatgtatt tcttggatga catgcgcgta acccccgggg 63720

gtcttcagtt gagccaaatg tagagcaagt cagagtcctg agtgaagctg gctggggcag 63780

gaaaaacacg agctcagccg acatggtccc cagcagcttt tggcgtcagt gatagagaaa 63840

tcggagatag tggaggttgt ggcaagttga agtgtgccca ccacgtctaa gggaagctta 63900

gggactcagc tctggctcac ccctgctgtg cagaaatgtg cgttcatttt tctagagaag 63960

ccagaaaaac aaagatttac gtgaaatttt cccaatttta aatgttagcc acaaatgcag 64020

tgttttttta agaaaaccac acactttggc caaacaaaat gcctacgtcg gccgggtgag 64080

gcttaaaggt tgccggcttg catcagagat ccagaggaag tcacagcatt ttaacagcta 64140

aacttaatcc tcacaggaag tcatcaagtg aattaagtga tattgaagga ggggacacat 64200

ccatccatcc gacatttatt gagtgccaca taggacaggc atgggtatgt gaggatgagt 64260

agggtagaca acactctaca gcgagctgga agaggccagg gaggccccgt ctgccttgtt 64320

cttcgtaccc caatgcttgg cacttggtgg gcattcagta aggaatgaat gaacgcacgc 64380

acagaggaat gaatagatgg gcacacagtg tgtgccctca cagcacatgg cccatcactg 64440

ggtagaaggc aagtcagtaa gtagacaggg gtaatgcagg gagccgaggg cctccaggtg 64500

gaggaacatg gaggaccaca ggagcacaga gcagagttct catcccactg gagagcttag 64560

ggaactttct ggatgtgatg cgtgagcaga gtccaaaagg acaaaacgga gagagggaaa 64620

acaaggtgga agttcttgtt aggtgccact gccaccccag ggggtcagct tgggggactc 64680

cctggctctt gggggccaac cgcagagctg ccctgttccc acctatcagc tagcttggtg 64740

ttggcaccgt ggaaggaaag tgaacagtgt tggagatctg ggacagatgt gatattaata 64800

cagaatccat ttcttggagt gttttccaac tcttattcca agtggacacc cagaaacatc 64860

ccttttaaat gttaatgggg 64880

<210> 2
<211> 1222
<212> PRT
<213> Homo sapiens

<400> 2

Met Leu Val Gly Gln Gly Ala Gly Pro Leu Gly Pro Ala Val Val Thr
1               5                   10                  15

Ala Ala Val Val Leu Leu Leu Ser Gly Val Gly Pro Ala His Gly Ser

            20                  25                  30

Glu Asp Ile Val Val Gly Cys Gly Gly Phe Val Lys Ser Asp Val Glu
        35                  40                  45

Ile Asn Tyr Ser Leu Ile Glu Ile Lys Leu Tyr Thr Lys His Gly Thr
    50                  55                  60

Leu Lys Tyr Gln Thr Asp Cys Ala Pro Asn Asn Gly Tyr Phe Met Ile
65                  70                  75                  80

Pro Leu Tyr Asp Lys Gly Asp Phe Ile Leu Lys Ile Glu Pro Pro Leu
                85                  90                  95

Gly Trp Ser Phe Glu Pro Thr Thr Val Glu Leu His Val Asp Gly Val
            100                 105                 110

Ser Asp Ile Cys Thr Lys Gly Gly Asp Ile Asn Phe Val Phe Thr Gly
        115                 120                 125

Phe Ser Val Asn Gly Lys Val Leu Ser Lys Gly Gln Pro Leu Gly Pro
    130                 135                 140

Ala Gly Val Gln Val Ser Leu Arg Asn Thr Gly Thr Glu Ala Lys Ile
145                 150                 155                 160

Gln Ser Thr Val Thr Gln Pro Gly Gly Lys Phe Ala Phe Phe Lys Val
                165                 170                 175

Leu Pro Gly Asp Tyr Glu Ile Leu Ala Thr His Pro Thr Trp Ala Leu
            180                 185                 190

Lys Glu Ala Ser Thr Thr Val Arg Val Thr Asn Ser Asn Ala Asn Ala
        195                 200                 205

Ala Ser Pro Leu Ile Val Ala Gly Tyr Asn Val Ser Gly Ser Val Arg
    210                 215                 220

Ser Asp Gly Glu Pro Met Lys Gly Val Lys Phe Leu Leu Phe Ser Ser
225                 230                 235                 240

Leu Val Thr Lys Glu Asp Val Leu Gly Cys Asn Val Ser Pro Val Pro
                245                 250                 255

Gly Phe Gln Pro Gln Asp Glu Ser Leu Val Tyr Leu Cys Tyr Thr Val
            260                 265                 270

Ser Arg Glu Asp Gly Ser Phe Ser Phe Tyr Ser Leu Pro Ser Gly Gly
        275                 280                 285

Tyr Thr Val Ile Pro Phe Tyr Arg Gly Glu Arg Ile Thr Phe Asp Val
    290                 295                 300

Ala Pro Ser Arg Leu Asp Phe Thr Val Glu His Asp Ser Leu Lys Ile
305                 310                 315                 320

Glu Pro Val Phe His Val Met Gly Phe Ser Val Thr Gly Arg Val Leu
                325                 330                 335

Asn Gly Pro Glu Gly Asp Gly Val Pro Glu Ala Val Val Thr Leu Asn
            340                 345                 350

Asn Gln Ile Lys Val Lys Thr Lys Ala Asp Gly Ser Phe Arg Leu Glu
        355                 360                 365

Asn Ile Thr Thr Gly Thr Tyr Thr Ile His Ala Gln Lys Glu His Leu
    370                 375                 380

Tyr Phe Glu Thr Val Thr Ile Lys Ile Ala Pro Asn Thr Pro Gln Leu
385                 390                 395                 400

Ala Asp Ile Ile Ala Thr Gly Phe Ser Val Cys Gly Gln Ile Ser Ile
                405                 410                 415

Ile Arg Phe Pro Asp Thr Val Lys Gln Met Asn Lys Tyr Lys Val Val
            420                 425                 430

Leu Ser Ser Gln Asp Lys Asp Lys Ser Leu Val Thr Val Glu Thr Asp
        435                 440                 445

Ala His Gly Ser Phe Cys Phe Lys Ala Lys Pro Gly Thr Tyr Lys Val
    450                 455                 460

Gln Val Met Val Pro Glu Ala Glu Thr Arg Ala Gly Leu Thr Leu Lys
465                 470                 475                 480

Pro Gln Thr Phe Pro Leu Thr Val Thr Asn Arg Pro Met Met Asp Val
                485                 490                 495

Ala Phe Val Gln Phe Leu Ala Ser Val Ser Gly Lys Val Ser Cys Leu
            500                 505                 510

Asp Thr Cys Gly Asp Leu Leu Val Thr Leu Gln Ser Leu Ser Arg Gln
        515                 520                 525

Gly Glu Lys Arg Ser Leu Gln Leu Ser Gly Lys Val Asn Ala Met Thr
    530                 535                 540

Phe Thr Phe Asp Asn Val Leu Pro Gly Lys Tyr Lys Ile Ser Ile Met
545                 550                 555                 560

His Glu Asp Trp Cys Trp Lys Asn Lys Ser Leu Glu Val Glu Val Leu
                565                 570                 575

Glu Asp Asp Met Ser Ala Val Glu Phe Arg Gln Thr Gly Tyr Met Leu
            580                 585                 590

Arg Cys Ser Leu Ser His Ala Ile Thr Leu Glu Phe Tyr Gln Asp Gly
        595                 600                 605

Asn Gly Arg Glu Asn Val Gly Ile Tyr Asn Leu Ser Lys Gly Val Asn
    610                 615                 620

Arg Phe Cys Leu Ser Lys Pro Gly Val Tyr Lys Val Thr Pro Arg Ser
625                 630                 635                 640

Cys His Arg Phe Glu Gln Ala Phe Tyr Thr Tyr Asp Thr Ser Ser Pro
                645                 650                 655

Ser Ile Leu Thr Leu Thr Ala Ile Arg His His Val Leu Gly Thr Ile
            660                 665                 670

Thr Thr Asp Lys Met Met Asp Val Thr Val Thr Ile Lys Ser Ser Ile
        675                 680                 685

Asp Ser Glu Pro Ala Leu Val Leu Gly Pro Leu Lys Ser Val Gln Glu
    690                 695                 700

Leu Arg Arg Glu Gln Gln Leu Ala Glu Ile Glu Ala Arg Arg Gln Glu
705                 710                 715                 720

Arg Glu Lys Asn Gly Asn Glu Glu Gly Glu Glu Arg Met Thr Lys Pro
                725                 730                 735

Pro Val Gln Glu Met Val Asp Glu Leu Gln Gly Pro Phe Ser Tyr Asp
            740                 745                 750

Phe Ser Tyr Trp Ala Arg Ser Gly Glu Lys Ile Thr Val Thr Pro Ser
        755                 760                 765

Ser Lys Glu Leu Leu Phe Tyr Pro Pro Ser Met Glu Ala Val Val Ser
    770                 775                 780

Gly Glu Ser Cys Pro Gly Lys Leu Ile Glu Ile His Gly Lys Ala Gly
785                 790                 795                 800

Leu Phe Leu Glu Gly Gln Ile His Pro Glu Leu Glu Gly Val Glu Ile
                805                 810                 815

Val Ile Ser Glu Lys Gly Ala Ser Ser Pro Leu Ile Thr Val Phe Thr
            820                 825                 830

Asp Asp Lys Gly Ala Tyr Ser Val Gly Pro Leu His Ser Asp Leu Glu
        835                 840                 845

Tyr Thr Val Thr Ser Gln Lys Glu Gly Tyr Val Leu Thr Ala Val Glu
    850                 855                 860

Gly Thr Ile Gly Asp Phe Lys Ala Tyr Ala Leu Ala Gly Val Ser Phe
865                 870                 875                 880

Glu Ile Lys Ala Glu Asp Asp Gln Pro Leu Pro Gly Val Leu Leu Ser
                885                 890                 895

Leu Ser Gly Gly Leu Phe Arg Ser Asn Leu Leu Thr Gln Asp Asn Gly
            900                 905                 910

Ile Leu Thr Phe Ser Asn Leu Ser Pro Gly Gln Tyr Tyr Phe Lys Pro
        915                 920                 925

Met Met Lys Glu Phe Arg Phe Glu Pro Ser Ser Gln Met Ile Glu Val
    930                 935                 940

Gln Glu Gly Gln Asn Leu Lys Ile Thr Ile Thr Gly Tyr Arg Thr Ala
945                 950                 955                 960

Tyr Ser Cys Tyr Gly Thr Val Ser Ser Leu Asn Gly Glu Pro Glu Gln
                965                 970                 975

Gly Val Ala Met Glu Ala Val Gly Gln Asn Asp Cys Ser Ile Tyr Gly
            980                 985                 990

Glu Asp Thr Val Thr Asp Glu Glu  Gly Lys Phe Arg Leu  Arg Gly Leu
        995                 1000                 1005

Leu Pro  Gly Cys Val Tyr His  Val Gln Leu Lys Ala  Glu Gly Asn
    1010                 1015                 1020

Asp His  Ile Glu Arg Ala Leu  Pro His His Arg Val  Ile Glu Val

1025                    1030                    1035

Gly Asn Asn Asp Ile Asp Asp Val Asn Ile Ile Val Phe Arg Gln
    1040                    1045                    1050

Ile Asn Gln Phe Asp Leu Ser Gly Asn Val Ile Thr Ser Ser Glu
    1055                    1060                    1065

Tyr Leu Pro Thr Leu Trp Val Lys Leu Tyr Lys Ser Glu Asn Leu
    1070                    1075                    1080

Asp Asn Pro Ile Gln Thr Val Ser Leu Gly Gln Ser Leu Phe Phe
    1085                    1090                    1095

His Phe Pro Pro Leu Leu Arg Asp Gly Glu Asn Tyr Val Val Leu
    1100                    1105                    1110

Leu Asp Ser Thr Leu Pro Arg Ser Gln Tyr Asp Tyr Ile Leu Pro
    1115                    1120                    1125

Gln Val Ser Phe Thr Ala Val Gly Tyr His Lys His Ile Thr Leu
    1130                    1135                    1140

Ile Phe Asn Pro Thr Arg Lys Leu Pro Glu Gln Asp Ile Ala Gln
    1145                    1150                    1155

Gly Ser Tyr Ile Ala Leu Pro Leu Thr Leu Leu Val Leu Leu Ala
    1160                    1165                    1170

Gly Tyr Asn His Asp Lys Leu Ile Pro Leu Leu Leu Gln Leu Thr
    1175                    1180                    1185

Ser Arg Leu Gln Gly Val Arg Ala Leu Gly Gln Ala Ala Ser Asp
    1190                    1195                    1200

Asn Ser Gly Pro Glu Asp Ala Lys Arg Gln Ala Lys Lys Gln Lys
    1205                    1210                    1215

Thr Arg Arg Thr
    1220

<210>  3
<211>  4261
<212>  DNA
<213>  Homo sapiens

<400>  3
gcggcgtgca gtgtgagggg cgggacccgg ctgccggcgg tgggtctagc tgggggaggt        60

cgggccatgc tggtgggcca gggcgcgggg ccgctggggc ccgcggtggt caccgccgcg 120

gtggtgctgc tgctgagcgg cgtggggccg gcgcacggct cggaggacat cgtggtgggc 180

tgcggtggct tcgtcaagtc ggacgtggag atcaactact ctctcatcga gataaagctg 240

tacaccaagc atgggacttt gaaataccag acagactgtg cccctaataa tggttacttt 300

atgatccctt tgtatgataa gggggatttc attctgaaga ttgagcctcc cctagggtgg 360

agttttgagc cgacgaccgt ggagctccat gtggatggag tcagtgacat ctgcacaaag 420

ggtggggaca tcaactttgt cttcactggg ttctctgtga atggcaaggt cctcagcaaa 480

gggcagcccc tgggtcctgc gggagttcag gtgtctctga gaaacactgg gaccgaagca 540

aagatccagt ccacagttac acagcctggc ggaaagtttg cattttttaa agttctgcct 600

ggagattatg aaatcctcgc aactcatcca acctgggcgt tgaaagaggc aagcaccaca 660

gtgcgtgtaa ccaactccaa tgccaatgcg gccagtcccc tcatagttgc tggctacaat 720

gtgtctggct ctgtccgaag tgatggggag cccatgaaag gcgtgaagtt tcttctcttt 780

tcttctttag taactaaaga ggatgtcctg ggctgcaatg tctcaccagt gcctgggttc 840

cagccccaag acgagagtct ggtgtatttg tgctacacgg tctccagaga agatggctcg 900

ttctctttct attccttgcc aagtgggggc tacactgtga ttccgttcta tcgaggggag 960

aggattacct ttgatgtggc gccttccaga cttgacttca cagtggagca tgacagcttg 1020

aaaatcgagc ccgtgttcca cgtcatggga ttctccgtca ccgggagggt cttgaacgga 1080

cccgaaggag atggtgttcc agaagcagta gtcaccctga ataaccaaat caaagttaaa 1140

acaaaagctg atggctcatt ccgccttgag aacataacca cagggacata caccatccat 1200

gctcagaaag agcacctcta ctttgaaacg gtcaccatca aaattgcacc gaacacacct 1260

cagctggctg acattattgc aacagggttc agtgtctgtg gtcagatatc aatcattcgc 1320

ttccccgaca ccgtcaagca gatgaataaa tacaaagttg tcctgtcatc tcaagacaag 1380

gacaagtctt tggtcaccgt ggagacagat gctcatggat cattttgttt taaagcaaaa 1440

ccagggactt acaaagtgca ggtgatggtt cctgaggcag aaaccagagc agggctgacg 1500

ttgaaacccc agacatttcc tcttactgtg accaacaggc ccatgatgga tgtggccttt 1560

gtacagttct tggcatcagt ttctgggaaa gtctcttgtt tggacacctg tggtgacttg 1620

ctggtgactc tacagtccct gagccgccag ggtgagaagc ggagcctcca gctctccggc 1680

aaggtcaacg ccatgacttt cacctttgac aacgtgctcc ctggaaaata caaaataagc 1740

atcatgcatg aggattggtg ctggaagaac aagagcctgg aggtggaagt gctggaggat 1800

gacatgtctg cagttgagtt caggcagacg ggctacatgc tgagatgttc cctgtctcac 1860

gccatcactc tggaatttta tcaggatgga aatgggcgtg agaatgtggg gatttataac 1920

ctctccaaag gagtcaaccg attctgcctg tccaagcctg gtgtgtacaa agtgacccct 1980

cgctcctgcc accggtttga gcaagcgttc tacacctatg acacgtcttc acctagtatc 2040

ttgacattga cagccattcg ccaccatgtc cttggaacta tcaccaccga caaaatgatg 2100

gatgtcactg tgactatcaa gtcttccatc gacagtgaac ccgccttggt cttaggccct 2160

ctgaagtctg tgcaggagct gcggagggag cagcagctgg ctgagatcga ggcccgcagg 2220

caggagaggg agaaaaacgg caatgaggaa ggcgaagaaa gaatgaccaa gcctcccgtg 2280

caggagatgg tagatgagtt acaaggcccc ttctcgtatg atttctctta ctgggcgcgg 2340

tctggagaga aaatcactgt tacaccgtca tctaaagagc tgctctttta tcccccttca 2400

atggaagccg ttgtcagtgg agaaagctgc ccagggaagc tgatcgagat ccatgggaag 2460

gcaggcctgt ttttagaagg ccagatccac cccgagttgg aaggagtcga gattgtcatc 2520

agtgaaaagg gggcaagttc accgctgatc acagtcttta ctgatgacaa aggtgcctac 2580

agtgttggcc ccctgcacag tgacctggag tacacggtga cctcacagaa ggagggctat 2640

gttctgactg cggtggaagg aaccatcgga gacttcaagg cctatgccct ggcaggcgta 2700

agctttgaga taaaagctga ggatgaccag cccctcccgg gagtcctctt atccctgagc 2760

ggtggcctgt ttcgttccaa cctcttgacc caggacaacg gcattctgac attctcaaac 2820

ctgagccctg gccagtatta cttcaaaccc atgatgaagg agttccggtt tgagccatcc 2880

tcacagatga tcgaggtgca ggaaggccag aacctgaaga tcaccatcac ggggtaccga 2940

accgcttaca gttgctatgg cacagtgtct tccttaaacg gagagcccga acaaggggtt 3000

gccatggaag cggtgggcca gaacgactgc agcatttacg gagaagacac cgtgacagac 3060

gaagagggca agttcagatt acgtggattg ctgccgggat gtgtgtacca cgttcagctc 3120

aaggcagaag gcaacgacca cattgagcgg gcgctccccc accatagggt gattgaggtt 3180

gggaataatg acatcgatga tgtaaacatc atagttttcc ggcagattaa tcaatttgat 3240

ttaagtggaa atgtgatcac ttcctctgaa taccttccta cattatgggt caagctttac 3300

aaaagcgaaa acctcgacaa tccaatccag acagtttccc ttggccagtc cctgttcttc 3360

catttccccc cactgctcag agacggcgag aactatgttg tgcttctgga ctccacactc 3420

cccagatccc agtatgacta catcttgcct caagtttctt tcaccgcagt gggctaccat 3480

aaacacatca ccttgatttt taatcccacg aggaagctgc ctgaacagga catcgcacaa 3540

ggatcctaca ttgccctgcc attgacgctg ctggttctgc tggccggtta caaccatgac 3600

aagctcattc ctttgctgct gcagttgaca agccggctac agggagtccg cgcgctcggc 3660

caggcagcct ctgacaatag cggcccagaa gatgcaaaga gacaagccaa gaaacagaag 3720

acaaggcgga cttgaggagg aaggggacag ttgcagtctc acttgggaca ggccacagcc 3780

aggggtccgg ccactacccg cccgtgggat aaaagccaaa agcatgcgtc agctaacttc 3840

agcctgtgct gctgggcccg caccccatgt cccttgtcac tgtggcatcc tgcacccatc 3900

ctcacccctc cgtagagccc ctcgtgcaat gcaatgaatg gaccctcctg tcactctgct 3960

gaacagaatt tattttctga gtcaaatata atttattatt atttttgtca aagaagtatt 4020

taagctgtgc tgtggtgtga gaatgtcatt cttgatcttc agccttcgtt tgcaaggaga 4080

gttccagttg acgtggtgtt tggttccatg gcggggtacc ctagggattc atctgttttc 4140

ttcacttccc tttgcatctg agatcctgct ggaaaccaca gcaacctgta tccactatta 4200

ggaggtaaaa atcaataaaa tggcccattc atttgtgttg tagctcaaaa aaaaaaaaaa 4260

a 4261

<210> 4
<211> 20
<212> DNA
<213> Artificial Sequence

<220>
<223> Forward primer of the NOMO-1 gene

<400> 4
agctccatgt ggatggagtc 20

<210> 5
<211> 20
<212> DNA
<213> Artificial Sequence

<220>
<223> Reverse primer of the NOMO-1 gene

<400> 5
acggatgaag tacagagttc 20

<210> 6
<211> 34600
<212> DNA
<213> Oryctolagus cuniculus

<400> 6
   ggtttcttcc tagggtctgc attttcagga aatctagagt cagaagctag aaatgggtat 60
   caaaaccgtg ctactctgat gtgggatgta ggctttctca cctgatatct taactactag 120
   ctgaaatgcg tgcccctaaa cttatctttc cattccattt tcttacgaga tgtttgaagc 180
   accttgtaca tgtaaaacac gcttttaccc tttcttgtgg agtgattagc ttttgaagct 240
   aatgttttca caagtgtgta tgcccactgg taacttaaaa aagcaatgtc ttctgttaac 300
   cttcggagag aatcagtcgc tccatcttac aattagagtc ttctattttc tttatatttt 360
   accttagaaa agtagcatat ttttaaagat gatgatcaca cgttttgttt atttgaattc 420
   caaaagctca tcatgcaagg taagtgaaaa acagtgccac agttagattt ctttttagtt 480
   ttttaagatt tgcttattta ggctggcgct gcggctcaat aggctaatcc tccaccttct 540
   gcgccatcac accgggttct agtcccggtc ggggcgccgg attctgtccc ggttgcccct 600
   cttccaggcc agctttctgc tgtggccagg gagtgcagtg gaggatagcc caagtgcttg 660
   ggccctgcac cccatgggag accaggagaa gcacctggct cctgcctttg gatcagtgcg 720
   gtgcgctggc cgtggcggcc attggagagt gaaccaacag caaaaaggaa gacctttctc 780
   tctgtctctc tctcactgac cactctgcct gtcaaaaaat aaataaataa aaaaattaaa 840
   aaaagatttt tttgtttatt tgaaaagcaa agttactggg ggcggggggg agggagagag 900
   agatcttcca tccactgatt cactccccaa gtggctgcat gggcccgacc tgagccaggc 960
   cgaagacagg agccttgagc ttcctatggg tctcccaggt ggttgcaggg gcccaagtgc 1020
   ttggatcatc tatacggctt tcccaggtgt gttagcagga ggctagattg gaagtggagc 1080
   agctggatct cttaactggc acccatatgg gatgccagtg tcacaggtaa tggctttacc 1140
   tgctatgcca caatgccgat ccccctcaaa tttcttaaaa agcacaaaca catacagggc 1200
   aggattgtgg ttaagccacc acttggaatg gccccatccc atattggcct ctcgcctgaa 1260
   cactgcagag tggcggagtg gtccctgcaa acacggcagg atctggcatc tggaactaga 1320
   gataggggcg gtccacctgt atagtcatct cagtgtttat gttgggagac gttctacaca 1380
   gttcaaaatc tgccttccaa accgcctttt ggtatgtaca tatatgtaca gtagggactt 1440
   cctgtgtggc atttctgggt gttacagctg ggcgttgctg tggccgtctg tcctggctct 1500
   gctggattgg attcccagag ctgcagagcg tctcctgtga tgaaatccga atcagggacg 1560
   aggtggctcc tgttcgctca ccgccattag cccgatgcag ccatttggga agccagtcca 1620
   gccaagccca gccaaaccaa gcaggaaccc tggagccttc tttgctgtgt gatccccagg 1680
   gcactccctc ctctagtcag ctccttgggc ttctgtttcc cccgtttctg agactgcagg 1740
   gacttcagtg tgcccaggaa gcttagtgtc tgcctgagtg gaggccccag ctctggggtt 1800
   catgagcatc ctgcagactg actcctgggt cttattcagt ctcccctgca ccttggtgtc 1860
   tctacatccg agctctgcca gccagtctga gagcccagag gtttccccaa tatgcctttt 1920
   gcttcaggaa agtctgttgt ctgttttccc tccttttttt tttttttttt ttcttttttt 1980
   cccctctgtt gtggttttcc agcatgactg cttcctctct gtcccggaaa aggcagattt 2040
   cccagtgcag agagttgaaa gatctgggct gccagagaag ttatctcact gtcttcaggc 2100
   tggaggtatc cttgctttat agatcagcta ctcagtccca ccaaggcttc aggtcaagtc 2160
   aggccgggcc ttctgcccag agtgaacaca gggagccagc ctgggaagct gggggactga 2220
   gacgagacgt ggagctttgg gtatgaagtg ggccctcctt atttaggagg ggccagctgt 2280
   ctgtgttgac agctgccaag ggcgccagct tttactggag ctctgaagtg agcagatggc 2340
   tttcagaatt tgtgagggtg gcaagaccta gggccggatg gtgtgggtgc ttctaggaag 2400
   cgaaagcatc attgagagtc ccctggggga gcgttctggg ggaggctcac ctggcccatc 2460
   ctcccttcct cttctctgag ctcctatcta agacagcagt tccactgctg ctgaccctca 2520
   gatggccccg aagggttccg gtgttctgtg cacttcccag cttttggggt gcacattgga 2580
   acgtttgtga atggagttaa tggtcataag ggcaacaact tgattcaggt tttttttttg 2640
   acaggcagag ttagaaagtg agagagagac agaaagaaag gtcttccttc cattggttca 2700
   cccccaaatg gctgctatgg ctggcatgct gcaccgatcc gaagctagga gccaggtgct 2760
   tcctcttggt ctcccatgca ggtgcagggc ccaagcactt gggccatcct ctactgcctt 2820
   cccgggccgc agcagaaagc tgcactggaa gaggagcaat cgggacagaa tccggtgccc 2880
   cgaccgggac tagaacctgg ggtgctggtg ccacaggcag agaattagcc tagtgagcta 2940
   cggcactggc caattcagtt tttttttttt tttttatttt taagatttat ttatcagtca 3000
   gaattacaga gggggagaga gagagatcga gagatcttcc ctctgctggc tcattcccca 3060
   aatgctggga tggctggagt tttggccagg tcaaagccag gagcctcgtc caggtctccc 3120
   actttggtgt agaggtctaa gcacttggcc catcctctgc tgctttccca ggcacattag 3180
   cagggagctg gattggaagt gaagcagctg ggacttgaac cggcgcccat atggaatgct 3240
   ggtgctgcag gtggtggctt aacccgctgt gccacagcgc tggcacctga ttcagttgat 3300
   tttctcaaag taaaagcaac tgcattggcc ctgtccgctc tctataattg ggagccaagc 3360
   actggtgatg ctcatttcac agctgattgc ctgcaagtca ggcacaggct tgtgttccct 3420
   ggcaaactgg ggcactctgt atcctcctgc tcggaaacac ggggcaagtg ggaacagcat 3480
   ttacagaagg agcaggactg cctgcttgtt gttcagtaga ataagtgcct ggtgagatgg 3540
   tggctgtcta ctgaaggaac atgtaggcga cagggttgga gagtctaggc cagccatctc 3600
   tgtctgcttt agttggttgg tttggtacat acagtggagc ctctttcttc tcatggttcc 3660
   tgtctcacct ctctattctt gtggttgctg aggagccact gatctgcctg tgccctgaag 3720
   caaaaaacct aggcccctcc tccctcttgt ccagataagg ggccggcagg gcgctgtggc 3780
   tcaacccgtt aatgccctgg cctgaagcgc cggcatccca tataggcgcc gattcaagac 3840
   caggctgctc tgcttccgat ccagctctct gctgtggcct aggaaggcag tgaagaatgt 3900
   cccaagtcct tggggccctg caacccgcgt gggagacctg gaggaagctc ctggcttcag 3960
   atcattgcag ctccggccat tgcggccgat tggggagtga accattggat ggaagacctc 4020
   tctctgcctc tcatcggtct gtgtaactct gactttcaaa taaataaatc tttaaaagaa 4080
   aaaaaaagaa gacgactgta tgtgggccag tgctgtgatc tgcagtgcca gcatcccata 4140
   taggtaccag ttcaagtccc agctgctctg cttccaatcc agctccctgc taatgcccct 4200
   gggaaagcag cggaggatgg cccaagttct tgggccccct gcatccacgt aggagacctg 4260
   gagaaagctc ttggctcctg gctgctggcc tcagactggc ccaactccgg ccgttgcagc 4320
   tttttaggga gtgaaccagc aaatggacga tctctctgtc tgtctcagcc tctctaactc 4380
   tgactttcaa ataactaaat aaacctttaa gaaaaaaaaa ctgcaccagt gaggtcaggg 4440
   gtcagtgcct gggccagccc cttctcgcac cctgatgctc tgatgggacc atcaatacta 4500
   cagagttggc ccactggctc tgtgggttct ggctccatgg attccaccaa ctgcagattg 4560
   tgcaccacgt agttagccct gcagtggctg tgtctgtact gaacatgtac acctttttcc 4620
   ttgtcgtcat tccctaaaca atacagtatc acaacattaa cagaacatgt acgtcatgtt 4680
   agtcattgag aattggtgtg atgcgtgtgg gaagatgtgt ataggttcta tgcagatacc 4740
   atgctctcat attttggtgt cctcgggggt cctggcacca gccccctgtg gataccgagg 4800
   gatgactgtg aacacagcac agggggaagt tgcctttgta cagcagtaag gtgcaggggc 4860
   ttggcagccc gccgaattcc ggagctcatc agtggttcag tggcacccca gcggccttcc 4920
   tgtttggatt cctgcagggg ctctggggac tggcaccatg gaagcgaagg gtggtgacag 4980
   gtggggtgag gggtggctgc tgaaagcaat tgttgctctc cccgtgggat gtcctgggaa 5040
   gagcatgggt gattttgctg tccgtgtggg gaactcagcc tgcctgtggg gtgtgatcac 5100
   tgtgtggtta tgggcctctg aatcctaacc tgtaccgtgg ggttgtgagg ctggactcat 5160
   agcagatggc ccagaagggt tatttcacac ttctccttac tccactccca ccctgagctt 5220
   tcctggccct gcagccctga ctctagtccc tttctggctt tctgctgggc ccagggcccc 5280
   tgtggcagga gcagaggctg cgcttgcctg gcctgttcct ctgggagttt ctgtggagcc 5340
   tgagccgttg cttctcttgt cacgaacctg ggttcatggc cacagagctc tgagctgtga 5400
   tttcccggca agcacaggga cgggggaagc cgggagcttc tcgcgtgcgt gctctgccct 5460
   tgctaggcag tgaattgagg cttctgggct cagctgatgt tctccaagga ggacttggaa 5520
   tgaacccgag gcacaggggg tgaggtcagt ggtttgctca gcaagaaggc cagagtcggt 5580
   gacgacctgt cacagctccc aggatccctg gctgtggtcc ctggctctgg ctcctcccct 5640
   tcccgtcctc aagggaggtg aaaatcagtg atgcggattt atttactgag tgtgtcctct 5700
   gtgtcagagt gggaggccca cgtttatttt atccccagcc tgcatgatgg ctttcacatg 5760
   ttttctcgac cattcctcac catgtgctct gtgtaaactg aagcccagtg tgatctagca 5820
   gtttgtcact tgggctagtg agtggtgggc ttgaacccat ctgcctctgt tgggaggctc 5880
   gttcttttca caccaccaca ctgctatgcc aggggctgca caaagagctg agcaagtgtc 5940
   ccagccccca gtgacaccag agcagccaaa tctgcggtgt ggacaggggt ggctttatgt 6000
   gtggacagtg ggggctttgt ggtgctgctt aaaatcgccc accagagtcc ccaagtgcct 6060
   gtgggaaaag tctctgaatc tctttatctc accattcact tggatctcgc gtttgactcc 6120
   attatgtagc cttgcttgtt ttgatttaag gcactgatgt aagtttcttg ctcttcagta 6180
   aatgtggtcc tgggaaggta tttggggttt atccccaggt ggcttcctga acccctggct 6240
   ccatctgtcc cctgggggta aggccattat tccctcccac caccaccaag atgatggcac 6300
   attccgtgct gtgaatggct cacagctggg ggaaggaggg gaggggtttc atttgactgg 6360
   agaggcggga tacagagagt ggctgaggag gtggtaaagt ctggtttggg gccatgttct 6420
   gagagtctga agaccatagt gccaccctta ggctggtagg cagagagaag ccgttgaggg 6480
   tgataccgtg actctgcccc agctcggctg tcactcatcc aggtcctcca tgagaggtct 6540
   gcaggggact gtgggaagtc cctcccatag agagcctgaa gtccagtggg agtggtgcac 6600
   acaagctgag ggtgtcgctg agaagggcta gattgtgaca gtgcagagca aacagctgtg 6660
   agtgagtcca agaagctctg gatgccagtt ggcactgttg tttgcgtgtg gtgagaagac 6720
   aggcatatga ggatggggag agtcccggag aaggaggaac tgatgcggct tggtgaccta 6780
   ttaacatgtg ggagctagga gagggccgag ctgctgtagg tgctggcgga aggagaatgg 6840
   ggaagctggg caggcgagag ctgactgggg aaatggcccc agctgcctga gaaaagggga 6900
   agtgctggcc cctcctgcgg gcctctgggg ctggacggtg ctcattcgga tcggataact 6960
   agtgccctgg aacccctaga cattgtgcaa atgctgcagc tcatcccggg accaggcaaa 7020
   cttttgaact tttggttcgg gtctctgtgg gacagatgac tcactctgag cccctcctaa 7080
   cctctgttgg tgattagcgg ggtgagaggc caggagccgc cctgctctcc agcagatcca 7140
   cttgcctgga agagctgaga ttgcagcagt ggagtggatc ggagtggagt ggatttggaa 7200
   cagctgagga agggcatttc cagcacatga gtctcctggg gtcgggctgt ctttgtacgt 7260
   ggatctgtgt tttctgtggg atgggcctgg agtggggccc tagacttgac cttaaaaggt 7320
   ccagagtctc tgggggatga gatctgatga gtgaaatgtg ttcttggggc cggcagcatg 7380
   agcaaaggca ccacctcttc tcacctgcta ctgctgtggc ttcctagagc ctgatggggt 7440
   gttggggttg ggaaggtggg ggagctgcag cagccagagg ccagtgggtc cagcctgagt 7500
   gctgagtgca tagtgcagac ggggcctagc acagttgggc ttttgcctgg agctcagggt 7560
   gacctccgga attgcctttc agcttttatt ctctccgatc tccagttacc tgtgctctgt 7620
   gaccttgggc aaaagccaga cacccaatac ggtggaaagt gaacctatct acacactctc 7680
   tgcaaggaag ggctcccgga caaacaggaa acaggctcca gcggctggca ggaagcccag 7740
   ggaagacccg ccctatccaa aggatcacct cctcccctcc atgggcagat taggaaactc 7800
   tgggcccttc cctcctggtg ttctgtgtgt gcccccagct cacacttcct ttagggctgg 7860
   gagagtaaaa tggcagttcc tgttgggcga ggcatgagtc agcggaagga gatttggtgg 7920
   ctgagtgttc atgccctcca ggcatggctg ttaaatattt aaatgacact atggtattga 7980
   tcacttggcg tccatcccct tggggagagc agttcctgcc tgggattcgg ataatagtag 8040
   tgtcttggat ttagacaaac tccccaagaa ctcagagggc tgtataaatc tgccccacag 8100
   ttgtcttatt tcctccttgg agctcactgt caggaatggg gacaatctga gttttgccca 8160
   ttttactgac ggagaagaca tccagagagg ttacgcgttc cctccgttga tggcacctag 8220
   ctaccaggcc tggaatctcg gcatctggag tcctgctcac acggctgttt tgctctgccc 8280
   gcttagccct tccctgcctt cttccatagc ccttctgcca ccacttttca ttctagctgc 8340
   tgaggacatg gcctggagca aggcccctgt cctctgagct gaggagacag ttgactagaa 8400
   ccgggattgg cagggagggc ctgtaggaga ctgagttcct agcccgtgtc caggtccctg 8460
   ggctgggtgg gggcagggca ggcctggcct gattgtggat tggctcttac tggccaggat 8520
   cttcctttcc tctgaggaca aggactgtta tcaggataaa tatacccggc tccccattcc 8580
   tggggagtta ctcaccaagg aaaagcaccc cgctgcggat tagattacct ctgctctaac 8640
   tgctctcgtg ctccagcctg ggggagggga tggggacttg gagccctggg aaggggagac 8700
   ccaccctctc ggtcctcccc ttcctgattg agaagcgtct caccgaaggg gctgagatga 8760
   gaccatcgct gagcaggtcc cgatgtttat atagctcctt ctttaggaag ggctgagccc 8820
   tgctctccac ttgcatctct gagcctacca gcctgtttga cactctgggc caccaaggct 8880
   gctcaggttg ccagcaggga cctgaaacct ctgtgcccaa gaggattaga ttggccacaa 8940
   agccaggctt ctctggaccc tcttggttca ggggctggtg gagctggtgt tttccctggg 9000
   gtacgtgtat tagatggcag acagggacgg ctgcccctgt ggcagcaccc tgttgcctgt 9060
   gccagagcct ccagggctgt gatcgcagct tcttcctcgt gcctggtggt tcctagggaa 9120
   gccggccagc cctggcctgg ggcgagcaga gctgagtgaa ggtggcctgc cgcgggaagg 9180
   gggagagctg ccttgcacag agcttgtgct tgctgtgggc ctcttttcag ccaggccgca 9240
   gaataccagg caggtccagg tggagttttc ctgggcccca gggcatggtg gcgagggacc 9300
   caggcagggg agtctgtggg ctctggggag ggacaagaaa gctgcctcag tgaccccctt 9360
   cgtcccacct gtggatctgg cagtgaagtc ctggtgggga agtttctccc accagctttc 9420
   tttttgccag aatggtgccc cttctgccag ttgggggcga ggctgcagct cctttgccct 9480
   ggggtaggat ggagtgggag gcgttttctc tggaagggag cccctgactg ctttgcaact 9540
   ccagtttact ggccaggcgc tctgttccac cttgctccgg aagcactttg tatctgttca 9600
   tcatttaatc ctaatcctct gaataaaatg gtggggttcc caatgggcaa caaggaaact 9660
   gaggcagaag agaagtgaag cagtctgctg ggcagttctg agtcttgcaa gtcagaattc 9720
   cctgtgcaag aagtagcagc cagagtctgc ggagcctgag atgagagact ggagcagcct 9780
   gggtcctcct ctcacacatg cacacttgca ccgccctgga tggaccttta tctcatggct 9840
   cttggcgccc gtccagctgc agcctctggt cttgacagtc cttatcacat agtgagacgt 9900
   ggccaggttt gtgaaggagc cttgagttgt ttctccactg tacgtggggg tggaaggatt 9960
   tggttaattt tctttaactc attgttgtgc cacgtgtcag cctggctcgc cagggtgtgg 10020
   cctggggaac aagctccccc tcccctatgc ctgtgggctg ggaacctgag gggttcagct 10080
   gccacctgtg ctctggaatg cagatgggct ggcccctcag gccccacctt cttggatggc 10140
   agacctaagg attattctag tttaatagct ggggcccagg gaaggatctc accctcctgg 10200
   tccagccccc atgtactgta gctgcccagc cacctgcatt ggggtttctt cccaacctaa 10260
   agtcactgct gcctgatcta ttgcagctgc agggttgaga caggggcacc caggggtgct 10320
   ttgtttgcag agagggtact gaatgggagg atgtgcattg gttgtggtct ccagggccct 10380
   gtgagctgct tgagtcacat ctccaaatga cagttcaggc acagtcccag ggtcttggca 10440
   cccatgccac ctcacgctct ttctaagcag cctgctccat ccttccagct cagccaggtt 10500
   ctctaggaat tgaaactact gcacagggcc tgggggctcc caggtcttcc ccagctctct 10560
   ggaaaattgc tggcttgagg ttgcctgtgg tttctcttca gtggcaggcg gctgtcgggt 10620
   caatggtgcc ctctagtgga cagcgacagg cagtctgcac ctctgcaccc cttccttacc 10680
   agggccacct ctgtggcctt tgtccctggc ttgctgggga ctttggcctc agcctctaga 10740
   gagccccccc cccccccatg tagcatgggc tctcccaggg actcttgctg tccctgtctg 10800
   aatgccagtg cctgggcctg ggtcagctag cccagaaagg caccaagtct gcggagtgca 10860
   gaaaggggat gggagggcca gggggagctt cctcccatag ggtgacttgg gtctgagggt 10920
   ggagccagaa ccttaacgga gtgatgtgaa gaagtttgag ggcttccttt gctcgaatga 10980
   gggctcactc ctggattctt ccaggcagag tatctggaat ctgacctcct ggcaggtggg 11040
   aacggaggat tgttcctggt ttgcagggta caagtgaata catttttctc tgaagagggg 11100
   caccttcctt ggggggtaca acagagggaa tctctgctcc tggggtattt tgagcaaggt 11160
   gaaaggccat tgctgtatct gaaaactggg ggaaccggat ggaatagagc cctccctgct 11220
   cgtggactcc tttgtataac cctgggctgg gtgtacttgg ccaggggagg agtttgtcag 11280
   cttgcgggga gactttggcc cttaaccatt gtgcagcctg tggggaaaca gcaggtgttt 11340
   gcagccgtcg gagtctgaag cagagctcgg agcctgctcc tgggctgcct cccgtgtccc 11400
   tggctctctt ccttgaggag tttgcgagaa ggtgtttcgg gggatagtgt caccaaaaca 11460
   cgtcccagtc accaggcaag gatgaagaag ggtagggaga cactgtagct tctgtaggta 11520
   tttgttgggg ttcttctctc tctctgacaa cagtttgtgg aaaactgggg ggaaagtagt 11580
   gtgtcttatc cctaacagct acccatgcta tgcttgttaa ctttattgct ttacttttct 11640
   cctcaaggtt gtccttttca tgtctagcct agaatccacc cagaaagatt taggaggaaa 11700
   actcaggctt tggatcctac cctgtcctct ttggaggaag atctttttct ggatttttgt 11760
   ttgtttgttt ttaggattga tttatttatt tctttgaaag agttacagag gagaggagag 11820
   gcagagagag agtgagcaag agtgtgagag cgagtgatat attcttccat ctgctgtttc 11880
   actccctaaa tggctgcaat agtcatgcct cggccaggct gaagccagga gccaggagct 11940
   tcttccaggt ctcccacatg ggtgcaggga cccaaggatc tgagccatgt tccactgctt 12000
   tcccaggcac gttagtaggg agctggatag gaaatggagc agctgggatg ctggcatagc 12060
   tggcagcagc ttttacctgc tatgccacag tgctagcccc agtaaattaa aaattaaaaa 12120
   acaaattgca gtcataaaat agaatccagt ctgtcagcca atcagccaaa cttggactcg 12180
   cacatgggtg agggcggagg ttgtccgatg gagtgggcag ggaagattaa ccatttgtct 12240
   cactgaatgt tggtagccag agaggcctga gctcagagcc cagcttggtt actgcagctg 12300
   gccttggctg tggggaagga ctctgaatct gagttttact gtctgtctgg tattctaagg 12360
   aggcatgcag tagtgcaggg gaagctcgca gtacctggta ggcattcggg cttagctgct 12420
   gctcctgcca tcccttccag aattgcgcta gttgcctgct ttggggggca tttcctgtgg 12480
   tttttcccag ctggctctac ccagagtgga gaagagggca cctcgcccct gctgggctgg 12540
   gcccggaggg aaaagggaag ctgtgtgaaa ccaagcagca ggtgtggccc tgcaagggcc 12600
   actgggattg gctgtggaca ctggttcctt ttccgcagtt acctagacac atatcaactc 12660
   cctctggttc ctgtttgctg gaggcaggaa gccagacctc cgccacagga cctggagtgg 12720
   gagggtgctg caggaagcga ccttcctcgg aagtcaggaa tttccttgga agtcagaccg 12780
   ggaccttggg ttccagagat cagaacaggc tttccctctg accatctgac caggagcctg 12840
   gggtaggtgt ggaggcctgg cgggagggat cagggcaggc agctgggaga aggtctggcc 12900
   ccgtgaatgg aggcctgggt gggggctttg cctccctctt ccttccaggg agtgagggtg 12960
   tgggccatga ggaaggatcc gaccagcagc tttaaggctt gcacctccaa cattcaagaa 13020
   gttgaacttt taaaatacag gatcaccttt gggacacaat agcctcttgc aggctgggtt 13080
   ttgggggttc ggggctttta aggagacaga agtcctgcca ctcactggct ttgctacctg 13140
   gggcaagtcc ccttcctctc caagcccacc ttcctcttct ctaaagggtg ggtcacagtt 13200
   aggatgcact gagaccacgt atgcaacgcg cttcgtatga tgccttgaat ggtactaaac 13260
   atagccatcc tctttggatg attacgatcc caaaggccag gacatcgtgg ggagctgcga 13320
   gtgagcaagt ttgggagcat ggcaggaatg agggcgtggc atctggggcc aggctgtcag 13380
   cgctgtagtt ctgttgaggg tcccaggaag ggagggggga ggggtcaatg gttccacccg 13440
   ggccctctgg ctgtacccat ctagggaaac tccgggcccc tacttggctg ctggctgcct 13500
   gggtaatgga agcttacact cgagggccag ttccgttccc ttgctgctgt taaaagcaga 13560
   ataggagcct acgagcctgt ggggccgggc tgcttgccag gctgcagcag aaacagcgag 13620
   cagagcagtc ttgcccctgg ccactatgcc agccatattc cagcccctac ctccccacac 13680
   acctcactcc atagcctgga acctagcaaa gagcccgcag cctcgggttg tgctcccacc 13740
   tgctcaggat ggcaggtccc accccaggtg ttccctccgg tcccagggct gagaaagtgt 13800
   gaattcttgg aagctaagta agggcctggc actgcccgtc ccctccccat gacgtggggc 13860
   ccctccctcc cactctctcc tccgtgccct gaggagtcag agttccaggc aggcatttct 13920
   cactcacgcc agccagctgc acagccgtgt cgggagggag cagggggcca ggcgcctgcc 13980
   acccggaggt aggcaagttc caaggggcag cccagaggta agcctcccag cgcctcctgt 14040
   cttgggctgc agagaagagg tcagctttgg gagccattct tgtcggggct gggctgggct 14100
   ggcagtttcc tgtcctgagg ccggtcattt ctctcctggg tggggcaggt gctcagaaga 14160
   cattaactcc tgtggcgcgg gcaggcgggg cactgccagc agagttccca ggatcctcgg 14220
   ctcttacctg cttcctgtgc tttctctgca gacgccctgc tagcagactt ggagtctacc 14280
   acctcccaca tctccaaacg gcctgtgttc ctgtccgagg agccccctta ctcctaccca 14340
   actggaaacc atacctacca ggagattgct gtgccgcccc ctgtcccccc acccccgtcc 14400
   agcgaggccc tcaatggtgc agtccttgac cccttagacc agtggcagcc cagcggctcc 14460
   cgattcatcc accagcaggt aaggtggggc caggggccag gaagcctcct ggggtctggg 14520
   ggcaacagga gtttgtttgg caagggtgtg gggtgcccat ggaggagtag gatcagcacc 14580
   aggctgtcgc caaccagatg ctgctcttcc cacccaccac gtgaaggggg cagtgaccaa 14640
   gtattaatca ccagtatccc agtcagactg tggaaattcc ctggtgagac tgctgagagc 14700
   aggagcttcc tgggagtgac tcgggctgct cctgtcccct ctgagcattc ttgttcctgc 14760
   agccgccatc cccatcgccc gtgtacggct ccagtgccaa aacttctggt gcctccaacc 14820
   cccaggacgg cgttggttcc ccgtgttctc gagtaggtga agaggaacac gtctacaggt 14880
   acagcctcag cccctcccca gcacctctcc tccggctctg gaactcaggt ccctggccag 14940
   tgctggcctc ccagggtttt gcctccaggg cattggttaa acccgcagat ggccttaagg 15000
   ctcctggcca gctcatgcgc ctgttgcctg gcctgtctac cttgtcactg ccatctctgt 15060
   gtcctgccca ggcaggaact ggagtagtgg aggaactgga gcaagacttc cttcctcctc 15120
   tcttcttacc acatcccttc cctatcctct gtcctcttgg tgggggagac tttacttcct 15180
   cccttattct ccttgagaat ttggggacct tcaagccagt gcaaggccac ggtgtatctg 15240
   tcactgtctt gtctttgctt cccaaagctt ccccaacaag cagaagtcgg ctgagccttc 15300
   acccactgtg atgagctcct ccttgggcag caacctttct gaacttgacc gcttgctgct 15360
   ggaactgaat gctgtgcagc ataacccccc aggctttcct gcaggtagga ctggggcccc 15420
   ggggcagggg tctgtcagac cctacagccc acctgttagc ttctgtatcc ccagagggct 15480
   ctgggactaa gtggtctttt tctttctctc tcctcaagat gagaccaact ccagccctcc 15540
   attgcccggg gccctgagtc cccactatgg catcccggag aacaacagcc ctctgggggg 15600
   caaagccggg ccgatgacaa aggaaaaacc taaacggaat gggaaccggg gcctggagga 15660
   tgtgaggccc agtgtggaga gcctcttgga tgaactggag agctccgtgc ccagccccgt 15720
   gtgagtgttc atggggctct gcccagcctg gggaggggag ggtcttgcct tgacagtcca 15780
   tggcctccag tgtccatcat cttagccctg ctgtcaccca ttcctgcaaa tgcctatctt 15840
   ttagtataga agtatatagt gtagtatagt atagatccac acagacacca tccctgcctt 15900
   cagccagcca ctgtcagttg gaaaaagaca gacctggttg ccgataactc attggtggtt 15960
   agcacagcct ttggagccat gtaatggtgt tcaaatcttg actctgccaa actctaaagt 16020
   cttagttatg gggtctgggg aaatgggatt atggggataa gaccatcttc ccagtagggg 16080
   tgagactaca tcacatggaa agagcttggt ttctggctca tggcacccgg tgcacacgca 16140
   gtgcttagtg aagtccagct ctcactggag ctgttgtgtt gctgtaacag tctcacacct 16200
   gcacgcaacg tggaggagga cacacatagg gtgcttgggc tgtttgacag gtgagctctc 16260
   acctgggcca ggaggccgga ggaggattcc tgggaaaggg gccattgaaa cctctgtcct 16320
   ttctcccaag tcagctcttc ttggggtgct ggccccttgg cttacagccc cttcccagtg 16380
   atgagtctcc agggaagatt aggcagcgtg ggcttcccca gggttggagg cagcaggacc 16440
   ccagtgcccg agctcaggtg ttgggctgag ctcctgtgaa gcctgtggct gggctcggtc 16500
   cctcactgat gccatgcctg ccctgcagcc ctgccatcac tgtgaaccag ggggagatga 16560
   gcagcccgca gcgagtcacg tccagccagc agcagacacg catctcggcc tcctctgcca 16620
   ccagggagct ggacgagctg atggcctcct tgtcggattt taaggtgcca agtggacccc 16680
   tccaccttcc tgccctaaac tgccctcccc ccaatcttct cccttacctc aggatgagag 16740
   tgcctcagtg ttcaggtgcc tcgtccctga gccggggccc ctgcaatgcc ctggccttgc 16800
   caggattcca ggatgcaggg cagggaggcc aggccagtct gtgtgtgttg ttgggggggg 16860
   ggggggggtt ggagtggctg tgagtggctg tggccgacag tgaaggcaga gaagcactca 16920
   gctgggacat tccttcctcc ctatgcactc ccaggcaccc aggtccaggg gatcaggagg 16980
   ctgggatttc ggacaaaggg ccctgttgtt tgtggctggt tgggggggga aggggtgctc 17040
   ctcatggcca tgggaacccg tgtctgagaa gttcattgcc ctagagtccc atctcctccc 17100
   acgattgccg ctggccgtct ttcaacacat ctttctttct gcctctggcc tccctcacta 17160
   gattgcctca gcctttctcc ctcttcctca gcattcccac aggcgcactg tgtgggaaac 17220
   ctaaccggtc aggccactca gtgggccagc cctgcctgag gtgccccttc cccccgcctt 17280
   gcgggccttt ctgggcctgc caggctgtga tgttttattt agagcatgtg gagccttggt 17340
   ggggattgat attgacaggc ctctcctgca gttgctgact ctacccttcc acgtggtcag 17400
   ctggagactc tgaggcacga cctggggaac atgagaaagg cagcgagcag cggtgcattc 17460
   tcggccctga tgcctccctt cggcttgggg ttcacggtgg gcgggacagt ggctgagagt 17520
   aggaggtgga gcctgggctc acgaccttag tgctgaggct ccagctctaa gccagcggtt 17580
   ctgggcagcc gcagtcagaa gcaagtaaat tggatttagg cattggccta gggctgtaaa 17640
   ggctatagct gctttctgga ttattattat ttttttaaga tttatttatt tgaaaaacag 17700
   tgacaaaggg acacacagag atcttccatc cactagttca ttccccaaat ggctgcaatg 17760
   gctggggtag ctggggaccc agaactccat ccagatctcc cacgtgggtt acagaggccc 17820
   aagtacttgg accatcttct tctgctttcc ttgtctcatt aggagggagc tgggtaggaa 17880
   gtggagcagc cggaacttga actgttacac tcttgtccac ctcaccttag gctcttctcc 17940
   cttacctacc gagagcgccc gcaagtgtgg cttagtggct gctttctgtt acttgggttt 18000
   catgaggccc atggatggga agcccagaaa ggcctctttt ggggtctggc tgttcttcag 18060
   gggtcagtcc ccaggcttca gctttgcagc cacctgggtc agtgtaaatg gtggctctca 18120
   gagcctaagg ccgagcccgc cccttgctgg gaacatcctg cctcacttct ccccctttct 18180
   ctctcctcgc ccctctgcct ttgtcttcct cttcctgcag accagctcct ccactgtggc 18240
   cctgagctcc ctggggccca gctcagctcc gtcctcacac catacccttc cccctactcc 18300
   ttctcctctt cctgtgccat ctgcctccag atcctccttc ccaggccagg accacaccca 18360
   ggaggtcctc tgcactgagc aacacggtcg gggccatctg ccttctgggg ctcccagctg 18420
   gcttgatttg gctggccttg gggtaactcc tgattccccc aactcaaggc ctccctctgc 18480
   agagggatcc ctggggccgt ttggtgccga gagccaagtt cgagtcagga gggacctatt 18540
   gaaacccaca catgagctct gtggggctct gtcctgccac actccaccca ctgctaggag 18600
   tgcagattcc ctggagtttg gagacccccg ggggccactg gctaaccctg tgtgtccaga 18660
   gaaggccatg gatgccagtg gggtgtggcc atgggctttg gagatgtcta ggcccaagac 18720
   cacccgcgga gctgcttgca gcttccagga agtaactgag ccagctgttg tggccatgga 18780
   ccgacaggcc atcttcccag acacctggag tctcacagag gagtgtggcc aacagaagca 18840
   gagggtgagg acacaaccca gggggctggg gagcagctgc cctccccctg tcgacgagga 18900
   gcagttgggt ggcaaggtgc ccaagtgggt aagcctggtt ggaccatccc aggaacctga 18960
   gaccccaagg atcccaaagg gcaccactgg agagactgct gaggccagga aggatcaatc 19020
   ggagcttcca cacgccacgg tcctgggcac acccagcgcc acggggagga tttccacctc 19080
   tggccaggca ggcgccagct cgcacagctg ctaggcatgg ccctgtgcgg gttgagtgta 19140
   ggccactcct tgctaactgc atgcagcctc tcactccgcc cacgtctgct tggacagaaa 19200
   atgctccgat aaccatggcc tgcaaccctg atgaccagca cttggcagtg gttcctctgg 19260
   ggcccgcaga gctccctgga ccctagtgtt ctctctgtga ccacccatct cctgcttctt 19320
   gaagcctttt ccctcgagat gagctgccta gcacagtctg tgtgtgaaat gaatggttca 19380
   aagcatggat tttggaacca gacacaagcc acacctccag cacttgctac ctgtgtgatc 19440
   ttggagaacc ttcccagcct cagttttccc atctttaaac tggggtagta atggcccatt 19500
   ttcctccaag gttgtctaga agattaaatg agatgatttt ctggaaaatt tttagggcac 19560
   agcacttggc tcctcagtaa gccacatctg atgttattgc ttggtttttg cttgatcaaa 19620
   actttatgcc tttaacataa gcaaagcaac agaaaagaaa aaacagtgtg aacaagtagg 19680
   ttataataca agcaagggag aattctgttg tcctgtctag agcacctacc tattatcctc 19740
   tgtggtaatt cttggaataa ccttgtgagg aaagccaaga agataactgt taccccattt 19800
   tacagatgca gaagctgaga cccctggaag ttaagtttcc tggggctggg agctgacttg 19860
   gcgatgaatg ctgggccaga ggtatctctt gtagcctgag ctcctgggcc agtttggccg 19920
   ctctctgcct ccacctgctc agcttagctc ttccttgccc ttctcccttg gtgcctgagt 19980
   gtgggctgag cagcgctgtg cccaagccca gagtgggact gtgacagctt cccttccccc 20040
   ccagatccgt tctgtgatca ggaggagccg ggagacaggc cacgcacacc ccatgtcccg 20100
   ggagccttcc cctcgccgcc ggctggaccc tgcgaccctg agcaggaccc catcccagga 20160
   gcgtctcatc gcagagctgc agggtcggct gggcatccag ccggaggcag aggaggcagc 20220
   aggggccccc gcccaggact ggctgactga gggcatcgtc atcaccgtgc agccacgcgg 20280
   gaggcaggct ggggggcagc ttgtagagaa ggtagccagg agatgctgag ggctagacca 20340
   ggctggggcg gcagtgttcc ccagccccag tgctggctgg aagggacact gggggtgact 20400
   agggcgggct cctgcatcat gcggccttgt acggtggtgc gagagtggga agggccaggg 20460
   agagcctggg gacgtcacct ttctgccccc tgtgctcgcc gagctgcttc cctcttcctc 20520
   atctctccct tctttctcct tcctaggttg tcttccctcc tggctctccc atccccctga 20580
   gaagaaccac ctctgtcctg gcttctcctc cttctgtccc tttgcaccag catcaccaag 20640
   acacctcggc ctgcagctct tctcccctgc ccagcctgcc tcctccttcc cctctggggc 20700
   tctccgcctg tacctatggt tctcctgggg tccagagtgc aagggcagag ccccagggcg 20760
   atgtgcaggg ccccagcccc cccacgcctg cacctcacag tgtgaggtcc gtgggctgcc 20820
   agaccgatga ggacccgctc ttccccccga tgcaggcagg cctcccgggg tccccagctc 20880
   caggtgtacc tgtttgtccc ttgtcctgat gcctgggcta tgcccccact caatgtacac 20940
   acacagaaac acacacacag acattgtctc tgagcccaca gccctgatga agtagccaga 21000
   gtcccttagg acttgaccct ggaccccatt gaatctgatg tccaggcccc agcccttggg 21060
   aatccagctg cccccttgct ttagtggggc ctcccctctg cctgcccttc ccccactgtc 21120
   tgggacctgc gtcaggctgt catctgagag gctcccagaa aggaaatcag aaactcacgc 21180
   tctcccctgg cagcccagta cttgctgact ttactgactc cactcacctc cccttctggg 21240
   ggtcccaggc ctgcggtggg ctttacctcc ctccctgact gcatctcgcc ctgggttggt 21300
   cggtctgtct gtctgtctgt ctgtctgtct gtctctctct ctctctctct ctctcggggc 21360
   tgtttcactc tagatctttc ctcctggttt tagttctgcc agtgggggaa acatcttctt 21420
   acctatcctg aatcccctcg tgtgcaacag cccaatgaca aggtcctgtt ctttcctgtt 21480
   taattaaatt tcttcttatc tcctccccta tctaagatcc aaggcctgga acaaagagtg 21540
   gacggagagc tgtgctgggc ggccagttgg cctccgaacg gccggcagag cagtccggaa 21600
   gggcaagacg agggaggggt cagtgacagg gctgggcact cagttttctg cttgttgtat 21660
   tcccctggac ggtgcagcct ggtgccatca gccctttgac ttctgggagc cccccaacca 21720
   ttaggatgcc ctgccaatgt cattcctgcc ttaagggaag gctctccagt cccccagagt 21780
   caagtccttt ttgctatagt cactcatctg gtaaacgaaa tgggctaccc caagcctcag 21840
   aagtcctgac ttgtccctgt ttccacagtt catggcccag ggaaagacgg ggagcagctc 21900
   accccctggg ggacccccaa agcccgggag ccagctggac agcatgctgg ggagcctgca 21960
   gtctgacttg aacaagctgg gcgttgccac agttgccaaa ggggtctgcg gggcctgcaa 22020
   gaagcctatt gccgggcagg tgatgacggg gagagaaaga ggagggagtg ggcagtaggc 22080
   cggggacttt tgcccctctg ggtggtgggc tccgtggtag gccacagccc taagccacct 22140
   tctgtcttcc tcctcaggtt gtgaccgcca tggggaagac gtggcacccc gagcacttcg 22200
   tctgcactca ctgccaggag gagatcggat cccggaactt ctttgagcgg gacggacagc 22260
   cctactgtga aaaggactat cacaacctct tctccccacg ctgctactac tgcaacgggc 22320
   ccatcctgga tgtgagtgcc cgccctgcag cacccaggga cagcggtggc atccgtagtg 22380
   caggccctgc tcccacactg gctcttccat ctgcgtggta gctctcggag ggtgcaggtg 22440
   ctgccttggg aggcttgaag cggtcatgtc ccatgccgaa gtctcactgc cgggaagcag 22500
   cgggttggga gtcagagggc ccggtccaca gatagatgga gtcaaatgct ggctgtctca 22560
   ccctcctgct gtgtgatgtt agggaagtgc cagcctctct gagcccgtgt tccttctctt 22620
   agaaaactgg agtcacatgg aacctatgct ctcgacccca tgtgaagagt aaaggcgaga 22680
   gagtctagca tgttgtatcc tccagtgaat ctgagggttt catgatatgc atagctgttg 22740
   ggagaggccc tggtcttgag agtgcttttc tctttccttg gctgtgagcc tggggagggg 22800
   tggggaggac agcctgcctg tgagtcttgg ccatgacccc acactttgct ttcacagaaa 22860
   gtggtgacag cccttgaccg gacgtggcac cctgagcact tcttctgtgc ccagtgtgga 22920
   gctttctttg gtcctgaagg tactgaccgg tgggctgact caagctcctg ggggcgggct 22980
   gggaggcagg gctgggaggc cctgctgagg ttgtaggccc tcactctgcc cctgaactcc 23040
   ctttgctcag ggttccacga gaaagacggc aaggcctact gccggaagga ttacttcgac 23100
   atgtttgcgc ccaagtgcgg cggctgcgcc cgggccatcc tggagaacta catctcggcc 23160
   ctcaacaccc tgtggcaccc cgagtgcttt gtgtgccggg taaggagcct cctctgcgtc 23220
   tgggttgggg gagggctgga gcagtcagtg cgggagaagc gtctcccttg tggtactctt 23280
   cgcgtccagt gtggaggccc ccgagaggtg accagcagga ggtacattcc ccaagtccag 23340
   caggtcctga gcagggctcg tactgatgct gagacagact aggagccagg cagacatgtc 23400
   atctgtcgta gtccagtgcc gagggcctgg gcccgagtgt gagtcgtgtg agacagcgta 23460
   ggtattgggc aggcagtttg caaagtgcat cttggtgggc agtgaagccc acagactctg 23520
   cagcgggctc tctggactgg gttcgctggg gaaacactgt tccgtgcggg acttctcaga 23580
   actttttagt cctaaacatg gatgcaaatc tccaagtgca aatctagcag cgggagctga 23640
   gtacagaccc ttgttttcag tgtcagggat tccgggagca ctgtcacagc cacctgggta 23700
   ggtgcccaga agtcagactc acatgtgagc ccaggctgtg cagttcagga gccgggtgac 23760
   ccagggcaac ctcactggta cgcctcagtt gccatctgtg ttggaagaac taaatgagca 23820
   catagcacag cactcggcac ccaggggctg attagcaagt gggagtcact gcgagtggga 23880
   cagccaggac cgtctcttgc ccctgtcctg ccggccaggg ccccagccag gatggacagc 23940
   ccctgagccc ccgcccctgt cttctgcctg gcaggagtgc ttcacgccgt ttgtcaacgg 24000
   cagcttcttt gagcacgacg ggcagccgta ctgtgaggtg cactaccacg agcggcgcgg 24060
   ctcgctgtgc tccggctgcc agaagcccat cacgggccgc tgcatcaccg ccatggccaa 24120
   gaagttccac cccgagcact tcgtctgtgc cttctgcctc aagcagctca acaagggcac 24180
   cttcaaggag cagaacgaca agccctactg tcagaactgc ttcctcaagc tcttctgcta 24240
   ggcccgcctg gccatcgccc ccagcccagc atccccgact gctactgtga cccagagacc 24300
   tcgcccaggg gtgaggggcg aacgaccgtg actggaactg gaacgctcat cctcagctgg 24360
   ggcaggatgg gaggagccag gagggcgcct gcctgcctct ccttgcccct gccgggacct 24420
   ctgggcctcc ttcttcctcc tgtggagctc cccaggtgcc caccctgcac ccctcccgga 24480
   ggtggaggtt ggggcccatc ctacaccaca tgccgtcccc atggaccggc ctggctaggc 24540
   cagcacccca cactggagcc atctcttact tgtattttgg tagtggagtt ggagcagggg 24600
   gtggcaggca gggtcagatg gggcatctct ttatccttgt ctgcccctca ccccaacctg 24660
   actatcctgg tgagagtgtt gggggatccc cagctctctc cagacaatgc cagcatcgat 24720
   ccatccctcc agggctggaa gcgcccacgg agccacggga ggttcctcgt gtccttcccg 24780
   cccttctgtc tccccaggcc ggggtgactg ctccccacct ccctgtgccc ttttgtaact 24840
   gctctggtcc tactgagaaa ggcctgtcca gcaataataa cattttatag ccactccctc 24900
   tgtcctggga cggccggcgt gaggtgggag caccggcagt cctggacact gtatctgccg 24960
   actttgatag atttctacac tgaggtttga attcttaatc tcccgagttg cttttacttc 25020
   tctatacaaa atgattttga agagatttta aagatgttcc cttttgtagt ctcctccttg 25080
   tccaccgcca ctgggcccgt cggcaccggg gctctggatt gggagtttgc tttgtactga 25140
   gggcttgggg tggggaagca gtttgtattt tattttttct tagcacaagc aggtgaactt 25200
   gggagcagct ctgcacctcc ccttctttgc cttcacagct caccaggact gttttataaa 25260
   ctgctgtatt ttgaaaccct ttctgtaact gcccgggcca gtgagctctt ggctaaagct 25320
   ggcctcaggg tgcctggccc tttgggacct gaacttctga acctcatctg tcctattcct 25380
   gggggaaggg aagggaggca caccctggtg ggctgctccc tgtgcgagta agccgcgagg 25440
   agcatccgct cccccatgga tgctgtggcc gcaaggagaa cccccagcag tcactcccgg 25500
   ccccctcacc acctctgggc tcttgtgcgt cctcagcaga gctggagtct gattttgact 25560
   ttaaagatca caaggctctc ttgggtcttg gttccactgg ggcttccctc cctggagtct 25620
   gggtaggggt ttctcccctt ttacaggtga ggccggctct cagctggtcg gtctgaggag 25680
   caggtcccag gtagttgttg cacaggggcc tgatgggatt tggggcagag ggaaaggacc 25740
   ccactggagg cacagaaagc ccccagagct gtccttccca cagctgaagc aggagcagcc 25800
   aggcccaggc ttcttggtca aaggaaagag accccggagg gtctacagtg gggagatgca 25860
   gaccaaacca tgagtctcga gcagaggctg agtgcacggg ggtctcggga gacactgcct 25920
   cctttgtggc cgaggtgtgg ggaggtgagc ttggttcccc caaaggtcgg ctgtctccac 25980
   ttccacccgc gggccttacg ctctgtttac agtgacccat cctgggccct tccccgagag 26040
   gggctggggc ttctgtgctc tatccccccc accccgagtc aatggttacc tgatgatttg 26100
   acttcaatct ttttatcctg tgtaaacttc taatctggct tttcccattt caattcttgt 26160
   gatgtatgcc aataaagttt gccattattt tcaacctgtg taagtgccat ttcttggggc 26220
   cccagcagag accaaaccct ccccagagcc aaggaccacc agattgccgg taaataattg 26280
   acattccctt atctgaagcc agaagccctg gggatgtcac aggtctttcc tggcccctgt 26340
   gagagttttg ctgtctcctg ggaagatggt gacaggaggc tggggagcag ttataggagc 26400
   aggccccacc cataggtgct ttcactaggt tccctgaggg catctttacc tagacctgcg 26460
   agtgaccagc tgaaagaatt cccaagagag ctgacaagca caggcagcct gctgtgcgag 26520
   agcagagcca ggaccaggct gtgatcccag acctcggctc tgcacactcc ctgtgtgcag 26580
   ctaacatgcc cacccctggc ctggaggacc ctcgacagga gccgctggcg agtggaaggt 26640
   gcggtctcac agttggggtg ggtctctact gcctcctggt ggtcatgtcc tggtggtgcg 26700
   tgtctcccct ttgttatatt gtcatattca aagcccctgg atctggctgt acagctgtgt 26760
   tccacatcaa gccgggacct cagtttcctc tcgtctggaa ggctccgcaa gaggagcacg 26820
   ttgagtggga atctgaaccc tggcctcttc acagtgagct tagaagtgaa aacgaccttt 26880
   gattcaacca aagataatga cttggttaag tctatgttgg attcaatttt ttttaatcct 26940
   tcctcctgga ggtttttttt tttttttttt ttaagattta tttattcatt tgaaaggcag 27000
   agttacagag agaaacagaa agaaagtaag aaagtcttcc atctgctggt ttactcccca 27060
   aatggctaca atggctggag ctgggctgat ccaaagccag gagccaggag cttcttccag 27120
   gtctcccacg tgggtgcagg ggcccaaggt cttgggccat ctgctgctgc tttcccaggc 27180
   catggcagag agctgtattg gaagtgtaac agccgggtct cgaaccagtg cccatatggg 27240
   atgctggcac tgcaggtggc agctttaccc gctataccac agcggtggcc cctggattca 27300
   gtaggtttat gctacacttg taaatgtgct ttttcacacc actgtgttct atggatattt 27360
   tcccatgtca ctgaatatgt atctgtgtca ttttcaaata acttcatgat aaactgttac 27420
   attggtgtta ccattggaga tgttacttat ttgtttcatc aaagccagga tgtccttgga 27480
   aaggaacaga cttggagaag cactttgaga aggagataga gtttgaaagg gatcctgtaa 27540
   tcctagattg catttctgaa accagtatct gctgggtgag aattgggcga atcattcaac 27600
   ccctctctga atttttccat ggataagaca ggaatagtga taccgtacct cctcagaggt 27660
   gcagaaatta acattgggga gtgttggccg gcgccgcggc tcactaggct aatcctctgc 27720
   cttgcggcac tggcacaccg ggttctagtc ccggttgggg tgccgggttc tgtcccggtt 27780
   gcccctcttc caggccagct ctctgctgtg gccagggagg gcagtggagg atggcccaag 27840
   tgcttgggcc ctgcacccca tgggagacca ggaaaagcac ctggttcctg gctcctgcct 27900
   tcggataggc acggtgcgtt ggctgcagcg caccgggcca cggcgggcca ttggagggtg 27960
   aaccaatggc gaaaggaaga cctttctttc tctctctctc tctctctctc tctctctcac 28020
   tgtccactct gcctgtcaaa aaagaagaag aaaaaagaaa aaaaataaga cctggtgaag 28080
   gagccaaagt acttgcacca tgctgagcgt gcagtaatcc actgttacta gaagaggatc 28140
   tccgcagtaa tggactcctg cttcagggca ggaggagcct ttagaacaaa atacagattt 28200
   ctgtagattc tgaaccagga gggctggagt agcgcaccgc gctgatccga tggcaggagc 28260
   caggagccag gtgcttttcc tggtctccca tggggtgcag ggcccaagga cttgggccat 28320
   cctccactgc actcccgggc cacagcagag agctggactg ggagaggagc aaccgggaca 28380
   ggatccagcg ccccgaccga gactagaacc cggtgtgccg gcgccgcaag gtggaggatt 28440
   agcctagtga gccgtggcgc ccgccatcct agggcgaatt tttaaagatc cagctcccta 28500
   tcatgtgcct gggaaagcag cagaggatgg cccaagcact tgggcccctg catccacgtg 28560
   ggaaaccctg atgaagtttc tggctcctgg ctctggcagt tgcagccatt tgggggggtg 28620
   tgaaccagtg gatggaaaat ctctctctct gtcctctctc tctaatgctg cctttgaaat 28680
   aaataaatct taaaaaaaat attgagagag agaggctggt ttactcccca tatgggtgca 28740
   acctggtcca ggggcaggcc aaagccagga gcctggaact ctgtccagtt agggacccaa 28800
   gcactcaggc catcttgtgt agagctggac aggaagcaga gcagctgggt cttgaactgg 28860
   cactcggata tgggatgcca gcatcgcagg tggcagcagc ttaacctgct atgcaacgct 28920
   ggcacccctg agggtggttt tgacaggttt gggaataagt cttgctaacc ttgacttaag 28980
   aagctggctt cactggggct ggcgctgtgg catagtggct taacatcctg gcctgaagtg 29040
   ccagcatccc atgtggtcgc cagttcgaga cccggctgtt acacttctga tccagctctc 29100
   tgctaggaaa agcagtagaa gatggcccaa gtccttgggt ccctgcaccc gcgtgggaga 29160
   cccggaagaa gcttctggct cctggcttca gattggccca gctccagccg ttgtggccaa 29220
   ttggggagtg aaccaacaga tggaaaacct ctctctgcct atatataata agtaaataaa 29280
   tatttgttta ttataaataa ataatttttt tttttacaaa aagctggctt catgaatatc 29340
   tccaaagagg tacaaaacaa gactcaggac aggaatatta ggcctccacc cttagactag 29400
   ctttgttaga cgagagttta gagaaattga acgtggggcc cgcccatgca gccccacagc 29460
   atgttcatcg catgtggccc agataaactt actggaaact tcaactctgt caagcgagaa 29520
   ggctgccagg ggagggaaca agcgatccat gcaaaagccc tggaaaagca gcaaagggca 29580
   ccgtctggct ggaaggaatg agttaggaag gcgggagatg agaccgtggc gtggagcggg 29640
   gagcagaggg tgtccttatt agctgttccc gtttactccg ggtttctgga attggggagc 29700
   ccgcgagttc tgagcacaca aggaaatgcc ccgacttagg tctcacagga tcaacacacc 29760
   gcaggagacg agagaccgcg ctgttttcaa ggcgggcgcg gttattcctt agccccacgt 29820
   cgtacgtggc tgaggtggcc aggtcggctc tagctccctc ccacacgaag tccgccccag 29880
   cccgacagga ggcagcatcc ccgagaaagc gcctccaacc cagacgggcg cctgtcttag 29940
   ctatactcac ctcaaaggcg gcttcctgtt ggttggagtg gcagcaatat caaccaatgg 30000
   ctgcctatat cagcccggct gtgccgggcc gccgacagcg gcgcccaata ggcaggggcg 30060
   ctgtccgcgg cttctcgacc gcatataaaa agcgcacgcc ggcgcgcggc ccttctctgc 30120
   caggcgtcct cgtgagaggt tcgtgcgggc cgtggaagtc gggggcggga aaaactagag 30180
   ggttttaggg aaattttggg ggttggggag ggcggaccgc aggggtcctg tcgtggctcg 30240
   cttcgggcct gggcggcggc ggcggcggcc gccgcctcag ccctggcgga cgggagctcg 30300
   ctcgtggcct ggtccgcgct gggccctgga cgggtaacgc ctgtcgggga caccgggact 30360
   gagcgccctc tctcccctca gtgacatcgt ctttaaaccc cacgtggcaa tccctgacgc 30420
   accgtcgtga tgcccaggga agacagggcg acctggaagt ccaactactt ccttaagatc 30480
   atcgtaagtt aggggggcgc ggcagctggg gccttgggct ctaggagggg cgatcgggtg 30540
   ccgggtgcct gcctgatgcc aattaccgac gcggcgacct tggggccagc ctcgcttgat 30600
   cctaacagtt ggacctgttg cagaggagca ggttcaggcg gctgctcctc cctcgggtct 30660
   gtcgggctcc caaggtactg cgcgcgatgc ttctgcagct ttggtctccg ttgggagcgt 30720
   ctggggcaag gagtgactca ctgtgaggag gcaggttgct gcctcttcag cacctgtcat 30780
   cagcagcttt gggtgctgct cttcgggcac ctggctgcgg cccagagctt agtctgcatt 30840
   gcatttgctc acgtctgtcg tatgttgttc tagacacagc acagaagagt catacagtaa 30900
   cccagattat cttgccgtgt ttgctgaatg tgtgttgtaa acatagccag tgctttgggt 30960
   cattcaaaag tagtgaatcg gagccgagcc tcataatagg ggaaatcaaa aagtaaaaag 31020
   caagttaaaa tttttctgag tgtagagttc tgatttccct tcgttcagtc atgacagcat 31080
   ctagcagggg tacgatccag tggggaggtc tgtcccaggg gtctaaaagg cagagaggga 31140
   gtaaagtggt tctcctgatt ccacctctcc tcctttcttc ctgctaaact tcgtttctgc 31200
   cttgttgttg tagcaacttc tggatgatta cccaaaatgc ttcatcgtgg gcgcagacaa 31260
   tgtgggctcc aagcagatgc agcagatccg catgtcgctc cgggggaagg ctgtggtgct 31320
   gatgggcaag aacaccatga tgcgcaaggc catccggggg catctggaaa acaaccctgc 31380
   cctggagaag tcagtcagct ccctgcccgg cgccccggtc cccgcagcgt ggggccagag 31440
   gggctctgtg ttgtataaac ttctttctcc gcaggctgct gcctcatatc cggggaaatg 31500
   tgggctttgt gttcaccaag gaggacctca ctgagatcag ggatatgcta ctggccaata 31560
   aggtagagga gagtcaagct ggctaaagag gcctgatgtt ggttctgtaa ggaaaacagt 31620
   caggctgtgt gagtggagaa gctgtgctca ccgttcctat ctttgctcag gtgccggctg 31680
   ctgcccgtgc tggtgccatt gcgccctgtg aggtcactgt gccagctcag aacactggtc 31740
   tggggcccga gaagacctcc tttttccagg ctttgggcat caccactaaa atctccaggg 31800
   gcaccattga aatcctggtg agtggacctg gtctgccagt gccagcctgg ccaatgcggg 31860
   cttctcttgg aggctgtggg cctttgtgtc caggtggtgg agtgcccact gttgccttgt 31920
   tctgcagagc gacgtgcagc tgataaagac tggagacaaa gtgggagcca gcgaggccac 31980
   gctgctgaac atgctgaaca tctcgccctt ctcgttcggg ctgatcatcc agcaggtgtt 32040
   cgacaatggc agcatctaca accccgaagt gctggacatc acggaggaca ccctgcactc 32100
   gcgcttcctg gaggtgccca atccagcccc cgctcgcgtg actggctgag agcgtgacca 32160
   gtggcagacg ccaagtcctg tctgcgctgc ttagagagtt gtctgtatag tcccttaatc 32220
   tacttccctt tgcagttgtg aaacacgctg acaggagact gttgatctaa tgaattagtg 32280
   taaagccttg gaacagtgct gggccctgga agggttcgtt ctggggccag ttacaaagcg 32340
   acaggagatt ctgggcacca tcatggtgac actgtcaagt ctccttccct ggagcgactg 32400
   attctgcttt ttttctgagt ccaccttgcc agatgtcacc ttatgaaggt tttcactgag 32460
   taatggtgat aggagattga tttaattttt ggaccaaaaa atcaattcta tttactgttt 32520
   gctcctgtta acttttttgg aagtctcatt ggtggatgaa attcataatg aagtaattta 32580
   aaagttttct ctcatgaaac tcagtggcta tgtgaatagc ctctgttttc cacaaaataa 32640
   tggtagcttt taaagtaaat agtgtcatag ctgagaaccc tcaggtgtgg ctgagggaga 32700
   ggccctgctg ggagcatttg gtgtgaggaa atgtgtcctt ggaaggagct gctggcctag 32760
   cagttgtctg ctgtgggtta cttctcctac cctccctagg gcagctcatc aaaaacgtca 32820
   tcttcccccc acagggtgtg cgcaacgttg ccagtgtgtg tctgcagatt ggctatccga 32880
   ccgtggcatc cgtgccccat tccatcatca atgggtacaa gcgtgtcctg gctttgtctg 32940
   tggagactga gtacaccttc ccacttgctg aaaaggtagg ttctgctagg acacagtggg 33000
   cctggcagca gggtgataag gggtagcctg ccaagttagt gtttttgtga tcatatttta 33060
   cacagtggat aggtttttta agagttaaga ggctgggaaa cagggcactt cagaaataag 33120
   atccagtggt gaggaggttg gggggggtgt cctgctggca gggggtgttc actgacttag 33180
   gtaatggctg tgaaatggct ggagccctgt ggatgaccat tttgaccact gacttcttcc 33240
   ctgcaggtca aggccttcct ggctgatcca tctgcatttg tggctgctgc ccctgtggct 33300
   gcggccagca ccgctgcccc tgctgctgcc gccgcagccc cagccaaggt ggaggccaag 33360
   gaagagtcgg aagagtcgga cgaagatatg ggatttggtc tcttcgacta atcactgcaa 33420
   agcagccagc tttatttgcg aaacaaggaa ataaaggctt acttctctta aagttctctt 33480
   tatcttgagg cttatagcgc tgtgtgctgg atcttacagc cctggtaaag ctagagcttt 33540
   gtatttgggt ggtgtctctg aatgaagcta ctaaccttct agtcctatac tttcctagac 33600
   ccagtttgtg agtgtacagt ggtccccccc gcccatcccc tttttaattt tattttataa 33660
   aatttatttc aaacgcagag ttagaaatct cccatctggg tcactcccca aacagctgca 33720
   agagccagag ctgtggcagg ctgaagccag gaacccagag ctgctgctgg gtctcccaca 33780
   tgggtacagg gccccaggcc cctgggccat cctgctgctt tcccacaggc attagtaggg 33840
   ggcagatgtg gagcatctca ctgtttaagc tgctatacct tttgttaaaa agatacattt 33900
   atttgaaaga gtaacagatc ttccatctgc tggttcactc caaattcctg caagaaccag 33960
   ggctgggcca ggttgcagcc agaggcctct cacatgagtg gcaggaaccc gggtacttgt 34020
   gcagtccttt tcctcccagg tggattggaa ctaggcactc ttggtaagga atggtgagtg 34080
   gcccacacag agcaaacgcc tgccctaaca acctgcagga attacagccc aagaccatcc 34140
   cctccccaag caggaaataa cagtatctta aatattggag attatacagg gcagttaagt 34200
   aacactatac catagtaaaa ctggtagcat gatgagttgt ttgagcacaa gaatcatggt 34260
   agcactacag ttgggtaact ttcaccatgc tgaaatgctg tgcagttgaa agctcaggaa 34320
   ttacgatttt ccatttagtt tctttggatg ggttgtctag atatgactgg gaaagtcaga 34380
   ccacaggtca ttgggggata ttttccctgc actacaaaag ctggttttcc acggccatcc 34440
   aaccattagt cgggctttcc tcctcactgc tcagtttgag ttcagggctg gctacagcca 34500
   gtttgctgga aaaaacaggc aatcctggaa gctttccagg ttcggcctgg agctgtgttg 34560
   ccgggtgtgt cttccggtaa ccagggtttg gcaaggtttg 34600

<210> 7
<211> 256
<212> DNA
<213> Oryctolagus cuniculus

<400> 7
ggaagtccaa ctacttcctt aagatcatcc aacttctgga tgattaccca aaatgcttca 60

tcgtgggcgc agacaatgtg ggctccaagc agatgcagca gatccgcatg tcgctccggg 120

ggaaggctgt ggtgctgatg ggcaagaaca ccatgatgcg caaggccatc cgggggcatc 180

tggaaaacaa ccctgccctg gagaagctgc tgcctcatat ccggggaaat gtgggctttg 240

tgttcaccaa ggagga 256

<210> 8
<211> 23
<212> DNA
<213> Artificial Sequence

<220>
<223> Forward primer of the 36b4 gene

<400> 8
cagcaagtgg gaaggtgtaa tcc 23

<210> 9
<211> 25
<212> DNA
<213> Artificial Sequence

<220>
<223> Reverse primer of the 36b4 gene

<400> 9
cccattctat catcaacggg tacaa 25

## Description

The present invention generally relates to the field of biomedicine, particularly to cancer prediction, detection, diagnosis, monitoring and treatment, and more specifically to methods for detecting early-onset colorectal cancers (ECRC) based on the reduced expression or absence of the *NOMO-1* gene.

### STATE OF THE ART

Colorectal cancer (CRC), specifically early-onset CRC (ECRC), which has an incidence of 2-8% of all CRC cases, has been increasing over the past few decades, reaching an incidence of 11% of colon cancers and 18% of rectal cancers (Ahnen DJ, et al. Mayo Clinic Proceedings. 2014;89(2):216-24). Its impact on the population is undeniable, and until recently it was commonly believed that for this subgroup of patients who had ECRC, within the general group of CRC patients, the main cause of CRC was hereditary. However, recent studies reject these theories and suggest that the appearance of ECRC in this subgroup of patients is due to microsatellite sequence stability (MSS), microsatellites being understood as small, repeated sequences of noncoding DNA that are found in an individual's genome (Perea J, et al. World J Gastreoenterol 2010; 16(29): 3697-3703; Perea J, et al. J Mol Diagn 2014; 16: 116-26). Furthermore, ECRC in young individuals, except in cases where a hereditary component is the main cause of the disease, is associated with Microsatellite Instability (MSI), these patients making up a specific subgroup of global CRC patients, and thus, determining the underlying molecular markers and mechanisms is essential.

ECRC has evolved from the controversial aspects with regard to the natural history and prognosis thereof to being characterized as a significant heterogeneity within this group (Losi L, et al. Am J Gastroenterol 2005; 100: 2280-2287). Moreover, it has been proposed that one's age at the onset of colorectal cancer should be a major criterion for subclassification of CRC (Perea J, et al. J Mol Diagn 2014; 16: 116-26). The majority of studies conclude that there are differentiating characteristics within the group of patients with ECRC, not only from a clinical point of view, but, even more importantly, according to some differentiating molecular aspects: a high degree of LINE-1 hypomethylation (Antelo M, et al. PLoS One. 2012; 7 (9):e45357); and greater frequency of chromosomal and genetic alterations (including some variants of susceptibility), possibly indicating familial or hereditary predisposition (Giráldez MD, et al . Carcinogenesis. 2012; 33: 613-19; Giráldez MD, et al. Clin Cancer Res. 2010 16(22):5402-13; Arriba M, et al. Mol Carcinog. 2015 Mar 25); or singular traits, such as mechanisms for maintaining telomeres (Boardman LA, et al. PLoSONE 8(11): e80015; Kirzin S, et al. PLoS ONE 9(8): e103159). Apart from these approaches, there is no molecular or genetic marker to date that can be associated with ECRC.

Phenotypic and genotypic heterogeneity of ECRC patients clearly arise from clinical studies. The results obtained in different clinical studies show how two different entities can be distinguished: (1) a hereditary subtype, generally familial aggregation, which represents a relatively low percentage of cases, with specific clinical-pathological characteristics of Lynch syndrome, and (2) a "sporadic" subtype, without a family history of CRC, with different locational and histopathological characteristics from those of individuals classified in the subgroup (1). Furthermore, there is significant variability in the underlying mechanisms of the ECRC development and, without a doubt, it is a serious concern for doctors and oncologists, specifically with regard to finding methods of prevention, diagnosis and clinical management of the disease. In this sense, in the state of the art there are no markers able to effectively diagnose this subgroup of ECRC patients within the global population of CRC patients.

Therefore, from a clinical point of view, there is an unmet need in the field of useful markers for the diagnosis, prognosis and/or monitoring of ECRC which are furthermore highly reliable. This being so, determining exact and reliable markers that identify and classify patients who are at risk, or who currently suffer from this type of cancer, and the use thereof in diagnostic, prognostic and/or monitoring methods for these types of patients, will on the one hand allow the characterization of patients who have ECRC to be improved, and on the other allow an individualized and suitable therapy to be provided for each specific clinical case.

### DESCRIPTION OF THE INVENTION

The present invention provides a new biomarker for the diagnosis, prognosis and/or monitoring of CRC, preferably ECRC, the *NOMO-1* (*Nodal Modulator 1*) gene, or any expression products therefrom. In the same way, the present invention describes an *in vitro* method of diagnosis, prognosis and/or monitoring of CRC, preferably ECRC, which comprises the detection and/or quantification of the presence or absence, and/or of the expression level, of the *NOMO-1* gene, or any expression products therefrom, in a biological sample, preferably a biological sample that contains tumor cells.

As the examples of the present invention show, the nucleic acid isolated from the tumor samples obtained from ECRC patients shows that the *NOMO-1* gene is not present, or is present in a lower copy number compared to the reference samples, preferably tumor samples of individuals with CRC. Thus, the present invention shows that the analysis of the presence or absence, and/or of the expression level, of the biomarker *NOMO-1,* is a useful method for the diagnosis, prognosis and/or monitoring of ECRC. In fact, as is shown below, the inventors have noticed that in patients with ECRC, there is a partial or complete deletion of at least one copy of the *NOMO-1* gene, which leads to the gene not being detected (absence) or to a reduction in the copy number of the same compared to a control. Therefore, the partial or complete deletion of at least one copy of *NOMO-1* implies a risk of developing, or the appearance of, ECRC.

Thus, a first aspect of the invention relates to the *in vitro* use of the *NOMO-1* gene and/or the expression products therefrom as a marker, hereinafter referred to as "marker or biomarker of the invention", for the diagnosis, prognosis and monitoring of ECRC.

For the purposes of the present invention, the terms "marker", "biomarker" or "biological marker" refer to a molecule, for example a nucleotide or genetic sequence, or a polypeptide or protein sequence, which indicates a specific disease state, providing information of clinical interest regarding the condition of a subject compared to a specific disease. For the purposes of the present invention, the term biomarker refers to the *NOMO-1* gene and/or the expression products therefrom.

For the purposes of the present invention, the term "colorectal cancer" or "colon cancer" or "CRC" includes any type of neoplasm of the colon, rectum or appendix. Colon cancer can be detected, for example, although without limitation, by means of a rectal examination, fecal occult blood test (FOBT), sigmoidoscopy, colonoscopy, virtual colonoscopy, double-contrast barium enema, ultrasound scan, biopsy or nuclear magnetic resonance (NMR). For the purposes of the present invention, the term "early-onset colorectal cancer" or "early-onset colon cancer" or "ECRC" refers to CRC that appears in young subjects, at an age in which these types of neoplasms are not common. For the purposes of the present invention, the disease is considered to be ECRC when the age of the individual is 45 or younger at the moment of the diagnosis.

The term "diagnosis", as used in the present invention, generally refers to the process by which a disease, nosological entity, syndrome, or any health-disease condition is identified. Specifically, the term "diagnosis of early-onset colorectal cancer", "early-onset CRC" or "ECRC" refers to the ability to identify or detect the presence of ECRC. The detection of ECRC, as understood by a person skilled in the art, does not intend to be correct for 100% of the samples analyzed. However, it requires that a statistically significant amount of the analyzed samples be classified correctly. The statistically significant amount can be established by a person skilled in the art by means of the use of different statistical tools; illustrative, non-limiting examples of said statistical tools include determining confidence intervals, determining the p-value, the Student's t-test, or Fisher's discriminant functions, etc. (for example, see Dowdy and Wearden, Statistics for Research, John Wiley & Sons, Nueva York 1983). Preferably, the confidence intervals are at least 90%, at least 95%, at least 97%, at least 98% or at least 99%. Preferably, the p-value is less than 0.1, 0.05, 0.01, 0.005 or 0.0001. Preferably, the teachings of the present invention allow the disease to be correctly detected in at least 60%, in at least 70%, in at least 80% or in at least 90% of the subjects of a specific group or population analyzed.

The term "prognosis" refers to the method by which a prediction of the future development or course of a disease is established, preferably a neoplastic disease, more preferably ECRC, including relapses, capability of metastatic dissemination or response to specific treatment.

The term "prediction" or "prognosis" or "monitoring", as described in the present invention, refers, but is not limited, to the probability that a patient, such as a patient suffering from ECRC, will respond in a favorable or unfavorable way to a specific treatment, and to the extension of said responses, or that the patient will survive, after the surgical removal of a primary tumor and/or chemotherapy for a period of time without a recurrence of ECRC.

The nucleotide sequence of the *NOMO-1* gene has the GenBank reference number: ENSG00000274779. In a more preferred embodiment, the *NOMO-1* gene comprises the sequence SEQ ID NO: 1, and more preferably consists of the SEQ ID NO: 1. The *NOMO-1* gene encodes a protein that forms part of a protein complex that participates in the nodal signaling pathway during the embryonic development of vertebrates. The term "expression product", as used in this description, refers to any product of transcription (RNA, including alternative rearrangement forms) or expression (protein) of this gene, to any form resulting from the processing of said products of transcription or expression. The expression products of this gene are preferably mRNA of SEQ ID NO: 3 (NM_014287) encoding the nomo-1 protein. The peptide sequence of the nomo-1 protein has the GenBank reference number NP_055102.3. In a more preferred embodiment, the expression product of the *NOMO-1* gene comprises the sequence SEQ ID NO: 2, and more preferably consists of the SEQ ID NO: 2.

Another aspect of the invention relates to an *in vitro* method for the diagnosis, prognosis and monitoring of early-onset CRC, henceforth *"in vitro* method of the invention", which comprises: (a) detecting and/or quantifying the copy number and/or the expression level of the *NOMO-1* gene, and/or the expression product therefrom, in an isolated biological sample of an individual, (b) comparing the quantity detected in step (a) to a reference quantity, and (c) assigning the individual of (a) to the group of patients at risk of developing ECRC when the quantity detected in (a) is less than the reference quantity.

In a more preferred embodiment, the *in vitro* method of the invention comprises in step (a) detecting and/or quantifying the copy number of the *NOMO-1* gene, (b) comparing the quantity detected in step (a) to a reference quantity, and (c) assigning the individual of (a) to the group of patients with a predisposition, risk of developing, or a diagnosis of ECRC when the quantity detected in (a) is less than the reference quantity.

For the purposes of the present invention, the term "allele", as used herein, refers to one, two or more forms of a gene or genetic locus or polymorphism. Sometimes different alleles can give rise to different phenotypes; however, other times, different alleles will have the same result in the expression of a gene. The majority of multicellular organisms have two sets of chromosomes, meaning they are diploids. These chromosomes are called homologous chromosomes. Diploid organisms have a copy of each gene (and one allele) in each chromosome. If both alleles are equal, they are homozygous. If the alleles are different, they are heterozygous.

In this way, the expression "at least one copy of the *NOMO-1* gene is partially or completely deleted" means that when carrying out step (a) of the method, the *NOMO-1* gene is either not detected (which means that the only copy of said gene is deleted in the sample of the individual) or a reduction in the copy number of the gene is detected as a consequence of the total or partial deletion of one or more copies of the same compared to well-established controls (as will be illustrated below).

In a preferred embodiment of the first aspect of the invention, when step (a) is carried out, the *NOMO-1* gene is not detected in the sample of the patient. In an even more preferred embodiment, the subject in whom the *NOMO-1* gene is not detected is an individual that shows a homozygous deletion of the *NOMO-1* gene, indicative of a predisposition or risk of developing ECRC, or of having ECRC. In another even more preferred embodiment, the method of the invention is characterized in that in step (a), the gene has at least a partially or completely deleted copy.

In another even more preferred embodiment, the first aspect of the invention is characterized in that in step (c), the absence of the expression of the *NOMO-1* gene compared to the reference quantity, or the absence of at least one copy, partially or completely deleted from the *NOMO-1* gene, is indicative of a diagnosis, predisposition or risk of developing ECRC. In another even more preferred embodiment, in step (c), the reduction in at least 50% of the copy number of the *NOMO-1* gene compared to the reference quantity, is indicative of a diagnosis, predisposition or risk of developing ECRC.

When determining the absence of the *NOMO-1* gene in a sample of a patient, or a reduction in the copy number, different methodologies are carried out which are, in turn, complementary. According to preferred embodiments of the *in vitro* method described in the present invention, the nucleic acid obtained form the isolated sample of the subject can be genotyped, and this will indicate that there has been a complete deletion of the copy or copies of the *NOMO-1* gene, and consequently, that there is a predisposition or risk of developing ECRC. If the genotype obtained by means of the analysis of the *NOMO-1* gene is not conclusive (meaning, it cannot be determined that there is a complete absence of *NOMO-1*), the copy number present in the sample of the patient is determined and compared to the copy number in a control sample. If it is observed that the copy number of the *NOMO-1* gene in the patient is less than the copy number of the control, it will imply that one or more copies of the *NOMO-1* gene in the sample of the individual has been deleted and, as such, there is a predisposition for developing ECRC.

The expression "completely or partially deleted" means that the gene is completely deleted or that a fragment thereof is deleted (for example, an exon of *NOMO-1*). When in the present invention the expression "absence" is used in relation to the *NOMO-1* gene, it means that when step (a) is carried out according to the method described in the present invention, no copy of the *NOMO-1* gene is detected in the sample of the individual.

In another more preferred embodiment, the *in vitro* method of the invention comprises in step (a) detecting and/or quantifying the expression level of the *NOMO-1* gene, and/or the expression product therefrom, in an isolated biological sample of an individual, (b) comparing the quantity detected in step (a) to a reference quantity, and (c) assigning the individual of (a) to the group of patients at risk of developing ECRC, when the quantity detected in (a) is less than the reference quantity.

In a more preferred embodiment, the *in vitro* method of the invention comprises in step (a) detecting and/or quantifying the copy number of the gene and the expression level of the *NOMO-1* gene, and/or the expression product therefrom, in an isolated biological sample of an individual, (b) comparing the quantity detected in step (a) to a reference quantity, and (c) assigning the individual of (a) to the group of patients at risk of developing ECRC, when the quantity detected in (a) is less than the reference quantity. In a more preferred embodiment of the *in vitro* method of the invention, it is characterized in that the *NOMO-1* gene comprises the sequence SEQ ID NO: 1 and even more preferably, the *NOMO-1* gene consists of the SEQ ID NO: 1. In another more preferred embodiment, the expression product of the *NOMO-1* gene comprises the SEQ ID NO: 2, and even more preferably consists of the SEQ ID NO: 2.

The biological sample of the subject under study is a biological sample that contains a nucleic acid, for example DNA, genomic DNA (gDNA), complementary DNA (cDNA), RNA, heterogeneous nuclear RNA (hnRNA), mRNA, etc. of the subject being evaluated. For the purposes of the present invention, the term "isolated biological sample" refers, but is not limited, to cellular populations and biological tissues and/or fluids of a subject, obtained by means of any method known by a person skilled in the art that may be used for said purpose. The biological sample can contain any material suitable for detecting the desired biomarker and can comprise cells and/or non-cellular material of the subject. Preferably, the "isolated biological sample comprises tumor cells", and as it is used in the description refers, but is not limited, to biological tissues and/or fluids of an individual obtained by means of any method known by a person skilled in the art that may be used for said purpose. The biological sample can be, for example, but not limited to, a tumor biopsy or fine needle aspiration. In an even more preferred embodiment, the isolated biological sample for determining the copy number of the *NOMO*-1 gene and/or the expression levels of the biomarker of the invention is a tissue sample of the colon, preferably obtained from a biopsy.

On the other hand, the biological sample is a sample of biological fluid. The terms "biological fluid" and "biofluid" are used interchangeably in this document and they refer to aqueous fluids from biological origin. The biofluid can be obtained from any location (such as blood, plasma, serum, urine, bile, cerebrospinal fluid, vitreous or aqueous humor, or any bodily secretion), an exudate (such as liquid obtained from an abscess or any other site of infection or inflammation) or liquid obtained from a joint (for example a normal joint or a joint affected by a disease, such as rheumatoid arthritis). In a preferred embodiment, the biofluid of the invention is preferably blood, concentrated leukocytes, peripheral blood mononuclear cells or peripheral blood lymphocytes.

The sample can be taken from a human, or also from non-human mammals, for example, but not limited to, rodents, ruminants, felines or canines. Thus, in a preferred embodiment of this aspect of the invention, the "individual" or "subject", used interchangeably throughout the present document, from which the isolated biological sample of step (a) of the *in vitro* method of the invention is taken, is a mammal. In a more particular embodiment, the mammal is a human, man or woman of any age or race.

The detection and or quantification of the copy number of the *NOMO-1* gene refers to determining if gene amplification is detected or not and to what degree. It also therefore includes determining the expression level of the *NOMO-1* gene and/or the expression product therefrom in the isolated biological sample, referring to the measurement of the quantity or concentration, preferably in a semi-quantitative or quantitative way. This measurement can be taken directly or indirectly. The direct measurement refers to the measurement of the quantity or concentration of both the copy number in genomic material and the expression product of the gene, preferably mRNA, based on a signal that is obtained and directly correlated to the number of molecules of the expression product of the gene present in the sample. Said signal - which we may also refer to as signal intensity - can be obtained, for example, by measuring an intensity value of a chemical or physical property of the expression product. The indirect measurement includes the measurement obtained from a secondary component (for example, a component different from the genetic expression product) or a biological measuring system (for example, the measurement of cellular responses, ligands, "labels" or products of enzymatic reactions).

According to the present invention, detecting the copy number or the quantity of the expression product of the gene can be done by any method for determining the quantity of DNA or the expression product of the genes known by a person skilled in the art. In a preferred embodiment, detecting the copy number of the gene is done by amplifying a fragment of DNA that contains the gene and comparing the copy number to a reference gene, for which it is known that two copies exist. The analysis of the copy number is done by means of RT-qPCT. In the case of determining the quantity of the expression product of the gene, the same is done by determining the mRNA level derived from the transcription thereof, prior to complete RNA extraction of the isolated biological sample, which can be done using methods known by a person skilled in the art. The analysis of the mRNA level can be done, by way of illustration and without limiting the scope of the invention, by means of amplification by polymerase chain reaction (PCR), reverse transcription in combination with the ligase chain reaction (RTLCR), reverse transcription in combination with the polymerase chain reaction (RT-PCR), reverse transcription in combination with the quantitative polymerase chain reaction (RT-qPCR), or any other method for amplifying nucleic acids; DNA microarrays prepared with oligonucleotides deposited by any mechanism; DNA microarrays prepared with oligonucleotides synthesized *in situ* by means of photolithography or by any other mechanism; *in situ* hybridization using specific probes marked by any marking method; by means of gel electrophoresis; by means of membrane transfer and hybridization with a specific probe; by means of NMR or any other image diagnosis technique using paramagnetic nanoparticles or any other type of detectable nanoparticles functionalized with antibodies or by any other means.

In another preferred embodiment, detecting the expression product quantity of the gene is done by determining the level of the nomo-1 protein, by means of, for example, although without being limited to, immunocytochemical and immunohistochemical techniques, Western Blot, ELISA, *Enzyme-linked immunosorbent assay*), RIA (radioimmunoassay), EIA (enzyme immunoassay), DAS-ELISA (double antibody sandwich ELISA), techniques based on the use of protein biochips or microarrays as well as specific antibodies or assays based on the colloidal precipitation in formats such as test strips. Other forms of detecting and quantifying the protein include techniques of affinity chromatography, ligand binding assays, etc.

In an even more preferred embodiment, detecting the copy number and/or quantity of the expression product of *NOMO-1* is done by means of RT-qPCR and detecting the expression product therefrom is preferably done by means of Western-blot or ELISA.

In a preferred embodiment of the *in vitro* method of the invention, it is also possible, in addition to detecting a fragment of the proteins used as biomarkers, to detect and quantify a functionally equivalent variant thereof.

In the meaning used in this description, the term "variant" refers to proteins substantially homologous to the protein encoded by the *NOMO-1* gene. In general, a variant includes additions, deletions or substitutions of amino acids, as long as said variants are functionally equivalent to the original protein. The term "variant" also includes proteins resulting from post-translational modifications such as, but not limited to, glycosylation, phosphorylation or methylation.

The expression "functionally equivalent", as used in the present description, means that the protein or fragment of the protein in question essentially maintains the immunological properties described in this document. Said immunological properties can be determined by means of conventional methods, such as those previously described.

The term "fragment", as used in the present description, refers to a portion of the protein encoded by the *NOMO-1* gene or one of the variants thereof.

The expression level of a gene or the expression product therefrom, determined in a biological sample from a subject under study, is said to be "greater" than the reference level or quantity of said gene or the expression product therefrom when, according to the invention, the level of said gene or the expression product therefrom in the biological sample of the subject or individual is at least 1.5 times, 2 times, 3 times, 4 times, 5 times, 10 times, 20 times, 30 times, 40 times, 50 times, 60 times, 70 times, 80 times, 90 times, 100 times, or even more, compared to the reference level of said gene or the expression product therefrom. Similarly, the expression level of a gene or of the expression product therefrom, determined in a biological sample from a subject or individual under study, is said to be "less" than the reference level or quantity of said gene or the expression product therefrom when, according to the invention, the level of said expression product therefrom in said biological sample of the subject is at least 1.5 times, 2 times, 5 times, 10 times, 20 times, 30 times, 40 times, 50 times, 60 times, 70 times, 80 times, 90 times, 100 times, or even more, below the reference level of said expression product of said gene. For the purposes of the present invention, the term "less" also refers to the complete absence of the expression of the gene or the expression product therefrom.

The term "reference quantity", as used in the present description, refers to any value or value range derived from the quantification of the *NOMO-1* gene and/or the expression product therefrom in a control biological sample. The suitable reference quantity can be determined by the method of the present invention from a reference sample that can be analyzed, for example, simultaneously or consecutively, together with the problem biological sample. Thus, for example, but without limitation, the reference sample can be the negative control, meaning the quantity detected by the method of the invention in samples from individuals who do not have the disease. Alternatively, for example, it can also be the quantity detected by the method of the invention in samples from individuals who have CRC but who do not have ECRC. In a preferred embodiment, the reference quantity comes from an isolated biological sample that can comprise, or not, tumor cells, wherein said sample can come from a control individual who is healthy or who does not have ECRC, or from an individual who has CRC, as long as it is not early-onset. A "reference sample", as used herein, refers to a sample obtained from a group of healthy subjects who do not have a condition of the disease or specific phenotype. Preferably, the reference sample can include mucus samples of the colon of patients who do not have colon cancer or who do not have a family history of colon cancer. On the other hand, the reference sample could be a sample or a set of samples of colon cancer obtained from patients diagnosed with non-early-onset CRC.

Said reference quantity, according to the present invention, allows the presence of ECRC to be differentiated with respect to individuals who have non-early-onset CRC and, therefore, can be used in the diagnosis, prognosis and monitoring of the evolution of an ECRC.

Steps (a) and/or (b) of the *in vitro* method of the invention can be completely or partially automated, for example, but without limitation, by means of a robotic device for detection and/or quantification in step (a) of the copy number and/or the quantity of the expression of the *NOMO-1* gene, or the expression product therefrom, in the isolated biological sample.

In addition to the aforementioned steps, the *in vitro* method of the invention can comprise other additional steps, for example, but without limitation, related to the pretreatment of the isolated biological sample prior to the analysis thereof.

Thus, the *in vitro* method of the invention is useful for establishing the diagnosis, prognosis and monitoring of ECRC.

Another aspect of the invention relates to a kit for the diagnosis, prognosis and/or monitoring of ECRC, hereinafter "kit of the invention", which comprises the primers, probes, antibodies, or any of the combinations thereof, necessary for detecting the copy number and/or the quantity of the expression of the *NOMO-1* gene and/or the expression product therefrom, preferably in an isolated biological sample.

The primers, probes and/or antibodies comprised in the kit of the invention are complementary and, therefore, have hybridization capacity with the *NOMO-1* and/or at least an expression product thereof. In general, the kit of the invention comprises all necessary reagents for carrying out the previously described method of the invention. The kit can further include, without any type of limitation, buffers, enzymes, as well as, for example, but without limitation, polymerases, cofactors for obtaining an optimal activity thereof, agents for preventing contamination, etc. On the other hand, the kit can include all of the necessary supports and containers for the start-up and optimization thereof. The kit can further contain other molecules, genes, proteins or probes of interest, which function as positive and negative controls. Preferably, the kit further comprises instructions for carrying out the method of the invention. Preferably, the kit of the invention comprises the oligonucleotides of SEQ ID NO: 4 and SEQ ID NO: 5.

Another aspect of the invention relates to the *in vitro* use of the kit of the invention for the diagnosis, prognosis and/or monitoring of ECRC, preferably in an isolated biological sample, as described throughout the present document.

Throughout the description and the claims, the word "comprises" and its variants are not intended to exclude other technical characteristics, additives, components or steps. For those skilled in the art, other objects, advantages and characteristics of the invention may be deduced from both the description and the practical use of the invention. The following examples are provided by way of illustration and are not intended to be limiting of the present invention.

### EXAMPLES

The invention is illustrated below by means of tests carried out by the inventors which reveal the effectiveness of the method of the invention in the diagnosis, prognosis and monitoring of ECRC. These specific examples provided serve to illustrate the nature of the present invention and are only included for illustrative purposes, and therefore, they should not be interpreted as limiting the invention claimed herein. Thus, the examples described below illustrate the invention without limiting the field of application thereof.

### Example 1. Determination of the NOMO-1 gene as a biomarker in the diagnosis and characterization of patients who have ECRC compared to patients that have CRC.

A total of 34 samples of tumor tissue were collected from individuals consecutively diagnosed with ECRC, at an age equal to or less than 45 years old. Furthermore, 17 samples of tumor tissue were collected from individuals consecutively diagnosed with CRC but at an age equal to or greater than 70 years old, for the purpose of comparing them to the group of young individuals who had ECRC. The clinical characteristics of both groups of patients included in the study are shown in Table 1. The microsatellite instability (MSI) and the mutations related to Lynch syndrome were analyzed for all of the individuals (Table 1). All of them were monitored for at least five years after the surgery. All of the patients included in the study signed the informed consent form.

In samples of young subjects, younger than 45 years old, hereditary origin of the presence of ECRC was ruled out, as well as the presence of other solid tumors, such as endometrial cancer and glioblastoma.

**Table 1. Clinical characteristics of the patients included in the present study.**

| | **ECRC** | **CRC in the elderly** |
|---|---|---|
| | **n (%)** | **n (%)** |
| **Patients** | 34 | 17 |
| **Average age (SD)¹** | 38.65 (5.2) | 79.5 (5.2) |
| **Sex** | | |
| Male | 19 (56) | 7 (41.2) |
| Female | 15 (44) | 10 (58.8) |
| **Location** | | |
| Right colon | 11 (32.4) | 7 (41.2) |
| Left colon | 17 (50) | 3 (17.6) |
| Rectum | 6 (17.6) | 7 (41.2) |
| **Tumor differentiation²** | | |
| Poorly differentiated | 2/27 (7.4) | 0/16 (0) |
| **Mucinous tumors²** | 10/27 (37) | 1/16 (6.3) |
| **Signet ring cells²** | 0 (0) | 0/16 (0) |
| **Modified Astler-Coller staging** | | |
| A | 7 (20.6) | 3 (17.6) |
| B | 17 (50) | 6 (35.3) |
| C | 3 (8.8) | 6 (35.3) |
| D | 7 (20.6) | 2 (11.8) |
| **Associated polyps** | | |
| **Average number of polyps (SD)¹** | 21 (61.8) | 12 (70.6) |
| **Type** | 2.3 (3) | 2.8 (3.5) |
| Adenomatous | 7 (33.3) | 6 (50) |
| Hyperplastic | 3 (14.3) | 2 (16.7) |
| Mixed | 11 (52.4) | 4 (33.3) |
| **Synchronous or metachronous CRC** | 3 (8.8) | 3 (17.6) |
| **Recurrence³** | 4 (14.8) | 2 (20) |
| **Related mortality** | 7 (20.6) | 3 (17.6) |
| **Disease-free Survival (SD)¹** | 57.7 (43.6) | 27.2 (37.8) |
| **Global Survival (SD)¹** | 67.5 (37.2) | 27.3 (32.3) |
| **MSI** | 7 (20.6) | 1 (5.9) |
| **Mutations in MMR genes** | 6 (17.6) | 0 (0) |
| **Family history of cancer** | | |
| Amsterdam II Positive Families | 9 (26.5) | 0 (0) |
| Sporadic cases. | 12 (35.3) | 10 (58.8) |

| | | |
|---|---|---|
| ¹Statistical analysis done by means of Student's t test. ²The proportions shown are based on the variable total number of cases, since cases in which only a biopsy was obtained are excluded (stage D, for example)³. The cases that show recurrence are those with initial stages C or less. SD: Standard deviation. CRC: Colorectal Cancer; ECRC: Early-onset Colorectal Cancer. MSI Microsatellite Instability. MMR: System for repairing errors in DNA. | | |

The total DNA was extracted from the tumor tissue samples using conventional techniques. The quantity and purity of the DNA samples were obtained by the Nanodrop (Thermo Scientific D- 1000).

For detecting the presence or absence of the *NOMO-1* gene (SEQ ID NO: 1) in samples obtained from said patients, a RT-qPCR was performed to quantify the number of relative alleles of said gene in each patient. The RT-qPCR was done by means of the FastStart Universal SYBR Green Master (ROX) (2x con.) in the StepOnePlusTM Real-Time PCR System (Life Technologies-Invitrogen, California, U.S.A.). Briefly, a fragment of the *NOMO-1* gene (SEQ ID NO: 1) was amplified in the DNA obtained from the isolated samples of the subjects included in the study using the following primers: forward primer 5'-agctccatgtggatggagtc-3' (SEQ ID NO: 4) and reverse primer: 5'-acggatgaagtacagagttc-3' (SEQ ID NO: 5). As an internal control for normalizing the genetic expression levels, the gene 36b4 (SEQ ID NO: 6) was used, and was amplified in the same DNA using the following primers: forward primer: 5'-cagcaagtgggaaggtgtaatcc-3' (SEQ ID NO: 8) and reverse primer 5'-cccattctatcatcaacgggtacaa-3' (SEQ ID NO: 9). For the reaction of qRT-PCR, a concentration of 15 ng of DNA and a final reaction volume of 10 µl were used. SYBR Green (Roche, FastStart Universal SYBR Green Master (ROX) Applied Science, Germany) were used for detecting the products of the PCR. The One-step RT-PCR reactions were carried out in plates with 96 wells covered with an adhesive film (MicroAmp Optical Adhesive Film, Life Technologies-Invitrogen, California, U.S.A) to avoid the evaporation of the samples. The conditions of RT-qPCR were 10 minutes at 95°C followed by 40 cycles at 95°C for 15 seconds, subsequently at 58°C for 45 seconds and 72°C for 15 seconds. These reactions were carried out in an Applied Biosystems StepOnePlus thermo cycler and the results were obtained with the RQ Manager software (Applied Biosystems). The data were analyzed according to the analysis of relative expression (ddCt) previously described by Pfaffl (Pfaffl et al., 2002. Nucleic Acids Res. May 1 ;30(9):e36). The data of the presence or absence and/or of the expression levels of the *NOMO-1* gene in the samples of patients analyzed are normalized on a Log10 scale.

All of the results are expressed as values of the average ± standard deviation (SD) and the categorical variables are expressed in number of cases and percentage thereof. The differences are considered significant for values of p<0.05. All of the statistical analyses were preformed by means of the Windows SPSS program, v. 11.5 (SPSS, Inc., Chicago, IL).

The results of the detection of the presence or absence of the *NOMO-1* gene in the group of young patients that have ECRC have demonstrated that the 34 individuals analyzed showed residual amplification of said gene, always less than half of the copies detected in healthy tissue, in other words, 100% of the individuals analyzed showed homozygous loss of the *NOMO-1* gene, since it was not possible to detect the presence of said gene. In turn, of the 17 elderly patients with CRC, only two cases showed homozygous loss of *NOMO-1* while five had heterozygous loss, and in the other 10 cases, the there was a normal presence of the *NOMO-1* gene. Thus, within the elderly population diagnosed with CRC, only 2 of 17 individuals showed an absence of the *NOMO-1* gene, demonstrating that only 11.7% of the elderly population with CRC had homozygous deletion of said gene.

To validate the results obtained in the first group of patients, the sample was widened to an independent ECRC group of 41 individuals, representative of three independent institutions: University Hospital of Salamanca (Salamanca, Spain), Familial Cancer Clinical Unit of the National Center for Cancer Research (CNIO) (Madrid, Spain) and Hospital 12 de Octubre (Madrid, Spain). All of the patients signed the informed consent form before being included in the study.

For the analysis of the detection of the presence or absence (preferably analyzing the presence of the number of alleles of the gene) and/or of the expression levels of *NOMO-1,* the same previously described method was used in the new group of patients. Thus, the presence of the *NOMO-1* gene (SEQ ID NO: 1) was analyzed by means of RT-qPCR in samples of tumor tissue obtained from the new group of young patients that had ECRC (validation group). The results obtained show that of the 41 new cases, 27 showed homozygous loss (total absence of the expression of the *NOMO-1* gene); 7 showed heterozygous loss (50% amplification of the *NOMO-1* gene compared to the group of elderly individuals with CRC), and 7 showed a normal expression of the *NOMO-1* gene.

All of the results obtained were then grouped together, both from the first group of patients and the second group (validation group), obtaining a total of 75 individuals who have early-onset CRC. This grouping, as can be seen in Table 2, confirmed that 61 individuals (81.3% of the total population analyzed) showed a loss of the *NOMO-1* gene, thereby confirming that said patients had homozygous deletion of the *NOMO-1* gene. On the other hand, 7 individuals (9.3% of the total population analyzed) had heterozygous deletion of the *NOMO-1* gene, and the other 7 individuals (9.3% of the total population analyzed) showed a normal expression of said gene. These results demonstrate the usefulness of the *NOMO*-1 gene as a specific biomarker for the diagnosis, prognosis and/or monitoring of patients that have ECRC.

Subsequently, we sought to determine if the MSI phenotype could influence the diagnosis of ECRC, also associated with the absence to a greater or lesser degree of the expression of the *NOMO-1* gene. The patients were subdivided based on the MSI phenotype they had (Table 2). For young patients who had an MSS phenotype, the majority showed a homozygous loss of the *NOMO-1* gene (54 of 59) (91.5%), while only 7 of 16 patients that had the MSI phenotype showed homozygous loss of the gene (43.74%) (Table 2). Therefore, the heterozygous loss of the *NOMO-1* seems to be rare in the cases of individuals with ECRC that show an MSS phenotype (3.3%, 2/59) and much more frequent in cases of individuals with ECRC that have MSI phenotype (31.2%, 5/16) (Table 2).

**Table 2. Analysis of the expression of the NOMO-1 gene in groups of patients with early-onset CRC compared to the presence of the MSS or MSI phenotype.**

| | ECRC Original group (n:34) | | ECRC Validation group (n:41) | | ECRC Total (n:75) | |
|---|---|---|---|---|---|---|
| | MSS | MSI | MSS | MSI | MSS | MSI |
| **Homozygous deletion *NOMO*-1** | 31 | 3 | 23 | 4 | 54 | 7 |
| **Heterozygous deletion *NOMO*-1** | 0 | 0 | 2 | 5 | 2 | 5 |
| **Normal *NOMO-1*** expression | 0 | 0 | 3 | 4 | 3 | 4 |

| | | | | | | |
|---|---|---|---|---|---|---|
| ECRC: Early-onset Colorectal Cancer. MSI: Microsatellite Instability. MSS: Microsatellite Stability. | | | | | | |

To confirm that the absence of the *NOMO-1* gene expression, both in homozygosity and heterozygosity, is a specific marker in the diagnosis of ECRC, the alleles of said gene were quantified in different tumor tissue samples from individuals who were younger than 50 years old and who had other types of cancer, such as Familial adenomatous polyposis, glioblastoma multiforme and early-onset endometrial carcinoma. The results obtained with respect to the expression of the *NOMO-1* gene in the samples of said patients demonstrated that in none of said tumors analyzed was an alteration in the number of alleles of said gene produced compared to a reference quantity, all of them having a normal quantity with respect to the expression level of the endogenous gene analyzed and used as a reference.

Therefore, in light of the results shown in the present document, the absence of the expression of the *NOMO-1* gene is a clinical marker of ECRC, specifically useful in the diagnosis and/or prognosis of said pathology. Thus, early diagnosis of said individuals allows specific therapies to be designed for these types of patients, and also allows the *NOMO-1* gene to be used as a therapeutic target for treating said individuals.

## Claims

1. An *in vitro* method for the diagnosis, prognosis, or monitoring of the evolution of early-onset colorectal cancer (ECRC) in an individual, comprising:
a) detecting and/or quantifying the copy number and/or the expression level of the *NOMO-1* gene, and/or the expression product therefrom, in an isolated biological sample of said individual,
b) comparing the quantity detected in step (a) to a reference quantity, and
c) assigning the individual of (a) to the group of patients at risk of developing or having ECRC when the quantity detected in (a) is less than the reference quantity.

2. The method according to claim 1, wherein step (a) comprises detecting and/or quantifying the copy number of the *NOMO-1* gene.

3. The method according to claim 1, wherein step (a) comprises detecting and/or quantifying the copy number and the expression level of the *NOMO-1* gene.

4. The method according to any of the claims 1 to 3, wherein the *NOMO-1* gene comprises the SEQ ID NO: 1 and the expression product of the *NOMO-1* gene comprises the SEQ ID NO: 2.

5. The method according to any of the claims 1 to 4, **characterized in that** in step (c), the absence of the expression of the *NOMO-1* gene compared to the reference quantity is indicative of a diagnosis, predisposition or risk of developing ECRC.

6. The method according to any of the claims 1 to 4, **characterized in that** in step (c) the reduction of at least 50% of the copy number of the *NOMO-1* gene compared to the reference quantity is indicative of a diagnosis, predisposition or risk of developing ECRC.

7. The method according to any of the claims 1 to 6, wherein the detection of the copy number and/or expression levels of the *NOMO-1* gene are carried out by means of any of the methods that comprise: RT-PCR, RT-qPCR, microarray, northern blot, or any of the combinations thereof, and/or the detection of the expression product of the *NOMO-1* gene is carried out by means of any of the methods that comprise:
immunohistochemistry, ELISA, immunolabeling, or any of the combinations thereof.

8. The method according to any of the claims 1 to 7, wherein the reference quantity is obtained from an isolated biological sample of a healthy subject or of a subject who has CRC.

9. The method according to any of the claims 1 to 8, wherein the isolated biological sample is selected from the list that consists of a tissue sample and a biological fluid sample.

10. The method according to claim 9 wherein the tissue sample is a tumor tissue sample, preferably a biopsy.

11. The method according to any of the claims 1 to 10 wherein the individual is a mammal, preferably a human being.

12. An *in vitro* use of the *NOMO-1* gene and/or the expression product therefrom as a marker for the diagnosis, prognosis or monitoring of the evolution of ECRC.

13. The *in vitro* use according to claim 12, wherein the *NOMO-1* gene comprises the SEQ ID NO: 1 and the expression product therefrom comprises the SEQ ID NO: 2.

14. The *in vitro* use according to any of the claims 12 to 13 wherein the individual is a mammal, preferably a human being.

15. A kit for the diagnosis, prognosis or monitoring of the evolution of ECRC, which comprises the oligonucleotides that comprise the SEQ ID NO: 4 and SEQ ID NO: 5, to detect and/or quantify the copy number and/or the expression level of the *NOMO-1* gene, and/or the expression product therefrom.

16. The kit according to claim 15, **characterized in that** it additionally comprises probes, antibodies, reagents or any combination thereof.

17. The *in vitro* use of the kit according to any of the claims 15 to 16 for the diagnosis, prognosis or monitoring of the evolution of ECRC.
